(19) Europäisches Patentamt
European Patent Office
Office européen des brevets

(11) **EP 4 768 479 A1**

(12) **EUROPEAN PATENT APPLICATION**
published in accordance with Art. 153(4) EPC

(43) Date of publication:
**01.07.2026 Bulletin 2026/27**

(21) Application number: **24858462.5**

(22) Date of filing: **23.08.2024**

(51) International Patent Classification (IPC):
***C07D 409/10*** *(2006.01)* ***A61K 31/4178*** *(2006.01)*
***A61P 1/00*** *(2006.01)* ***A61P 9/00*** *(2006.01)*
***A61P 11/00*** *(2006.01)* ***A61P 13/12*** *(2006.01)*
***A61P 15/00*** *(2006.01)* ***A61P 27/02*** *(2006.01)*

(52) Cooperative Patent Classification (CPC):
**A61K 31/4178; A61K 31/4439; A61K 31/506; A61P 1/00; A61P 9/00; A61P 11/00; A61P 13/12; A61P 15/00; A61P 25/28; A61P 27/02; C07D 409/10; C07D 409/14; C07D 491/107**

(86) International application number:
**PCT/CN2024/114192**

(87) International publication number:
**WO 2025/044926 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.08.2023 CN 202311089313**
**14.03.2024 CN 202410298612**
**12.08.2024 CN 202411103404**

(71) Applicant: **Hubei Bio-Pharmaceutical Industrial Technological Institute Inc.**
**Wuhan, Hubei 430075 (CN)**

(72) Inventors:
- **ZHANG, Xuejun**
**Wuhan, Hubei 430075 (CN)**
- **ZANG, Yang**
**Wuhan, Hubei 430075 (CN)**
- **FU, Haoliang**
**Wuhan, Hubei 430075 (CN)**
- **SUN, Xiaochuan**
**Wuhan, Hubei 430075 (CN)**
- **ZHUO, Junming**
**Wuhan, Hubei 430075 (CN)**
- **AN, Ke**
**Wuhan, Hubei 430075 (CN)**
- **GAO, Zhenxing**
**Wuhan, Hubei 430075 (CN)**
- **ZHAO, Xin**
**Wuhan, Hubei 430075 (CN)**

(74) Representative: **M. Zardi & Co S.A.**
**Via G. B. Pioda, 6**
**6900 Lugano (CH)**

(54) **THIENYLSULFONYL CARBAMATES AS AT2R AGONISTS**

(57) Compounds represented by the following structure, or tautomers, stereoisomers, hydrates, solvates, pharmaceutically acceptable salts or prodrugs thereof. Said compounds can be used as AT2R agonists.

EP 4 768 479 A1

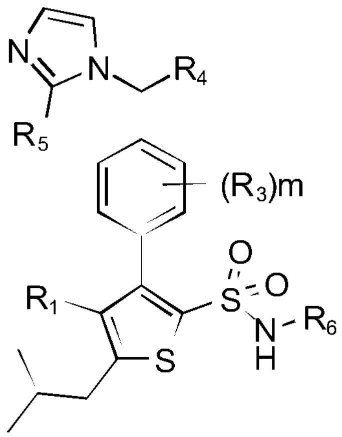

III.

## Description

### PRIORITY INFORMATION

This application claims priority and benefits to Chinese Patent Application No. 202311089313.4 filed with China National Intellectual Property Administration on August 25, 2023, Chinese Patent Application No. 202410298612.7 filed with China National Intellectual Property Administration on March 14, 2024, and Chinese Patent Application No. 202411103404.3 filed with China National Intellectual Property Administration on August 12, 2024, the entire disclosures of which are incorporated herein by reference.

### FIELD

The present disclosure belongs to the field of medicine. Particularly, the present disclosure relates to a thienylsulfonyl carbamate as an Angiotensin II (AngII) type 2 receptor (AT2R) agonist.

### BACKGROUND

Idiopathic pulmonary fibrosis (IPF) refers to an abnormal repair of damaged alveolar epithelial cells, which leads to proliferation of pulmonary fibroblasts and their transformation into myofibroblasts, excessive secretion of extracellular matrix, collagen deposition, alteration of alveolar structure, and ultimately formation of fibrosis. The pathogenesis of IPF has not been fully elucidated, but current research suggests that it is closely related to oxidative stress, inflammatory response, and regulation of a renin-angiotensin-aldosterone system (RAAS) by bodily fluids. It is currently believed that the RAAS system is instrumental in a process of the pulmonary fibrosis. An angiotensin converting enzyme (ACE) can hydrolyze angiotensin I (Ang I) into Ang II, and Ang II exerts important effects in occurrence and development of various inflammatory processes.

In humans, two major types of Ang II receptors have been identified, designated as Ang II type 1 receptor (AT1R) and Ang II type 2 receptor (AT2R). Ang II has exhibited functions of regulating blood pressure, and fluid and electrolyte homeostasis in numerous organs, including kidneys, adrenal glands, heart, blood vessels, brain, gastrointestinal tract, and reproductive organs. An effect of Ang II is regulated by a balance of an expression of two G protein-coupled receptors (GPCRs), including AT1R and AT2R. AT1R is expressed throughout an entire life cycle and is primarily responsible for regulating the blood pressure. Its blockers are widely used clinically as antihypertensive agents. AT1R can mediate most of physiological effects of Ang II. AT2R is primarily expressed in embryonic tissues and is related to blood pressure regulation, nerve growth, pain control, and myocardial regeneration. Drugs targeting AT2R can improve cardiovascular function and alleviate neuropathic pain. However, an expression of AT2R is significantly upregulated under pathological conditions, such as vascular injury, wound healing, and heart failure.

Recently, it has been shown that AT2R agonists may be used to treat and/or prevent gastrointestinal tract diseases such as dyspepsia and irritable bowel syndrome, as well as multiple organ failure. There remains a need for effective and/or selective AT2R agonists that are expected to be used for the aforementioned diseases.

### SUMMARY

An object of the present disclosure is to provide a thienylsulfonyl carbamate as an AT2R agonist and use thereof. The thienylsulfonyl carbamate has a structure represented in a first aspect of the present disclosure. The thienylsulfonyl carbamate can be used to prepare a medicament, a pharmaceutical composition, or a formulation for treating and/or preventing an AT2R-related disease or disorder; or can be used to treat and/or prevent an AT2R-related disease or disorder.

In a first aspect of the present disclosure, there is provided a compound, or a tautomer, stereoisomer, hydrate, solvate, pharmaceutically acceptable salt, or prodrug thereof. The compound has a structure represented by Formula III:

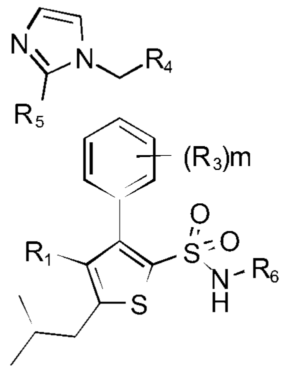

III,

where:

- $R_1$ is selected from halogen, $C_1$ to $C_6$ alkyl, and halogenated $C_1$ to $C_6$ alkyl;
- each $R_3$ is independently selected from hydrogen, halogen, cyano, $C_1$ to $C_6$ alkyl, and $C_3$ to $C_6$ cycloalkyl, wherein the $C_1$ to $C_6$ alkyl and the $C_3$ to $C_6$ cycloalkyl are optionally substituted with one, two, three, or four substituents selected from halogen, hydroxyl, amino, cyano, $C_1$ to $C_6$ alkyl, and $C_3$ to $C_7$ cycloalkyl, and wherein when a plurality of substituents are present, the plurality of substituents are the same or different;
- m is selected from 1, 2, 3, and 4;
- $R_4$ is selected from hydrogen, $C_1$ to $C_6$ alkyl, and halogenated $C_1$ to $C_6$ alkyl;
- $R_5$ is selected from halogen, $C_1$ to $C_6$ alkyl, and 4- to 7-membered heterocycloalkyl, wherein the $C_1$ to $C_6$ alkyl is substituted with one, two, three, or four substituents selected from halogen, hydroxyl, and 4- to 7-membered heterocycloalkyl, wherein the 4- to 7-membered heterocycloalkyl is substituted with zero, one, two, three, or four substituents selected from halogen, hydroxyl, cyano, $C_1$ to $C_6$ alkyl, and 4- to 7-membered heterocycloalkyl, and wherein when a plurality of substituents are present, the plurality of substituents are the same or different;
- $R_6$ is selected from a 5- to 7-membered heteroaromatic ring and -C(O)-X-$R_2$;
- X is selected from NRa and O;
- $R_2$ is $C_1$ to $C_6$ alkyl, 4- to 7-membered heterocycloalkyl, or a 5- to 7-membered heteroaromatic ring, wherein the $C_1$ to $C_6$ alkyl is substituted with zero, one, two, three, or four substituents selected from halogen, hydroxyl, $C_1$ to $C_3$ alkyl, 3- to 7-membered heterocycloalkyl, and a 5- to 7-membered heteroaromatic ring, wherein the 4- to 7-membered heterocycloalkyl and the 5- to 7-membered heteroaromatic ring are substituted with zero, one, two, three, or four substituents selected from halogen, hydroxyl, cyano, amino, $C_1$ to $C_6$ alkyl, halogenated $C_1$ to $C_6$ alkyl, $C_1$ to $C_6$ alkoxy, halogenated $C_1$ to $C_6$ alkoxy, $C_3$ to $C_7$ cycloalkyl, a 5- to 7-membered heteroaromatic ring, and 3- to 7-membered heterocycloalkyl, and wherein when a plurality of substituents are present, the plurality of substituents are the same or different;
- Ra is selected from hydrogen and $C_1$ to $C_3$ alkyl, or Ra and $R_2$ together with nitrogen atoms bonded thereto form a 5- to 8-membered heterocycloalkyl bicyclic system;
- when $R_5$ is selected from halogen and the halogen is Cl, when $R_3$ is selected from hydrogen, when $R_1$ is selected from $C_1$ to $C_6$ alkyl and the $C_1$ to $C_6$ alkyl is methyl, and when $R_6$ is selected from -C(O)-O-$R_2$, $R_2$ is not methyl; and when $R_6$ is selected from the 5- to 7-membered heteroaromatic ring, $R_3$ is hydrogen or F.

In the present disclosure, the term "group A is selected from A1, ..., and An" means that A is selected from one of A1, ..., and An, which is synonymous with the term "group A is selected from A1, ..., or An".

According to an embodiment of the present disclosure, provided is a compound represented by Formula I, or a tautomer, stereoisomer, hydrate, solvate, pharmaceutically acceptable salt, or prodrug thereof:

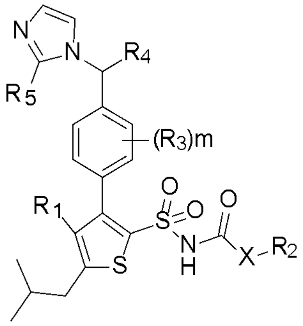

I,

where:

$R_1$ is selected from halogen, $C_1$ to $C_6$ alkyl, and halogenated $C_1$ to $C_6$ alkyl;
X is selected from NRa and O;
$R_2$ is $C_1$ to $C_6$ alkyl or a 5- to 7-membered heteroaromatic ring, wherein the $C_1$ to $C_6$ alkyl is substituted with zero, one, two, three, or four substituents selected from halogen, hydroxyl, $C_1$ to $C_3$ alkyl, and a 5- to 7-membered heteroaromatic ring, and wherein when a plurality of substituents are present, the plurality of substituents are the same or different;
each $R_3$ is independently selected from hydrogen, halogen, cyano, $C_1$ to $C_6$ alkyl, and $C_3$ to $C_6$ cycloalkyl, wherein the $C_1$ to $C_6$ alkyl and the $C_3$ to $C_6$ cycloalkyl are optionally substituted with one, two, three, or four substituents selected from halogen, hydroxyl, amino, cyano, $C_1$ to $C_6$ alkyl, and $C_3$ to $C_7$ cycloalkyl, and wherein when a plurality of substituents are present, the plurality of substituents are the same or different;
m is selected from 1, 2, 3, and 4;
$R_4$ is selected from hydrogen, $C_1$ to $C_6$ alkyl, and halogenated $C_1$ to $C_6$ alkyl;
$R_5$ is selected from halogen and $C_1$ to $C_6$ alkyl, wherein the $C_1$ to $C_6$ alkyl is substituted with one, two, three, or four substituents selected from halogen and hydroxyl, and wherein when a plurality of substituents are present, the plurality of substituents are the same or different;
Ra is selected from hydrogen and $C_1$ to $C_3$ alkyl, or Ra and $R_2$ together with nitrogen atoms bonded thereto form a 5- to 8-membered heterocycloalkyl bicyclic system;
when $R_5$ is selected from halogen and the halogen is Cl, when $R_3$ is selected from hydrogen, when $R_1$ is selected from $C_1$ to $C_6$ alkyl and the $C_1$ to $C_6$ alkyl is methyl, and when X is selected from O, $R_2$ is not methyl.

In a preferred embodiment of the present disclosure, the compound is selected from the following structure:

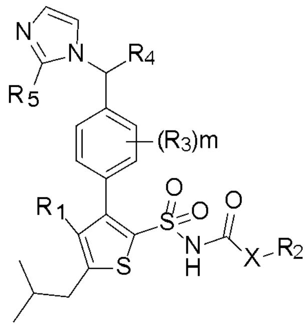

I, ˈ

where:

$R_1$ is selected from halogen, $C_1$ to $C_6$ alkyl, and halogenated $C_1$ to $C_6$ alkyl;
X is selected from NRa and O;
$R_2$ is $C_1$ to $C_6$ alkyl, wherein the $C_1$ to $C_6$ alkyl is substituted with zero, one, two, three, or four substituents selected from halogen, hydroxyl, and $C_1$ to $C_3$ alkyl, and wherein when a plurality of substituents are present, the plurality of substituents are the same or different;
$R_3$ is selected from hydrogen, halogen, cyano, $C_1$ to $C_6$ alkyl, and $C_3$ to $C_6$ cycloalkyl, wherein the $C_1$ to $C_6$ alkyl and the $C_3$ to $C_6$ cycloalkyl are optionally substituted with one, two, three, or four substituents selected from halogen, hydroxyl, amino, cyano, $C_1$ to $C_6$ alkyl, and $C_3$ to $C_7$ cycloalkyl, and wherein when a plurality of substituents are present, the

plurality of substituents are the same or different;
m is selected from 1, 2, 3, and 4;
$R_4$ is selected from hydrogen, $C_1$ to $C_6$ alkyl, and halogenated $C_1$ to $C_6$ alkyl;
$R_5$ is selected from halogen and $C_1$ to $C_6$ alkyl, wherein the $C_1$ to $C_6$ alkyl is substituted with one, two, three, or four substituents selected from halogen and hydroxyl, and wherein when a plurality of substituents are present, the plurality of substituents are the same or different;
Ra is selected from hydrogen and $C_1$ to $C_3$ alkyl;
when $R_5$ is selected from halogen and the halogen is Cl, when $R_3$ is selected from hydrogen, when $R_1$ is selected from $C_1$ to $C_6$ alkyl and the $C_1$ to $C_6$ alkyl is methyl, and when X is selected from O, $R_2$ is not methyl.

In a preferred embodiment of the present disclosure, $R_6$ is selected from the 5- to 7-membered heteroaromatic ring, a heteroatom in the 5- to 7-membered heteroaromatic ring being N.

In a preferred embodiment of the present disclosure, $R_6$ is selected from

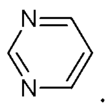

In a preferred embodiment of the present disclosure, the compound has a structure represented by Formula IV:

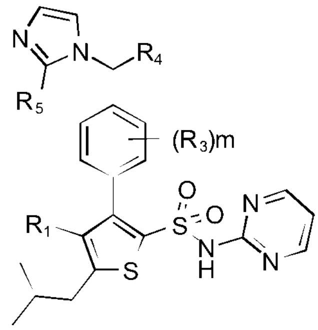

IV.

In a preferred embodiment of the present disclosure, when $R_6$ is selected from the 5- to 7-membered heteroaromatic ring, R3 is hydrogen or F.

In a preferred embodiment of the present disclosure, when $R_6$ is selected from the 5- to 7-membered heteroaromatic ring, R3 is hydrogen.

In a preferred embodiment of the present disclosure, $R_1$ is selected from halogen, $C_1$ to $C_3$ alkyl, and halogenated $C_1$ to $C_3$ alkyl.

In a preferred embodiment of the present disclosure, $R_1$ is selected from F, Cl, Br, I, methyl, ethyl, propyl, isopropyl, $CH_2F$, $CHF_2$, and $CF_3$.

In a preferred embodiment of the present disclosure, $R_1$ is selected from F, Cl, and methyl.

In a preferred embodiment of the present disclosure, $R_1$ is selected from methyl.

In a preferred embodiment of the present disclosure, X is selected from NRa and O, and Ra is selected from hydrogen and $C_1$ to $C_3$ alkyl.

In a preferred embodiment of the present disclosure, X is selected from NRa and O, and Ra is selected from hydrogen and methyl.

In a preferred embodiment of the present disclosure, X is selected from O.

In a preferred embodiment of the present disclosure, X is selected from NRa, and Ra is selected from hydrogen and methyl.

In a preferred embodiment of the present disclosure, X is selected from NRa, and Ra and $R_2$ together with nitrogen atoms bonded thereto form a 5- to 8-membered heterocycloalkyl bicyclic system. The 5- to 8-membered heterocycloalkyl bicyclic system contains one, two, or three heteroatoms selected from N, O, and S.

In a preferred embodiment of the present disclosure, X is selected from NRa, and Ra and $R_2$ together with nitrogen atoms bonded thereto form a 5- to 8-membered heterocyclic spiro ring. The 5- to 8-membered heterocyclic spiro ring contains one, two, or three heteroatoms selected from N, O, and S.

In a preferred embodiment of the present disclosure, X is selected from NRa, and Ra and $R_2$ together with nitrogen atoms bonded thereto form

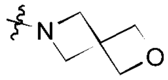

.

In a preferred embodiment of the present disclosure, X is selected from N, and Ra and $R_2$ together with nitrogen atoms bonded thereto form a 5- to 8-membered heterocycloalkyl bicyclic system. The 5- to 8-membered heterocycloalkyl bicyclic system contains one, two, or three heteroatoms selected from N, O, and S.

In a preferred embodiment of the present disclosure, X is selected from N, and Ra and $R_2$ together with nitrogen atoms bonded thereto form a 5- to 8-membered heterocyclic spiro ring. The 5- to 8-membered heterocyclic spiro ring contains one, two, or three heteroatoms selected from N, O, and S.

In a preferred embodiment of the present disclosure, X is selected from N, and Ra and $R_2$ together with nitrogen atoms bonded thereto form

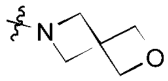

.

In a preferred embodiment of the present disclosure, the compound is selected from the following structure:

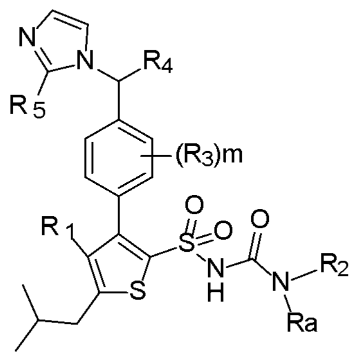

II,

where:

Ra is selected from hydrogen, and $R_2$ is selected from 5- to 7-membered heteroaromatic ring and $C_1$ to $C_4$ alkyl-5- to 7-membered heteroaromatic ring; or Ra and $R_2$ together with nitrogen atoms bonded thereto form a 5- to 8-membered heterocycloalkyl bicyclic system; and

$R_1$, $R_3$, $R_4$, $R_5$, and m are defined as described in the first aspect of the present disclosure.

In a preferred embodiment of the present disclosure, $R_2$ is selected from $C_1$ to $C_4$ alkyl, 4- to 7-membered heterocycloalkyl, and a 5- to 7-membered heteroaromatic ring, wherein the $C_1$ to $C_4$ alkyl is substituted with zero, one, two, three, or four substituents selected from halogen, hydroxyl, $C_1$ to $C_3$ alkyl, 3- to 7-membered heterocycloalkyl, a 5- to 7-membered heteroaromatic ring, and 4- to 7-membered heterocycloalkyl, wherein the 4- to 7-membered heterocycloalkyl and the 5- to 7-membered heteroaromatic ring are substituted with zero, one, two, three, or four substituents selected from halogen, hydroxyl, $C_1$ to $C_3$ alkyl, and a 5- to 7-membered heteroaromatic ring, and wherein when a plurality of substituents are present, the plurality of substituents are the same or different.

In a preferred embodiment of the present disclosure, $R_2$ is selected from $C_1$ to $C_4$ alkyl, 4- to 7-membered heterocycloalkyl, -$C_1$ to $C_4$ alkyl-4- to 7-membered heterocycloalkyl, -$C_1$ to $C_4$ alkyl-5- to 7-membered heteroaromatic ring, and -4- to 7-membered heterocycloalkyl-$C_1$ to $C_4$ alkyl, wherein the $C_1$ to $C_4$ alkyl is substituted with zero, one, two, three, or four substituents selected from halogen, hydroxyl, and $C_1$ to $C_3$ alkyl.

In a preferred embodiment of the present disclosure, $R_2$ is selected from methyl, ethyl, propyl, isopropyl, butyl, isobutyl, azetidinyl, oxiranyl, oxetanyl, tetrahydrofuran, -$C_1$ to $C_4$ alkyl-azetidinyl, -$C_1$ to $C_4$ alkyl-oxiranyl, -$C_1$ to $C_4$ alkyl-oxetanyl, -$C_1$ to $C_4$ alkyltetrahydrofuran, -$C_1$ to $C_4$ alkyl-pyridinyl, pyrimidinyl, pyridinyl, pyridazinyl, and pyrazinyl, wherein the methyl, ethyl, propyl, isopropyl, butyl, and isobutyl are substituted with zero, one, two, three, or four substituents selected from halogen, hydroxyl, and $C_1$ to $C_3$ alkyl.

In a preferred embodiment of the present disclosure, $R_2$ is selected from methyl, ethyl, propyl, butyl, isobutyl, pyridinyl, pyrimidinyl, -methyl-pyridinyl, -methyl-oxetanyl, and -oxetanyl-methyl, wherein the ethyl, propyl, isopropyl, butyl, and isobutyl are substituted with hydroxyl.

In a preferred embodiment of the present disclosure, $R_2$ is selected from methyl, ethyl,

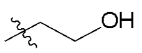, 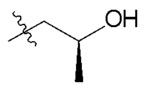, 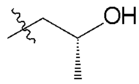, 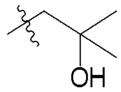,

butyl, pyrimidinyl,

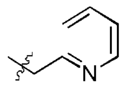,

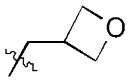, and 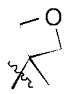.

In a preferred embodiment of the present disclosure, $R_2$ is selected from $C_1$ to $C_4$ alkyl or a 5- to 7-membered heteroaromatic ring, wherein the $C_1$ to $C_4$ alkyl and the 5- to 7-membered heteroaromatic ring are substituted with zero, one, two, three, or four substituents selected from halogen, hydroxyl, $C_1$ to $C_3$ alkyl, and a 5- to 7-membered heteroaromatic ring, and wherein when a plurality of substituents are present, the plurality of substituents are the same or different.

In a preferred embodiment of the present disclosure, $R_2$ is selected from methyl, ethyl, propyl, isopropyl, butyl, isobutyl, pyrimidinyl, pyridinyl, pyridazinyl, and pyrazinyl, and $R_2$ is substituted with zero, one, two, three, or four substituents selected from halogen, hydroxyl, $C_1$ to $C_3$ alkyl, pyridinyl, pyrimidinyl, pyridazinyl, and pyrazinyl.

In a preferred embodiment of the present disclosure, $R_2$ is selected from methyl, ethyl,

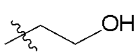, 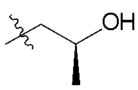, 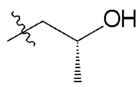, 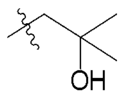,

butyl, pyrimidinyl, and

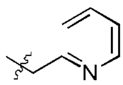.

In a preferred embodiment of the present disclosure, $R_2$ is selected from $C_1$ to $C_4$ alkyl, wherein the $C_1$ to $C_4$ alkyl is substituted with zero, one, two, three, or four substituents selected from halogen, hydroxyl, and $C_1$ to $C_3$ alkyl; and wherein when a plurality of substituents are present, the plurality of substituents are the same or different.

In a preferred embodiment of the present disclosure, $R_2$ is selected from methyl, ethyl, propyl, isopropyl, butyl, and isobutyl, and $R_2$ is substituted with one, two, three, or four substituents selected from halogen, hydroxyl, and $C_1$ to $C_3$ alkyl.

In a preferred embodiment of the present disclosure, $R_2$ is selected from methyl, ethyl,

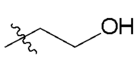, 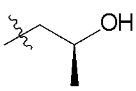, 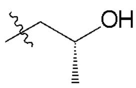, 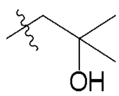,

and butyl.

In a preferred embodiment of the present disclosure, $R_3$ is selected from hydrogen, halogen, cyano, $C_1$ to $C_3$ alkyl, and $C_3$ to $C_4$ cycloalkyl, wherein the $C_1$ to $C_3$ alkyl and the $C_3$ to $C_4$ cycloalkyl are optionally substituted with one, two, three, or four substituents selected from halogen, hydroxyl, amino, cyano, $C_1$ to $C_6$ alkyl, and $C_3$ to $C_7$ cycloalkyl, and wherein when a plurality of substituents are present, the plurality of substituents are the same or different. m is selected from 1, 2, 3, and 4.

In a preferred embodiment of the present disclosure, $R_3$ is selected from hydrogen, F, Cl, methyl, and cyano, and m is selected from 1, 2, 3, and 4.

In a preferred embodiment of the present disclosure, $R_3$ is selected from hydrogen, F, Cl, Br, I, and methyl, and m is selected from 1, 2, 3, and 4.

In a preferred embodiment of the present disclosure, $R_3$ is selected from hydrogen, F, and Cl.

In a preferred embodiment of the present disclosure, $R_3$ is selected from hydrogen and F.

In a preferred embodiment of the present disclosure, $R_4$ is selected from hydrogen, $C_1$ to $C_3$ alkyl, and halogenated $C_1$ to $C_3$ alkyl.

In a preferred embodiment of the present disclosure, $R_4$ is selected from hydrogen and $C_1$ to $C_3$ alkyl.

In a preferred embodiment of the present disclosure, $R_4$ is selected from hydrogen and methyl.

In a preferred embodiment of the present disclosure, $R_4$ is selected from hydrogen.

In a preferred embodiment of the present disclosure, $R_5$ is selected from halogen, $C_1$ to $C_3$ alkyl, and 4- to 5-membered heterocycloalkyl, wherein the $C_1$ to $C_3$ alkyl is substituted with one, two, three, or four substituents selected from halogen, hydroxyl, and 4- to 7-membered heterocycloalkyl, wherein the 4- to 5-membered heterocycloalkyl is substituted with zero, one, two, three, or four substituents selected from halogen, hydroxyl, cyano, and $C_1$ to $C_6$ alkyl, and wherein when a plurality of substituents are present, the plurality of substituents are the same or different.

In a preferred embodiment of the present disclosure, $R_5$ is selected from F, Cl, $CF_3$,

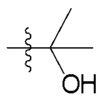 , and 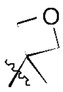 .

In a preferred embodiment of the present disclosure, $R_5$ is selected from Cl, $CF_3$,

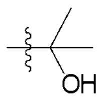 , and 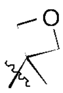 .

In a preferred embodiment of the present disclosure, $R_5$ is selected from halogen and $C_1$ to $C_3$ alkyl, wherein the $C_1$ to $C_3$ alkyl is substituted with one, two, three, or four substituents selected from halogen and hydroxyl, and wherein when a plurality of substituents are present, the plurality of substituents are the same or different.

In a preferred embodiment of the present disclosure, $R_5$ is selected from F, Cl, Br, I, $CF_3$, and

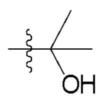 .

In a preferred embodiment of the present disclosure, $R_5$ is selected from Cl, $CF_3$, and

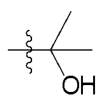 .

In a preferred embodiment of the present disclosure, when $R_5$ is selected from halogen and the halogen is Cl, when $R_3$ is selected from hydrogen, when $R_1$ is selected from $C_1$ to $C_6$ alkyl and the $C_1$ to $C_6$ alkyl is methyl, and when X is selected from O, $R_2$ is selected from methyl , $C_2$ to $C_6$ alkyl, and 4- to 7-membered heterocycloalkyl, wherein when $R_2$ is the methyl, the methyl is substituted with one, two, three, or four substituents selected from halogen, hydroxyl, $C_1$ to $C_3$ alkyl, and 3- to 7-membered heterocycloalkyl, wherein when $R_2$ is the $C_2$ to $C_6$ alkyl, the $C_2$ to $C_6$ alkyl is substituted with zero, one, two, three, or four substituents selected from halogen, hydroxyl, $C_1$ to $C_3$ alkyl, and 3- to 7-membered heterocycloalkyl, wherein when $R_2$ is the 4- to 7-membered heterocycloalkyl, the 4- to 7-membered heterocycloalkyl is substituted with zero, one, two, three, or four substituents selected from halogen, hydroxyl, and $C_1$ to $C_3$ alkyl, and wherein when a plurality of substituents are present, the plurality of substituents are the same or different; and preferably, $R_2$ is selected from ethyl,

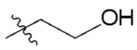, 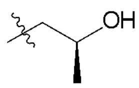, 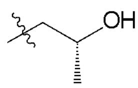, 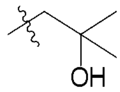,

butyl,

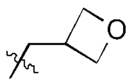, and 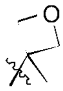.

In a preferred embodiment of the present disclosure, when $R_5$ is selected from halogen and the halogen is Cl, when $R_3$ is selected from hydrogen, when $R_1$ is selected from $C_1$ to $C_6$ alkyl and the $C_1$ to $C_6$ alkyl is methyl, and when X is selected from O, $R_2$ is selected from methyl and $C_2$ to $C_6$ alkyl, wherein when $R_2$ is the methyl, the methyl is substituted with one, two, three, or four substituents selected from halogen, hydroxyl, and $C_1$ to $C_3$ alkyl, wherein when $R_2$ is the $C_2$ to $C_6$ alkyl, the $C_2$ to $C_6$ alkyl is substituted with zero, one, two, three, or four substituents selected from halogen, hydroxyl, and $C_1$ to $C_3$ alkyl, and wherein when a plurality of substituents are present, the plurality of substituents are the same or different; and

preferably, $R_2$ is selected from ethyl,

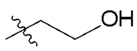, 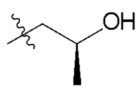, 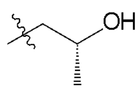, 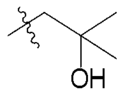,

and butyl.

In a preferred embodiment of the present disclosure, when $R_5$ is selected from Cl, when $R_3$ is selected from hydrogen, when $R_4$ is selected from hydrogen, when $R_1$ is selected from methyl, and when X is selected from O, $R_2$ is selected from ethyl, butyl,

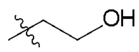,

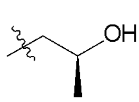, 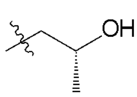, 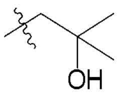, 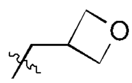, and 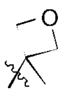.

In a preferred embodiment of the present disclosure, when $R_3$ is selected from hydrogen, when $R_4$ is selected from hydrogen, when $R_1$ is selected from methyl, and when X is selected from NH, $R_2$ is selected from

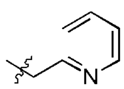.

In a preferred embodiment of the present disclosure, when $R_1$ is selected from methyl, when $R_3$ is selected from hydrogen and F, and when $R_4$ is selected from hydrogen, $R_6$ is selected from -C(O)-O-$R_2$, $R_2$ is selected from methyl, butyl,

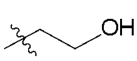, 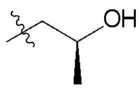,

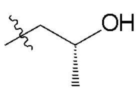, 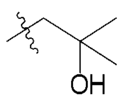, 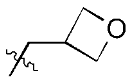, and 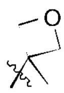,

and $R_5$ is selected from $-CF_3$, Cl,

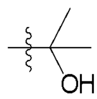

,

and

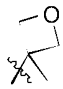

.

In a preferred embodiment of the present disclosure, when $R_1$ is selected from methyl, when $R_3$ is selected from Cl, and when $R_4$ is selected from hydrogen, $R_6$ is selected from -C(O)-O-$R_2$, $R_2$ is selected from butyl, and $R_5$ is selected from $-CF_3$ and Cl.

In a preferred embodiment of the present disclosure, when $R_1$ is selected from methyl, when $R_3$ is selected from hydrogen, and when $R_4$ is selected from hydrogen, $R_6$ is selected from a 5- to 7-membered heteroaromatic ring, wherein a heteroatom in the 5- to 7-membered heteroaromatic ring is N.

In a preferred embodiment of the present disclosure, when $R_1$ is selected from methyl, when $R_3$ is selected from hydrogen, and when $R_4$ is selected from hydrogen, $R_6$ is selected from a 5- to 7-membered heteroaromatic ring, wherein a heteroatom in the 5- to 7-membered heteroaromatic ring is N, and $R_5$ is selected from $-CF_3$ and Cl.

In a preferred embodiment of the present disclosure, when $R_1$ is selected from methyl, when $R_3$ is selected from hydrogen, and when $R_4$ is selected from hydrogen, $R_6$ is selected from a 5- to 7-membered heteroaromatic ring, wherein a heteroatom in the 5- to 7-membered heteroaromatic ring is N, and $R_5$ is selected from Cl.

In a preferred embodiment of the present disclosure, $R_1$ is selected from methyl, $R_3$ is selected from hydrogen, $R_4$ is selected from hydrogen, X is selected from NRa, and Ra and $R_2$ together with nitrogen atoms bonded thereto form

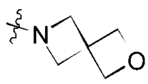

.

In a preferred embodiment of the present disclosure, when $R_1$ is selected from methyl, when $R_2$ is selected from methyl, when $R_4$ is selected from hydrogen, and when X is selected from O, $R_3$ is selected from F and Cl, or $R_5$ is selected from $-CF_3$ and

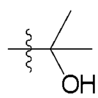

.

In a preferred embodiment of the present disclosure, for the compound, or the tautomer, stereoisomer, hydrate, solvate, pharmaceutically acceptable salt, or prodrug thereof, the compound includes:

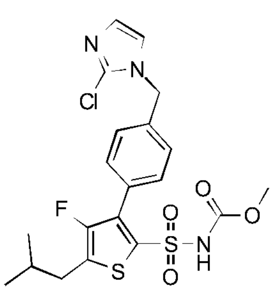

I-1,

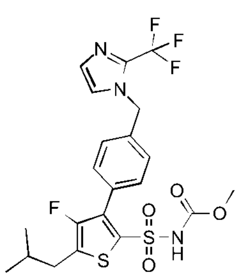

I-2,

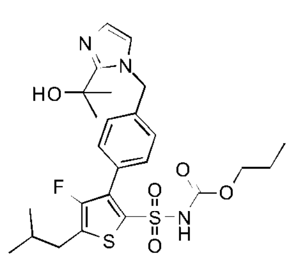

I-3,

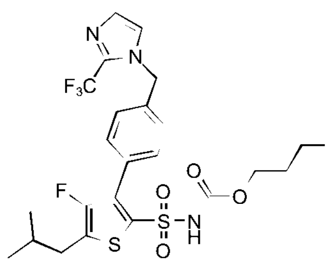
I-4,

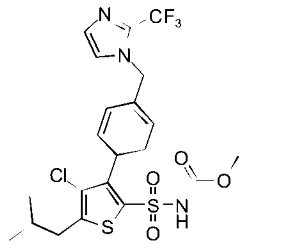
I-5,

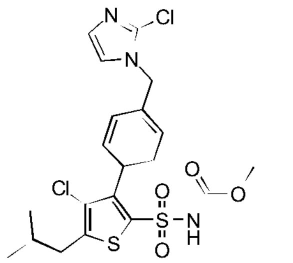
I-6,

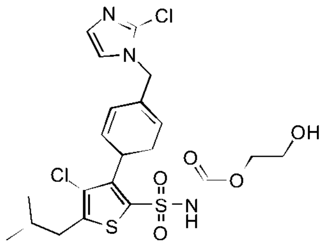
I-7,

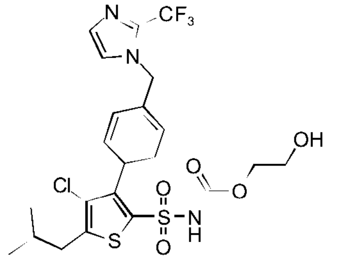
I-8,

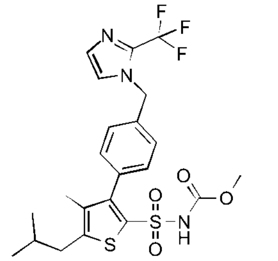
I-9,

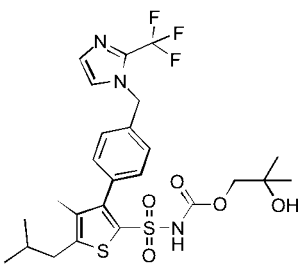
I-10,

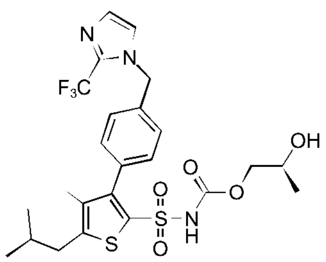
I-11,

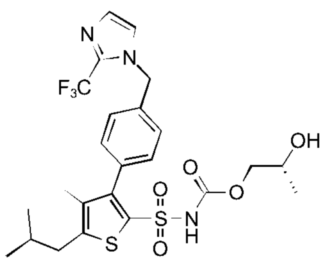
I-12,

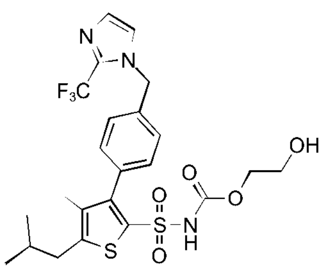
I-13,

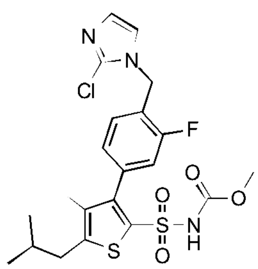
I-14,

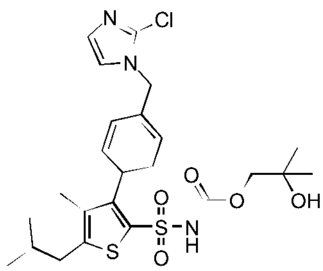
I-15,

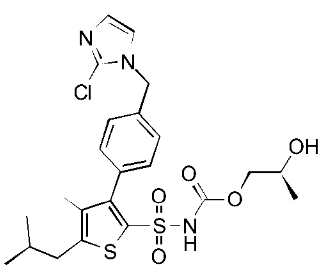
I-16,

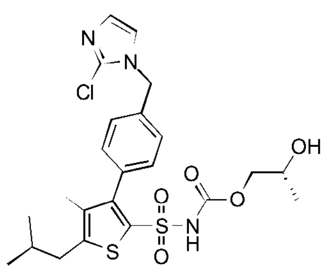
I-17,

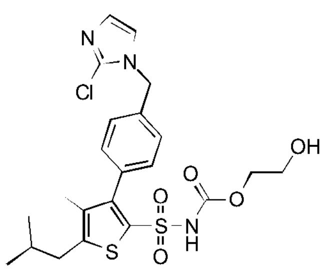
I-18,

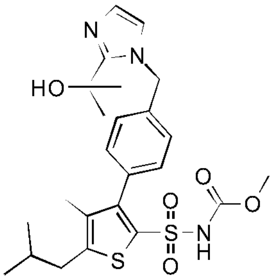

I-19,

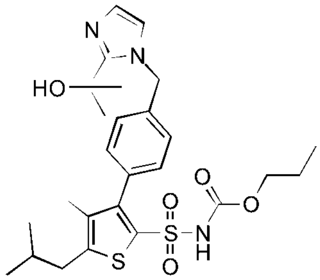

I-20,

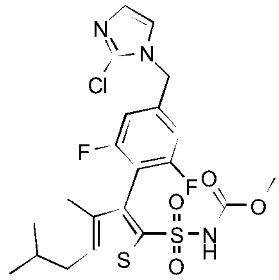

I-21,

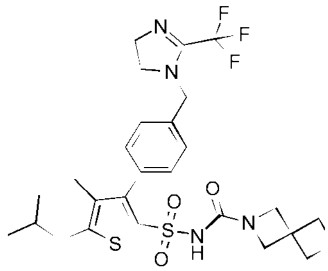

I-22,

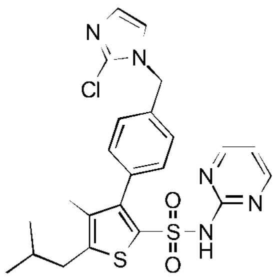

I-23,

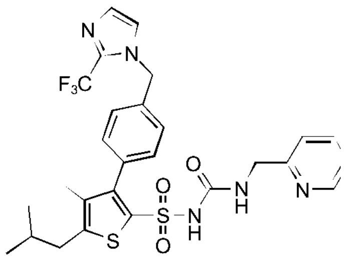

I-24,

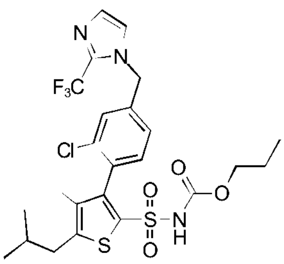

I-25,

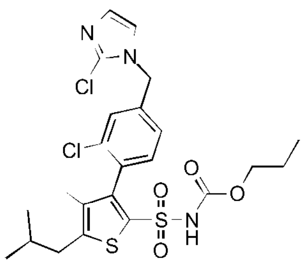

I-26,

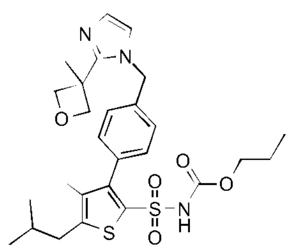

I-27,

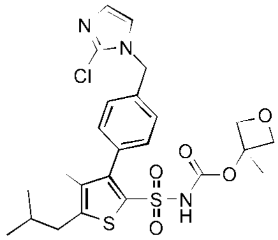

I-28, or

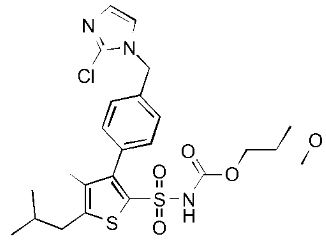

I-29.

In a second aspect of the present disclosure, provided is a pharmaceutical composition. The pharmaceutical composition includes the compound, or the tautomer, stereoisomer, hydrate, solvate, pharmaceutically acceptable salt, or prodrug thereof according to the first aspect of the present disclosure.

In a preferred embodiment of the present disclosure, the pharmaceutical composition further includes a pharmaceutically acceptable carrier.

In a third aspect of the present disclosure, provided is use of the compound, or the tautomer, stereoisomer, hydrate, solvate, pharmaceutically acceptable salt, or prodrug thereof according to the first aspect of the present disclosure, or the pharmaceutical composition the second aspect of the present disclosure. The use includes:

acting as an AT2R agonist; and/or preventing and/or treating a disease of insufficient endogenous generation of Angiotensin II, Ang II; and/or
preventing and/or treating a disease where an increased effect of Ang II is desired or required; and/or
preparing an AT2R agonist; and/or

preparing a medicament, pharmaceutical composition, or formulation for preventing and/or treating a disease where AT2R is expressed and a stimulation of AT2R is desired or necessary.

Provided is the compound, or the tautomer, stereoisomer, hydrate, solvate, pharmaceutically acceptable salt, or prodrug thereof according to the first aspect of the present disclosure, or the pharmaceutical composition according to the second aspect of the present disclosure for treatment of a disease of gastrointestinal tract, cardiovascular system, respiratory tract, kidney, eyes, female reproductive system, or central nervous system (CNS).

Gastrointestinal tract diseases to be mentioned include esophagitis, Barrett's esophagus, gastric ulcer, duodenal ulcer, dyspepsia (including non-ulcer dyspepsia), gastroesophageal reflux, allergic bowel syndrome, inflammatory enteritis, pancreatitis, liver disease (such as hepatitis), gallbladder disease, multiple organ failure, sepsis. Other gastrointestinal tract diseases to be mentioned include xerostomia, gastritis, gastric stasis, hyperacidity, biliary tract disease, abdominal disease, segmental ileitis, ulcerative colitis, diarrhea, constipation, acute colic, dysphagia, nausea, vomiting, and Sjögren's syndrome.

Respiratory tract diseases to be mentioned include inflammatory diseases such as asthma, obstructive pulmonary disease (such as chronic obstructive pulmonary disease), pneumonia, pulmonary hypertension, adult respiratory distress syndrome, and idiopathic pulmonary fibrosis.

Kidney diseases to be mentioned include renal failure, nephritis, and renal hypertension.

Eye diseases to be mentioned include diabetic retinopathy, retinopathy of prematurity, and retinal microvascularization.

Female reproductive system diseases to be mentioned include ovulatory mechanism disorder.

Cardiovascular diseases to be mentioned include hypertension, myocardial hypertrophy, heart failure, atherosclerosis, arterial thrombosis, venous thrombosis, endothelial dysfunction, endothelial damage, stenosis after balloon dilation, angiogenesis, diabetic complications, microvascular dysfunction, angina pectoris, arrhythmia, intermittent claudication, pre-eclampsia, myocardial infarction, reinfarction, ischemic damage, erectile dysfunction, and neointimal hyperplasia.

CNS diseases to be mentioned include cognitive dysfunction, feeding dysfunction, thirst, stroke, cerebral hemorrhage, cerebral embolism, and cerebral infarction.

The compound, or the tautomer, stereoisomer, hydrate, solvate, pharmaceutically acceptable salt, or prodrug thereof according to the first aspect of the present disclosure, or the pharmaceutical composition according to the second aspect of the present disclosure can be further used for regulation of growth metabolism and proliferation, for example, for the treatment of hypertrophy, prostatic hyperplasia, autoimmune disease, psoriasis, obesity, nerve regeneration, ulcers, inhibition of adipose tissue hypertrophy, stem cell differentiation and proliferation, cancer (such as gastrointestinal tract cancer and lung cancer), apoptosis, tumors (generally), proliferative diabetes, nerve damage, or organ rejection.

The compound, or the tautomer, stereoisomer, hydrate, solvate, pharmaceutically acceptable salt, or prodrug thereof according to the first aspect of the present disclosure, or the pharmaceutical composition according to the second aspect of the present disclosure is suitable for the treatment and/or preventive treatment of the aforementioned diseases.

A fourth aspect of the present disclosure provides a method for treating a disease, wherein the disease is: a disease of insufficient endogenous generation of Ang II; and/or a disease where an increased effect of Ang II is desired or necessary; and/or a disease where AT2R is expressed and a stimulation of AT2R is desired or necessary. The method includes: administering a therapeutically effective amount of the compound, or the tautomer, stereoisomer, hydrate, solvate, pharmaceutically acceptable salt, or prodrug thereof according to the first aspect of the present disclosure, or the pharmaceutical composition according to the second aspect of the present disclosure, to a person suffering from or susceptible to the disease.

According to an embodiment of the present disclosure, the disease type refers to the description for the use according to the third aspect of the present disclosure.

Additional aspects and advantages of the embodiments of the present disclosure will be provided at least in part in the following description, or will become apparent in part from the following description, or can be learned from the practice of the embodiments of the present disclosure.

## Terms and Definitions

Unless otherwise stated, the definitions of groups and terms described in the specification and claims include actual definitions, exemplary definitions, preferred definitions, definitions recorded in tables, and definitions of specific compounds in the examples, etc., which may be arbitrarily combined and integrated with each other. The group definitions and compound structures that are combined and integrated should fall within the scope of the present disclosure.

Unless defined otherwise, all technical and scientific terms herein have the same meaning as commonly understood by one of ordinary skill in the art to which the claimed subject matter belongs. The patents, patent applications, publications cited herein are hereby incorporated by reference in their entireties, unless stated otherwise. When a term has

multiple definitions, the definition in this section will prevail.

It should be understood that the foregoing general description and the following detailed description are illustrative and merely for explanation, rather than limiting the subject matter of the present disclosure in any way. In the present disclosure, unless specifically stated otherwise, the use of the singular also includes the plural. It must be noted that, as used in this specification and the claims, the singular forms include plural referents unless the context clearly dictates otherwise. It should also be noted that the use of "or" or "either" means "and/or" unless stated otherwise. Furthermore, use of the term "include", as well as other forms, such as "comprising", "including", and "containing", is not for limitation.

Definitions of standard chemical terms may be found in reference document (including "ADVANCED ORGANIC CHEMISTRY 4THED.", Carey and Sundberg, Vols. A(2000)and B(2001), Plenum Press, New York). Unless otherwise indicated, conventional methods in the related art, for example, mass spectroscopy, NMR, IR and UV/VIS spectroscopy, and pharmacological methods, are employed. Unless specific definitions are set forth, the terms in the related description of analytical chemistry, organic synthetic chemistry, and medicinal and medicinal chemistry are those known in the art. Standard techniques may be used in chemical synthesis, chemical analysis, pharmaceutical preparation, formulation and delivery, and treatment of patients. For example, reactions and purifications can be performed using the manufacturer's instructions of the kit, or in a manner well known in the related art or as described herein. The techniques and procedures described above may generally be performed with conventional methods well known in the art according to the description in a number of general and more specific documents cited and discussed throughout the present description. Throughout the description, groups and substituents thereof can be chosen by one skilled in the field to provide stable moieties and compounds.

Where substituent groups are depicted by conventional chemical formulae, written from left to right, the substituent groups also encompass the chemically equivalent substituents that would result from writing the structural formula from right to left. For example, $CH_2O$ is equivalent to $OCH_2$. As used herein,

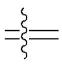 or 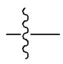

refers to the point of attachment of a group. As used herein, "$R_1$", "R1", and "$R^1$" have the same meaning and are interchangeable. For other symbols such as $R_2$, similar definitions have the same meanings.

The section headings used herein are for organizational purposes only and are not to be construed as limiting the subject matter described. All documents or portions of documents cited in the present disclosure, including but not limited to patents, patent applications, articles, books, manuals, and treatises, are incorporated herein by reference in their entireties.

In addition to the foregoing, the following terms, when used in the specification and claims of the present disclosure, have the meanings indicated below, unless otherwise specifically stated.

As used herein, numerical ranges recited in the specification and claims, when interpreted as "integers", should be understood as including both endpoints of the range and each integer within the range. For example, "an integer ranging from 1 to 6" should be understood as reciting each integer of 1, 2, 3, 4, 5, and 6.

In the present disclosure, "AT2 receptor" and "AT2R" have the same definition.

In the present disclosure, the term "halogen" refers to fluorine, chlorine, bromine, and iodine, either alone or as part of other substituents.

In the present disclosure, the term "amino" refers to $-NH_2$, either alone or as part of other substituents.

In the present disclosure, the term "hydroxy" refers to -OH, either alone or as part of other substituents.

As used herein, the term "alkyl", either alone or as part of other substituents, means a straight or branched hydrocarbon chain group consisting solely of carbon and hydrogen atoms, containing no unsaturated bonds, and having, for example, 1 to 6 carbon atoms and connected to the rest of the molecule by a single bond. Examples of alkyl include, but are not limited to, methyl, ethyl, n-propyl, isopropyl, n-butyl, isobutyl, tert-butyl, pentyl, isopentyl, neopentyl, and hexyl. Alkyl may be unsubstituted or substituted with one or more suitable substituents. Alkyl may also be an isotopic isomer of the naturally abundant alkyl enriched in an isotope of carbon and/or hydrogen (i.e., deuterium or tritium). As used herein, the term "alkenyl" refers to an unbranched or branched monovalent hydrocarbon chain containing one or more carbon-carbon double bonds. As used herein, the term "alkynyl" refers to an unbranched or branched monovalent hydrocarbon chain containing one or more carbon-carbon triple bonds.

The term "$C_1$ to $C_6$ alkyl", either alone or as part of other substituents, should be interpreted as a straight or branched chain saturated hydrocarbon group having 1, 2, 3, 4, 5, or 6 carbon atoms. Examples of "$C_1$ to $C_6$ alkyl" are, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or isomers thereof. In particular, the group has 1, 2, or 3 carbon atoms ("$C_1$ to $C_3$

alkyl"), for example methyl, methylene, ethyl, n-propyl, or isopropyl.

The term "oxo", either alone or as part of other substituents, refers to that two hydrogen atoms of methylene group are substituted with oxygen atoms, i.e., the methylene group is substituted with a carbonyl group, denoted as =O.

The term "cycloalkyl" or "carbocyclyl", either alone or as part of other substituents, refers to a cyclic alkyl. The term "m- to n-membered cycloalkyl" or "$C_m$ to $C_n$ cycloalkyl" should be interpreted as a saturated or partially saturated carbocyclic ring having m to n atoms. For example, "3- to 15-membered cycloalkyl" or "$C_3$ to $C_{15}$ cycloalkyl" refers to a cyclic alkyl group containing 3 to 15, 3 to 9, 3 to 6, or 3 to 5 carbon atoms, which may contain 1 to 4 rings. "5- to 8-membered cycloalkyl" contains 5 to 8 carbon atoms. It includes monocyclic, bicyclic, tricyclic, spirocyclic, or bridged ring structures. Examples of unsubstituted cycloalkyl include, but are not limited to, cyclopropyl, cyclobutyl, cyclopentyl, cyclohexyl, and adamantyl, or a bicyclic hydrocarbon group such as a decalin ring. Cycloalkyl may be substituted with one or more substituents. In some embodiments, cycloalkyl may be a cycloalkyl fused to an aryl or heteroaryl. The term "$C_3$ to $C_6$ cycloalkyl" should be interpreted as a saturated or partially saturated monocyclic or bicyclic hydrocarbon ring having 3 to 6 carbon atoms, including fused or bridged polycyclic ring systems. For example, cyclopropyl, cyclobutyl, cyclopentyl, and cyclohexyl.

"Halogenated cycloalkyl", either alone or as part of other substituents, refers to a cycloalkyl as described above, in which a number (at least one) of hydrogen atoms attached to cycloalkyl is substituted with fluorine, chlorine, bromine, or iodine.

"Halogenated alkyl", either alone or as part of other substituents, refers to a saturated aliphatic hydrocarbon group including branched and straight chains having a specified number of carbon atoms, and substituted with one or more halogens (e.g., $-C_vF_w$, where v=1 to 3, and w=1 to (2v+1)). Examples of halogenated alkyl include, but are not limited to, trifluoromethyl, trichloromethyl, pentafluoroethyl, pentachloroethyl, 2,2,2-trifluoroethyl, heptafluoropropyl, and heptachloropropyl.

The term "5- to 7-membered heteroaromatic ring", either alone or as part of other substituents, may be used interchangeably with "5- to 7-membered heteroaryl", and should be understood as an aromatic ring group having 5, 6 or 7 ring atoms and containing 1 to 3 heteroatoms independently selected from N, O, and S. The term "5- to 7-membered heteroaromatic ring" is to be understood as an aromatic ring group having 5, 6 or 7 ring atoms and containing 1 to 3 heteroatoms independently selected from N, O, and S. In particular, heteroaryl is selected from thienyl, furanyl, pyrrolyl, oxazolyl, thiazolyl, imidazolyl, or pyrazolyl; or pyridinyl, pyridazinyl, pyrimidinyl, or pyrazinyl.

The term "heterocycloalkyl" or "heterocyclyl", either alone or as part of other substituents, refers to saturated cycloalkyl in which one or more (in some embodiments, 1 to 3) carbon atoms are replaced with heteroatoms, such as, but not limited to, N, O, S, and P. It should be understood that when the total number of S and O atoms in heterocycloalkyl exceeds one, these heteroatoms are not adjacent to each other. Examples of heterocycloalkyl include, but are not limited to, tetrahydroisoquinolinyl, tetrahydroquinolinyl, tetrahydropyranyl, tetrahydrofuranyl, and tetrahydrothiopyranyl. The term "heterocycloalkyl" or "heterocyclyl" may also contain 1, 2 or 3 rings, including fused rings, bridged rings, and spiro ring structures. The term "3- to 7-membered heterocycloalkyl" is to be understood as a cycloalkyl having 3, 4, 5, 6, or 7 atoms, which contains 1, 2, or 3 heteroatoms selected from N, O, S, and P. The term "5- to 8-membered heterocycloalkyl bicyclic system" is to be understood as two rings having 5 to 8 atoms, including fused rings, bridged rings, and spiro ring structures, wherein heteroatoms are preferably N, O and S. It should be understood that when the total number of S atoms and O atoms in heterocyclyl exceeds one, these heteroatoms are not adjacent to each other. Examples of "5- to 8-membered heterocycloalkyl bicyclic system" include, but are not limited to,

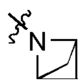 , 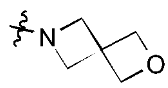 , and 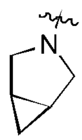 .

In the present disclosure, "optional" or "optionally" means that the subsequently described event or situation may or may not occur, and such an expression includes both occurrences and non-occurrences of the event or condition. For example, "optionally substituted aryl" means that the aryl is substituted or unsubstituted, and such an expression includes both substituted and unsubstituted aryl.

In the present disclosure, the term "salt" or "pharmaceutically acceptable salt" includes pharmaceutically acceptable acid addition salts and pharmaceutically acceptable base addition salts. The term "pharmaceutically acceptable" means that the compounds, materials, compositions, and/or dosage forms are suitable for contact with human and animal tissues without excess toxicity, irritation, allergic reactions, or other problems or complications within the scope of reliable medical judgment, and are commensurate with a reasonable benefit/risk ratio.

"Pharmaceutically acceptable acid addition salt" refers to a salt formed with an inorganic acid or organic acid that retains the biological effectiveness of the free base without other adverse effects. "Pharmaceutically acceptable base

addition salt" refers to a salt formed with an inorganic base or an organic base that retains the biological effectiveness of the free acid without other adverse effects. In addition to the pharmaceutically acceptable salts, other salts may be adopted in the present disclosure, and they can serve as intermediates in the purification of compounds or in the preparation of other pharmaceutically acceptable salts, or can be used for identifying, characterizing, or purifying the compounds of the present disclosure.

The term "amine salt" refers to a product obtained by neutralizing an alkyl primary, secondary, or tertiary amine with an acid. The acid includes the inorganic acid or organic acid described in the present disclosure.

The term "stereoisomer" refers to isomers formed due to different spatial arrangements of atoms in a molecule, including a cis-trans isomer, an enantiomer, a diastereoisomer, and a conformational isomer.

According to selected raw materials and methods, the compound of the present disclosure may be present in the form of a possible isomer or a mixture of isomers, for example, in the form of a pure optical isomer or a mixture of isomers such as a mixture of racemate and diastereoisomer, depending on the number of asymmetric carbon atoms. When an optically active compound is described, prefixes *D* and *L*, or *R* and *S* are used to represent an absolute configuration of a molecule with respect to a chiral center (or multiple chiral centers) in the molecule. Prefixes *D* and *L*, or (+) and (-) are symbols used for designating the rotation of plane polarized light caused by a compound, and (-) or *L* indicates that a compound is levorotatory. Compounds with the prefix (+) or *D* are dextrorotatory.

When a bond to a chiral carbon in the formula of the present disclosure is depicted as a straight line, it is to be understood that (*R*) and (*S*) configurations of the chiral carbon and produced enantiomerically pure compounds and mixtures are all included within the scope of the general formula. The racemate or enantiomerically pure compounds of the present disclosure are graphically represented with reference to Maehr, J. Chem. Ed. 1985, 62: 114-120. A wedge bond and a dashed bond are used to represent an absolute configuration of a stereocenter.

The term "tautomer" refers to functional group isomers formed by rapid movement of a certain atom in a molecule between two positions. The compound of the present disclosure may have a tautomerism phenomenon. The tautomeric compound may be present in two or more interconvertible forms. A prototropic tautomer is formed by migration of a hydrogen atom covalently bonded between two atoms. Tautomer is generally present in an equilibrium form, and separation of a single tautomer usually yields a mixture whose physicochemical properties are consistent with those of a mixture of compounds. The position of equilibrium depends on intramolecular chemical properties. For example, in several aliphatic aldehydes and ketones such as acetaldehyde, ketonic configurations prevail, while in phenol, an enolic configuration prevails. The present disclosure covers all tautomer of the compound.

In the present disclosure, "pharmaceutical composition" refers to a formulation of the compound of the present disclosure and a medium commonly accepted in the art for delivery of a biologically active compound to a mammal (e.g., a human). The medium includes a pharmaceutically acceptable carrier. The purpose of the pharmaceutical composition is to facilitate administration to an organism, facilitate the absorption of the active ingredients, and further facilitate the exertion of the biological activity.

In the present disclosure, "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier approved by the relevant governmental regulatory authorities as acceptable for human or livestock use.

The term "solvate" refers to the compound of the present disclosure or a salt thereof including a stoichiometric or non-stoichiometric solvent bonded through an intermolecular noncovalent force. When the solvent is water, the solvate is a hydrate.

The term "prodrug" refers to a substance that can be transformed into the compound of the present disclosure having bioactivity under the physiological conditions or by dissolving in a solvent. The prodrug of the present disclosure is prepared by modifying functional groups in the compound, and the modification can be removed by conventional operations or removed *in vivo* to obtain a parent compound. The prodrug includes a compound formed by linking one hydroxyl group or amino group in the compound of the present disclosure to any group, and when administered to an individual mammal, the prodrug of the compound of the present disclosure is cleaved to form a free hydroxyl group or free amino group.

The compound of the present disclosure may contain an unnatural proportion of atomic isotopes at one or more atoms forming the compound. For example, the compound can be labeled with radioisotopes such as deuterium ($^{2}H$), tritium ($^{3}H$), iodine-125 ($^{125}I$), and C-14 ($^{14}C$). All changes made to the isotope composition of the compound of the present disclosure, regardless of whether they are radioactive or not, shall fall within the scope of the present disclosure.

The term "adjuvant" refers to medicinal inert ingredients. Examples of types of the term "excipient" include, but are not limited to, an adhesive, a disintegrant, a lubricant, a glidant, a stabilizer, a filler, a diluent, etc. Excipients can enhance the operating characteristics of pharmaceutical preparations, that is, improve the fluidity and/or adhesiveness to enable preparations to be more suitable for direct compression.

As used herein, the term "treatment" and other similar synonyms include the following meanings: (i) preventing a disease or condition from occurring in a mammal, particularly where such mammal is susceptible to the disease or

condition but has not yet been diagnosed as having the disease or condition; (ii) inhibiting a disease or condition, i.e., suppressing development thereof; (iii) alleviating a disease or condition, that is, enabling the state of the disease or condition to regress; or (iv) alleviating the symptoms caused by the disease or condition.

**Beneficial Effects**

Through extensive and in-depth research, the inventors have unexpectedly developed a thienylsulfonyl carbamate compound as an AT2R agonist, and the compound has a structure represented in the present disclosure. The thienylsulfonyl carbamate compound of the present disclosure can prevent or treat an AT2R-related disease or disorder, exhibits excellent pharmacokinetic properties, and possesses high safety and drug-like properties.

**DETAILED DESCRIPTION OF THE EMBODIMENTS**

The present disclosure is further described below in conjunction with specific embodiments. It should be understood that the following description is only the most preferred embodiment of the present disclosure, and should not be regarded as limiting the protection scope of the present disclosure. On the basis of fully understanding the present disclosure, the experimental methods in the following embodiments for which specific conditions are not specified are generally carried out under conventional conditions or under conditions recommended by the manufacturer. Those skilled in the art can make non-essential changes to the technical solutions of the present disclosure, and such changes should fall within the protection scope of the present disclosure.

**Definitions**

Symbol or unit:

$IC_{50}$: half inhibitory concentration, which refers to the concentration at which half of the maximum inhibitory effect is achieved.
M: mol/L, for example, n-butyllithium (14.56 mL, 29.1 mmol, 2.5 M n-hexane solution) means a solution of n-butyllithium in n-hexane with a molar concentration of 2.5 mol/L.
N: equivalent concentration, for example, 2N hydrochloric acid means 2 mol/L hydrochloric acid solution.
RT: retention time

Reagents:

DCM: dichloromethane
DMF: N,N-dimethylformamide
DIPEA: N,N-diisopropylethylamine
EA: ethyl acetate
HATU: 2-(7-azobenzotriazole)-N,N,N',N'-tetramethyluronium hexafluorophosphate
MeOH: methanol
PE: petroleum ether
Toluene: methylbenzene
THF: tetrahydrofuran
XPhos Pd G4: methanesulfonate (2-dicyclohexylphosphino-2',4',6'-triisopropyl-1,1'-biphenyl)(2'-methylamino-1,1'-biphenyl-2-yl)palladium(II)
SPhos Pd G3: methanesulfonate (2-dicyclohexylphosphino-2',6'-dimethoxy-1,1'-biphenyl)(2'-amino-1,1'-biphenyl-2-yl)palladium(II)

Test method:

LCMS: liquid chromatography-mass spectrometry
TLC: thin layer chromatography

**Intermediate A1:** Preparation of Intermediate **A1**

N-(tert-butyl)-3-(4-(chloromethyl)phenyl)-4-fluoro-5-isobutylthiophene-2-sulfonamide

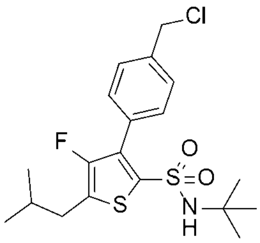

**A1**

The synthetic scheme of **Intermediate A1** is as follows:

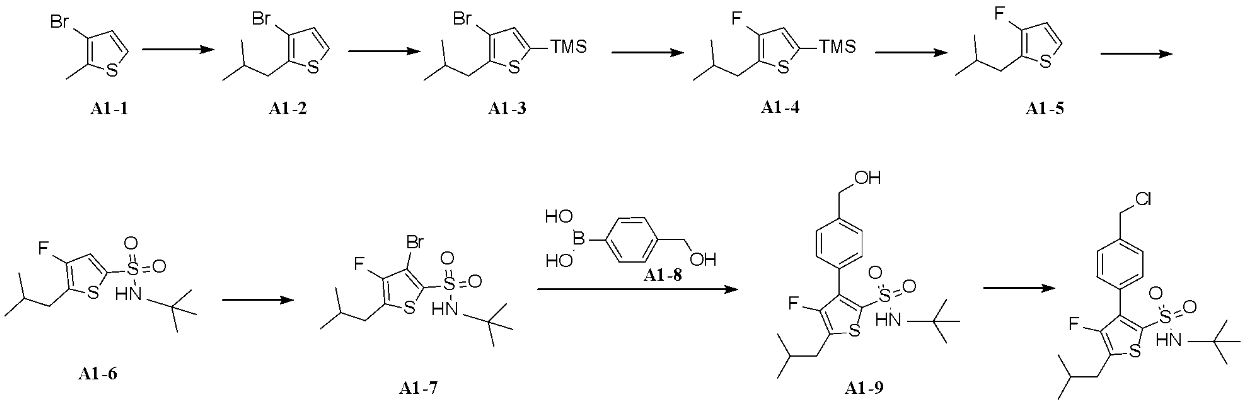

A1-1 A1-2 A1-3 A1-4 A1-5 A1-6 A1-7 A1-8 A1-9 A1

Step 1: Synthesis of 3-bromo-2-isobutylthiophene

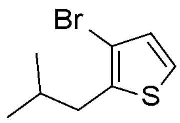

**A1-2**

Under a nitrogen atmosphere, azobisisobutyronitrile (241 mg, 1.47 mmol) was added to a solution of 3-bromo-2-methylthiophene (2.6 g, 14.7 mmol) and N-bromosuccinimide (2.62 g, 14.7 mmol) in carbon tetrachloride (100 mL). Then, the mixture was heated to 80°C and reacted for 2 hours. The reaction solution was cooled to room temperature, and a solid was precipitated out. The mixture was filtered, and the filtrate was concentrated under reduced pressure to obtain a crude product (3.2 g), which was directly used in a next step. Under a nitrogen atmosphere, the crude product (3.2 g) obtained above was dissolved in anhydrous tetrahydrofuran (70 mL), and the resulting solution was cooled to 0°C. Then, a solution of isopropylmagnesium chloride in tetrahydrofuran (1.3 M, 14.7 mL) was added dropwise to the reaction solution. After the mixture reacted at 0°C for 2 hours, the reaction was quenched with 20 mL of ice water. The mixture was extracted three times with 50 mL of ethyl acetate each time. The organic phase was concentrated to dryness. The residue was adsorbed onto silica gel and purified by column chromatography (eluent: petroleum ether) to obtain 3-bromo-2-isobutylthiophene (2 g, yield: 62%).
LC-MS, M/Z (ESI): 219.0 $[M+H]^+$

Step 2: Synthesis of (4-bromo-5-isobutylthiophene-2-yl)trimethylsilane

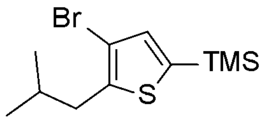

A1-3

Under a nitrogen atmosphere and at -78°C, a solution of lithium diisopropylamide in tetrahydrofuran (2 M, 5.2 mL) was added dropwise to a solution of 3-bromo-2-isobutylthiophene (1.88 g, 8.6 mmol) in tetrahydrofuran (45 mL). The mixture reacted at -78°C for 0.5 hours, and then trimethylchlorosilane (1.4 g) was added dropwise to the reaction solution. Then, the reaction solution was slowly warmed to room temperature and reacted at room temperature for 16 hours. The reaction was quenched with 50 mL of water, and the mixture was extracted three times with 50 mL of ethyl acetate each time. The organic phase was concentrated to dryness. The residue was adsorbed onto silica gel and purified by column chromatography (eluent: petroleum ether) to obtain (4-bromo-5-isobutylthiophen-2-yl)trimethylsilane (2.4 g, yield: 95%).

LC-MS, M/Z (ESI): 291.0 $[M+H]^+$
$^1H$ NMR (400 MHz, $CDCl_3$) $\delta$ 7.01 (s, 1 H), 2.68 (d, 2 H), 2.03-1.91 (m, 1 H), 0.97 (d, 6 H), 0.29 (s, 9 H)

Step 3: Synthesis of (4-fluoro-5-isobutylthiophene-2-yl)trimethylsilane

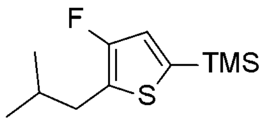

A1-4

Under a nitrogen atmosphere and at -78°C, a solution of n-butyllithium in tetrahydrofuran (2.5 M, 5 mL) was added dropwise to a solution (45 mL) of (4-bromo-5-isobutylthiophene-2-yl)trimethylsilane (2.4 g, 8.2 mmol) in tetrahydrofuran. The mixture reacted at -78°C for 1 hour, and then a solution of N-fluorobenzenesulfonimide (4.67 g, 14.8 mmol) in tetrahydrofuran (10 mL) was added dropwise. The mixture continuously reacted at - 78°C for 0.5 hours, and then the reaction solution was slowly warmed to room temperature and stirred overnight. The reaction was quenched with 50 mL of water, and the mixture was extracted three times with 50 mL of ethyl acetate each time. The organic phase was concentrated to dryness. The residue was adsorbed onto silica gel and purified by column chromatography (eluent: petroleum ether) to obtain (4-fluoro-5-isobutylthiophen-2-yl)trimethylsilane (1.7 g, yield: 89.9%).

LC-MS, M/Z (ESI): 231.1 $[M+H]^+$
$^1H$ NMR (400 MHz, $CDCl_3$) $\delta$ 7.0 (s, 1 H), 2.67 (d, 2 H), 2.02-1.91 (m, 1 H), 0.96 (d, 6 H), 0.29 (s, 9 H) ppm

Step 4: Synthesis of 3-fluoro-2-isobutylthiophene

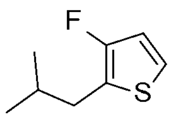

A1-5

Under a nitrogen atmosphere and at 0°C, trifluoroacetic acid (10 mL) was added to a solution of (4-fluoro-5-isobutylthiophene-2-yl)trimethylsilane (1.7 g, 7.4 mmol) in dichloromethane (10 mL). Then, the mixture reacted at 0°C for 2 hours. The reaction was quenched with 50 mL of water, and the mixture was extracted three times with 50 mL of ethyl

acetate each time. The organic phase was concentrated to dryness. The residue was adsorbed onto silica gel and purified by column chromatography (eluent: petroleum ether) to obtain 3-fluoro-2-isobutylthiophene (1.0 g, yield: 85.6%).

LC-MS, M/Z (ESI): 159.1 $[M+H]^+$

$^1$H NMR (400 MHz, $CDCl_3$) δ 6.97 (dd, 1 H), 6.74 (dd, 1 H), 2.59 (dd, 2 H), 1.95-1.81 (m, 1 H), 0.96 (s, 3 H), 0.94 (d, 3 H)

Step 5: Synthesis of N-(tert-butyl)-4-fluoro-5-isobutylthiophene-2-sulfonamide

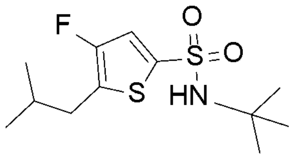

**A1-6**

Under a nitrogen atmosphere and at 0°C, chlorosulfonic acid (3.68 g, 31.6 mmol) was added to a solution of 3-fluoro-2-isobutylthiophene (1.0 g, 6.3 mmol) in dichloromethane (30 mL). Then, the mixture reacted at 0°C for 1 hour. The reaction was quenched with 50 mL of water, and the mixture was extracted three times with 50 mL of dichloromethane each time. The organic phase was dried over anhydrous sodium sulfate, filtered, and concentrated under reduced pressure. The resulting crude product was dissolved in anhydrous dichloromethane (30 mL), and triethylamine (1.93 g, 19 mmol) and tert-butylamine (0.7 g, 9.5 mmol) were added thereto sequentially at 0°C, and then the mixture reacted at room temperature for 2 hours. The reaction was quenched with 50 mL of water, and the mixture was extracted three times with 50 mL of dichloromethane each time. The organic phase was dried over anhydrous sodium sulfate, filtered, and concentrated under reduced pressure. The resulting crude product was purified by silica gel column chromatography (petroleum ether: ethyl acetate (V:V)=20:1 to 10:1) to obtain N-(tert-butyl)-4-fluoro-5-isobutylthiophene-2-sulfonamide (1.2 g, yield: 64.9%).

Step 6: Synthesis of 3-bromo-N-(tert-butyl)-4-fluoro-5-isobutylthiophene-2-sulfonamide

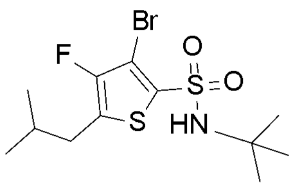

**A1-7**

Under a nitrogen atmosphere and at -78°C, a solution of n-butyllithium in tetrahydrofuran (2.5 M, 1.5 mL) was added dropwise to a solution of N-(tert-butyl)-4-fluoro-5-isobutylthiophene-2-sulfonamide (470 mg, 1.6 mmol) in anhydrous tetrahydrofuran (8 mL). Then, the mixture was warmed to -20°C and reacted for 2 hours. The reaction solution was then cooled to -78°C, and bromine (380 mg, 5 mmol) was added dropwise. Finally, the reaction solution was warmed to room temperature and reacted overnight. The reaction was quenched with 50 mL of water, and the mixture was extracted three times with 50 mL of ethyl acetate each time. The organic phase was dried over anhydrous sodium sulfate, filtered, and concentrated under reduced pressure. The resulting crude product was purified by silica gel column chromatography (petroleum ether: ethyl acetate (V:V)=20:1 to 10:1) to obtain 3-bromo-N-(tert-butyl)-4-fluoro-5-isobutylthiophene-2-sulfonamide (410 mg, yield: 68.8%).

LC-MS, M/Z (ESI): 372.3 $[M+H]^+$

$^1$H NMR (400 MHz, DMSO-d6) δ 8.16 (s, 1 H), 2.69 (d, 2 H), 1.92-1.78 (m, 1 H), 1.15 (s, 9 H), 0.88 (d, 6 H)

Step 7: Synthesis of N-(tert-butyl)-4-fluoro-3-(4-(hydroxymethyl)phenyl)-5-isobutylthiophene-2-sulfonamide

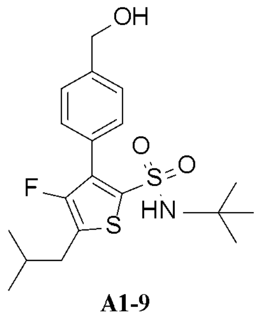

A1-9

Under a nitrogen atmosphere, 3-bromo-N-(tert-butyl)-4-fluoro-5-isobutylthiophene-2-sulfonamide (325 mg, 0.87 mmol), 4-(hydroxymethyl)phenylboronic acid (395.1 mg, 2.6 mmol), potassium carbonate (360 mg, 2.6 mmol), and [1,1'-bis(diphenylphosphino)ferrocene]palladium(II) dichloro (64 mg, 0.087 mmol) were suspended in anhydrous 1,4-dioxane (10 mL), and then the mixture reacted overnight at 75°C. The reaction solution was cooled to room temperature, and then was filtered. The filtrate was washed with 50 mL of water and extracted three times with 50 mL of ethyl acetate each time. The organic phase was dried over anhydrous sodium sulfate, filtered, and concentrated under reduced pressure. The resulting crude product was purified by silica gel column chromatography (petroleum ether: ethyl acetate (V:V)=10:1 to 2:1) to obtain N-(tert-butyl)-4-fluoro-3-(4-(hydroxymethyl)phenyl)-5-isobutylthiophene-2-sulfonamide (320 mg, yield: 92.06%).

LC-MS, M/Z (ESI): 400.1 $[M+H]^+$
$^1H$ NMR (400 MHz, $CDCl_3$) δ 7.61-7.55 (m, 2 H), 7.51-7.45 (m, 2 H), 4.77 (s, 2 H), 4.06 (s, 1 H), 2.68 (dd, 2 H), 1.98-1.91 (m, 1 H), 1.03 (s, 9 H), 0.99 (d, 6 H)

Step 8: Synthesis of N-(tert-butyl)-3-(4-(chloromethyl)phenyl)-4-fluoro-5-isobutylthiophene-2-sulfonamide

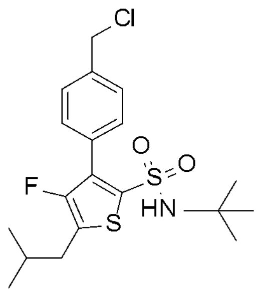

A1

Under a nitrogen atmosphere and at 0°C, thionyl chloride (238 mg, 2 mmol) was added dropwise to a solution of N-(tert-butyl)-4-fluoro-3-(4-(hydroxymethyl)phenyl)-5-isobutylthiophene-2-sulfonamide (320 mg, 0.8 mmol) in dichloromethane (4 mL), and the mixture reacted at room temperature for 3 hours. The solvent was concentrated under reduced pressure, and the resulting crude product was purified by silica gel column chromatography (petroleum ether: ethyl acetate (V:V)=10:1 to 4:1) to obtain N-(tert-butyl)-3-(4-(chloromethyl)phenyl)-4-fluoro-5-isobutylthiophene-2-sulfonamide (220 mg, yield: 66%).
LC-MS, M/Z (ESI): 418.1 $[M+H]^+$

**Intermediate A2:** Preparation of **Intermediate A2**

(2-(N-(tert-butyl)sulfamoyl)-5-isobutyl-4-methylthiophen-3-yl)boronic acid

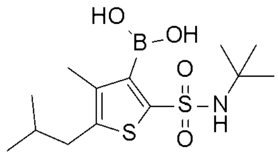

A2

The synthetic scheme of **intermediate A2** is as follows:

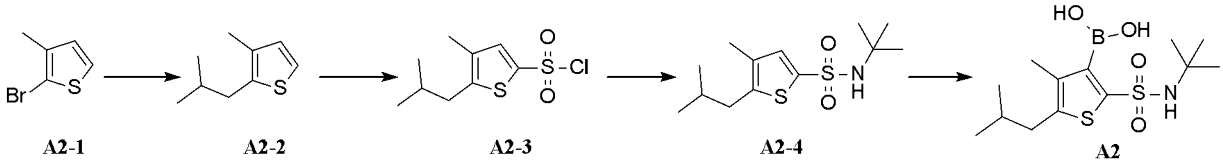

A2-1 A2-2 A2-3 A2-4 A2

Step 1: Synthesis of 2-isobutyl-3-methylthiophene

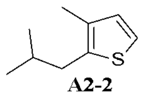

A2-2

Zinc chloride (13.8 g, 101 mmol) was dissolved in tetrahydrofuran (100 mL), and isobutylmagnesium bromide (2.00 M, 45.1 mL), 2-bromo-3-methylthiophene (10.0 g, 56.5 mmol), and bis(tri-tert-butylphosphine)palladium(0) (1.44 g, 2.82 mmol) were added. After the addition was complete, the reaction system was purged with nitrogen gas and slowly warmed to 90°C. The mixture reacted for 1 hour. The reaction solution was poured into water (200 mL), and the mixture was extracted three times with 200 mL of ethyl acetate each time. The organic phase was washed with saturated brine (50 mL), dried over anhydrous sodium sulfate, filtered, and concentrated to obtain 2-isobutyl-3-methylthiophene (6.00 g, yield 65.3%).

Step 2: Synthesis of 5-isobutyl-4-methylthiophene-2-sulfonyl chloride

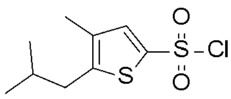

A2-3

2-isobutyl-3-methylthiophene (6.00 g, 36.9 mmol) was dissolved in dichloromethane (30 mL), chlorosulfonic acid (13.6 g, 116 mmol) was added, and the mixture was stirred at room temperature for 1 hour. The reaction solution was concentrated to obtain the product 5-isobutyl-4-methylthiophene-2-sulfonyl chloride (9.80 g, crude product).

Step 3: Synthesis of N-(tert-butyl)-5-isobutyl-4-methylthiophene-2-sulfonamide

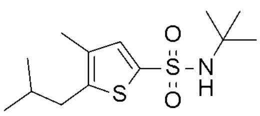

A2-4

5-isobutyl-4-methylthiophene-2-sulfonyl chloride (9.80 g, 38.7 mmol) was dissolved in dichloromethane (40 mL), and N,N-diisopropylethylamine (10.0 g, 77.5 mmol) and 2-methylpropane-2-amine (3.40 g, 46.5 mmol) were added, and the mixture was heated to 40°C and reacted for 1 hour. The reaction solution was poured into water (40 mL), and the

mixture was extracted three times with 200 mL of ethyl acetate each time. The organic phase was washed with saturated brine (100 mL), dried over anhydrous sodium sulfate, filtered, and concentrated. The crude product was purified by silica gel column chromatography (petroleum ether: ethyl acetate (V/V)=20:1 to 5:1, Rf=0.4) to obtain N-(tert-butyl)-5-isobutyl-4-methylthiophene-2-sulfonamide (1.50 g, yield 13.4%).
LC-MS, M/Z (ESI): 288.1 $[M-H]^+$

Step 4: Synthesis of (2-(N-(tert-butyl)sulfamoyl)-5-isobutyl-4-methylthiophen-3-yl)boronic acid

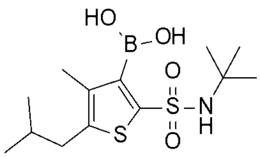

A2

N-(tert-butyl)-5-isobutyl-4-methylthiophene-2-sulfonamide (900 mg, 3.34 mmol) was dissolved in tetrahydrofuran (20 mL). The reaction solution was cooled to -60°C using a dry ice-ethanol bath. Under a nitrogen atmosphere, n-butyllithium (2.50 M, 3.98 mL) was slowly added dropwise. After the addition was complete, the mixture was stirred for 0.5 hours. Then, triisopropyl borate (1.17 g, 6.22 mmol) was added, and the mixture reacted at -60°C for 1 hour. The reaction solution was poured into water (20 mL), and the mixture was extracted three times with 20 mL of ethyl acetate each time. The organic phase was washed with saturated brine (20 mL), dried over anhydrous sodium sulfate, filtered, and concentrated. The crude product was purified by silica gel column chromatography (petroleum ether: ethyl acetate (V/V)=5:1 to 1:1, Rf=0.1) to obtain (2-(N-(tert-butyl)sulfamoyl)-5-isobutyl-4-methylthiophen-3-yl)boronic acid (0.7 g, yield 62.8%).
LC-MS, M/Z (ESI): 332.1 $[M-H]^+$

**Example 1:** Preparation of Target Compound **I-1**

Methyl ((3-(4-((2-chloro-1H-imidazol-1-yl)methyl)phenyl)-4-fluoro-5-isobutylthiophen-2-yl)sulfonyl) carbamate

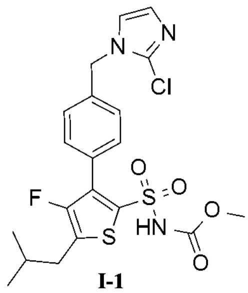

I-1

The synthetic scheme of Target Compound I-1 is as follows:

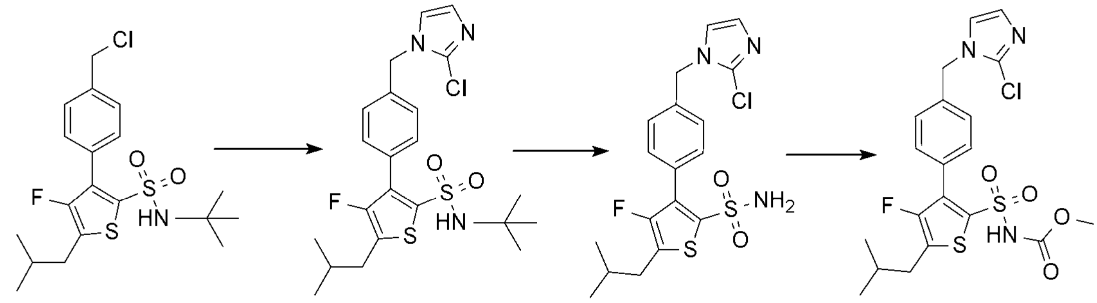

A1 1-1 1-2 I-1

Step 1: Synthesis of N-(tert-butyl)-3-(4-((2-chloro-1H-imidazol-1-yl)methyl)phenyl)-4-fluoro-5-isobutylthiophene-2-sulfonamide

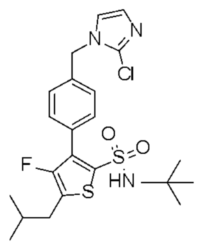

1-1

Under a nitrogen atmosphere, anhydrous DMF (3 mL) was added to a mixture of N-(tert-butyl)-3-(4-(chloromethyl)phenyl)-4-fluoro-5-isobutylthiophene-2-sulfonamide (220 mg, 0.53 mmol), 2-chloroimidazole (81 mg, 0.79 mmol), and potassium carbonate (220 mg, 1.59 mmol). Then the mixture reacted overnight at 60°C. The reaction solution was cooled to room temperature, and the reaction was quenched with 50 mL of water. The mixture was extracted three times with 50 mL of ethyl acetate each time. The organic phase was dried over anhydrous sodium sulfate, filtered, and concentrated under reduced pressure. The resulting crude product was purified by silica gel column chromatography (petroleum ether: ethyl acetate (V/V)=20:1 to 1:1) to obtain N-(tert-butyl)-3-(4-((2-chloro-1H-imidazol-1-yl)methyl)phenyl)-4-fluoro-5-isobutylthiophene-2-sulfonamide (200 mg, yield: 78.1%).
LC-MS, M/Z (ESI): 484.1 $[M+H]^+$

Step 2: Synthesis of 3-(4-((2-chloro-1H-imidazol-1-yl)methyl)phenyl)-4-fluoro-5-isobutylthiophene-2-sulfonamide (1-2)

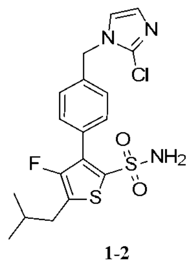

1-2

Under a nitrogen atmosphere, trifluoroacetic acid (4 mL) was added to a solution of N-(tert-butyl)-3-(4-((2-chloro-1H-imidazol-1-yl)methyl)phenyl)-4-fluoro-5-isobutylthiophene-2-sulfonamide (200 mg, 0.41 mmol) in dichloromethane (4 mL). Then the mixture reacted overnight at room temperature. The solvent was concentrated under reduced pressure. The crude product was adsorbed onto silica gel and purified by silica gel column chromatography (petroleum ether: ethyl acetate (V/V)=20:1 to 1:4) to obtain 3-(4-((2-chloro-1H-imidazol-1-yl)methyl)phenyl)-4-fluoro-5-isobutylthiophene-2-sulfonamide (140 mg, yield: 80%).
LC-MS, M/Z (ESI): 428.1 $[M+H]^+$

Step 3: Synthesis of methyl ((3-(4-((2-chloro-1H-imidazol-1-yl)methyl)phenyl)-4-fluoro-5-isobutylthiophen-2-yl)sulfonyl)carbamate

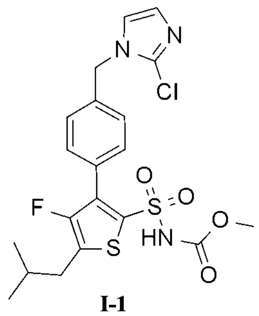

I-1

Under a nitrogen atmosphere and at 0°C, N,N-diisopropylethylamine (0.23 mL, 1.37 mmol) and methyl chloroformate (34.3 mg, 0.36 mmol) were added sequentially to a solution of 3-(4-((2-chloro-1H-imidazol-1-yl)methyl) phenyl)-4-fluoro-5-isobutylthiophene-2-sulfonamide (140 mg, 0.33 mmol) in dichloromethane (2 mL). Then the mixture reacted at 0°C for 2 hours, and the reaction was quenched with 20 mL of water. The mixture was extracted three times with 20 mL of ethyl acetate each time. The organic phase was concentrated to dryness, and the residue was purified by silica gel column chromatography (dichloromethane: methanol (V/V)=10:1) to obtain methyl ((3-(4-((2-chloro-1H-imidazol-1-yl) methyl)phenyl)-4-fluoro-5-isobutylthiophen-2-yl)sulfonyl)carbamate (55 mg, yield: 34%).

LC-MS, M/Z (ESI): 486.1 $[M+H]^+$
$^1H$ NMR (400 MHz, $CDCl_3$) δ 7.48-7.43 (m, 2 H), 7.25-7.20 (m, 2 H), 6.95-6.88 (m, 2 H), 5.15 (s, 2 H), 3.66 (s, 3 H), 2.71 (d, 2 H), 2.03-1.91 (m, 1 H), 1.0 (d, 6 H)

**Example 2:** Preparation of Target Compound **I-2**

Methyl ((4-fluoro-5-isobutyl-3-(4-((2-(trifluoromethyl)-1H-imidazol-1-yl)methyl)phenyl)thiophen-2-yl)sulfonyl) carbamate

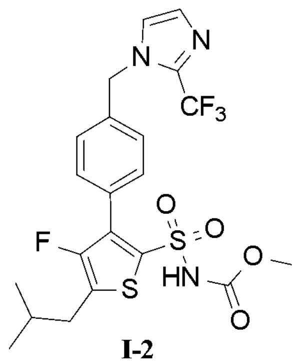

I-2

The synthetic scheme of Target Compound **I-2** is as follows:

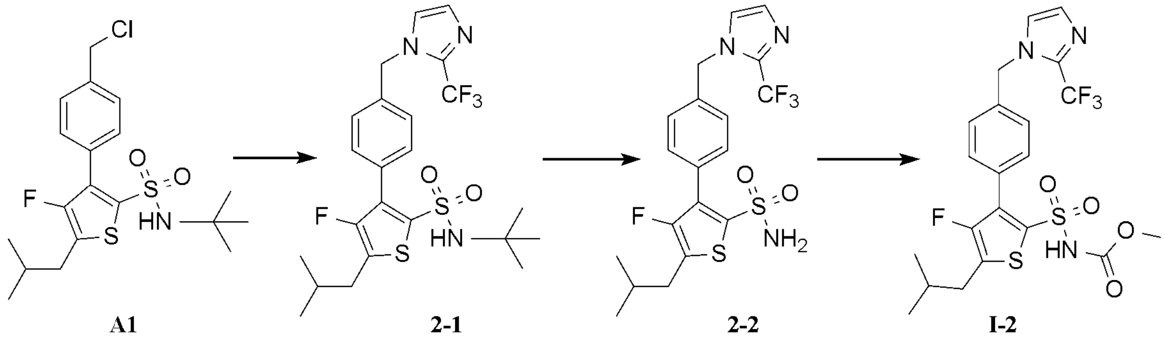

A1 2-1 2-2 I-2

Step 1: Synthesis of N-(tert-butyl)-4-fluoro-5-isobutyl-3-(4-((2-(trifluoromethyl)-1H-imidazol-1-yl)methyl)phenyl)thiophene-2-sulfonamide

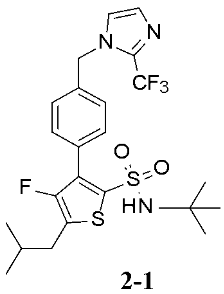

**2-1**

Under a nitrogen atmosphere, anhydrous DMF (5 mL) was added to a mixture of N-(tert-butyl)-3-(4-(chloromethyl)phenyl)-4-fluoro-5-isobutylthiophene-2-sulfonamide (308 mg, 0.74 mmol), 2-(trifluoromethyl)-1H-imidazole (151 mg, 1.11 mmol), and potassium carbonate (307 mg, 2.22 mmol). Then the mixture reacted overnight at 60°C. The reaction solution was cooled to room temperature, and the reaction was quenched with 20 mL of water. The mixture was extracted three times with 20 mL of ethyl acetate each time, and the organic phase was dried over anhydrous sodium sulfate, filtered, and concentrated under reduced pressure. The resulting crude product was purified by silica gel column chromatography (petroleum ether: ethyl acetate (V/V)=20:1 to 1:1) to obtain N-(tert-butyl)-4-fluoro-5-isobutyl-3-(4-((2-(trifluoromethyl)-1H-imidazole-1-yl)methyl)phenyl)thiophene-2-sulfonamide (360 mg, yield: 94%).
LC-MS, M/Z (ESI): 518.2 $[M+H]^+$

Step 2: Synthesis of 4-fluoro-5-isobutyl-3-(4-((2-(trifluoromethyl)-1H-imidazol-1-yl)methyl)phenyl)thiophene-2-sulfonamide

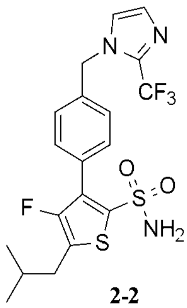

**2-2**

Under a nitrogen atmosphere, trifluoroacetic acid (6 mL) was added to a solution of N-(tert-butyl)-4-fluoro-5-isobutyl-3-(4-((2-(trifluoromethyl)-1H-imidazol-1-yl)methyl)phenyl)thiophene-2-sulfonamide (360 mg, 0.69 mmol) in dichloromethane (6 mL). Then the mixture reacted overnight at room temperature. The solvent was concentrated under reduced pressure. The crude product was adsorbed onto silica gel and purified by silica gel column chromatography (petroleum ether: ethyl acetate (V/V)=20:1 to 1:4) to obtain 4-fluoro-5-isobutyl-3-(4-((2-(trifluoromethyl)-1H-imidazol-1-yl)methyl)phenyl)thiophene-2-sulfonamide (230 mg, yield: 72%).
LC-MS, M/Z (ESI): 462.1 $[M+H]^+$

Step 3: Synthesis of methyl ((4-fluoro-5-isobutyl-3-(4-((2-(trifluoromethyl)-1H-imidazol-1-yl)methyl)phenyl)thiophen-2-yl)sulfonyl)carbamate

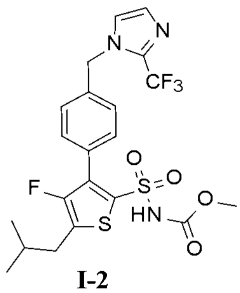

I-2

Under a nitrogen atmosphere and at 0°C, N,N-diisopropylethylamine (0.153 mL, 0.88 mmol) and methyl chloroformate (23 mg, 0.24 mmol) were added sequentially to a solution of 4-fluoro-5-isobutyl-3-(4-((2-(trifluoromethyl)-1H-imidazol-1-yl)methyl)phenyl)thiophene-2-sulfonamide (100 mg, 0.22 mmol) in dichloromethane (2 mL). Then the mixture reacted at 0°C for 2 hours. The reaction was quenched with 20 mL of water, and the mixture was extracted three times with 20 mL of ethyl acetate each time. The organic phase was concentrated to dryness, and the residue was purified by silica gel column chromatography (dichloromethane: methanol (V/V)=10:1) to obtain methyl ((4-fluoro-5-isobutyl-3-(4-((2-(trifluoromethyl)-1H-imidazol-1-yl)methyl)phenyl)thiophen-2-yl)sulfonyl)carbamate (43 mg, yield: 38%).

LC-MS, M/Z (ESI): 520.1 $[M+H]^+$

$^1H$ NMR (400 MHz, $CDCl_3$) δ 7.52 - 7.46 (m, 2 H), 7.22-7.17 (m, 2 H), 7.13-7.08 (m, 1 H), 7.03-6.99 (m, 1 H), 5.27 (s, 2 H), 3.52 (t, 3 H), 2.65 (d, 2 H), 1.98-1.86 (m, 1 H), 0.97 (d, 6 H)

**Example 3:** Preparation of Target Compound **I-3**

Butyl ((4-fluoro-3-(4-((2-(2-hydroxypropan-2-yl)-1H-imidazol-1-yl) methyl) phenyl)-5-isobutylthiophen-2-yl) sulfonyl) carbamate

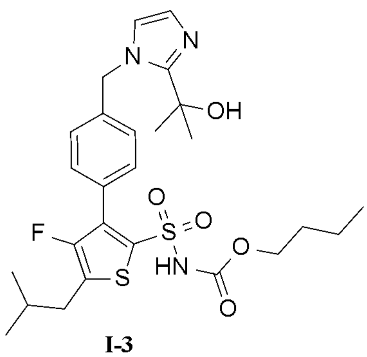

I-3

The synthetic scheme of Target Compound **I-3** is as follows:

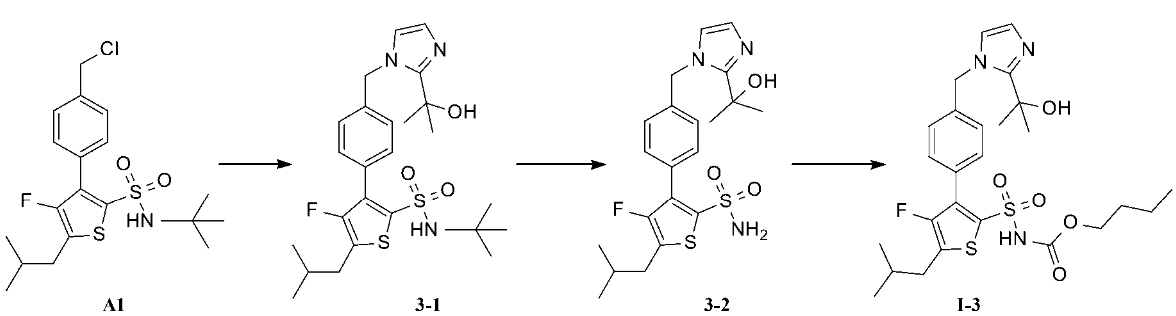

A1 3-1 3-2 I-3

Step 1: Synthesis of N-(tert-butyl)-4-fluoro-3-(4-((2-(2-hydroxypropan-2-yl)-1H-imidazol-1-yl)methyl)phenyl)-5-isobutylthiophene-2-sulfonamide

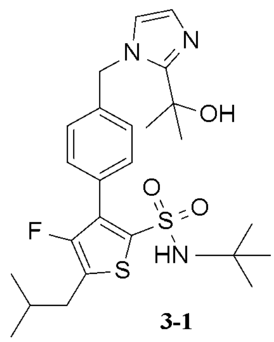

**3-1**

Under a nitrogen atmosphere, anhydrous DMF (2 mL) was added to a mixture of N-(tert-butyl)-3-(4-(chloromethyl)phenyl)-4-fluoro-5-isobutylthiophene-2-sulfonamide (80 mg, 0.22 mmol), 2-(1H-imidazol-2-yl)propan-2-ol (29 mg, 0.23 mmol), and potassium carbonate (98 mg, 0.71 mmol). Then the mixture reacted overnight at 55°C. The reaction solution was cooled to room temperature, and the reaction was quenched with 20 mL of water. The mixture was extracted three times with 20 mL of ethyl acetate each time. The organic phase was dried over anhydrous sodium sulfate, filtered, and concentrated under reduced pressure. The resulting crude product was purified by silica gel column chromatography (petroleum ether: ethyl acetate (V/V)=20:1 to 1:1) to obtain N-(tert-butyl)-4-fluoro-3-(4-((2-(2-hydroxypropan-2-yl)-1H-imidazol-1-yl)methyl)phenyl)-5-isobutylthiophene-2-sulfonamide (60 mg, yield: 53.7%).
LC-MS, M/Z (ESI): 508.7 $[M+H]^+$

Step 2: Synthesis of 4-fluoro-3-(4-((2-(2-hydroxypropan-2-yl)-1H-imidazol-1-yl)methyl)phenyl)-5-isobutylthiophene-2-sulfonamide

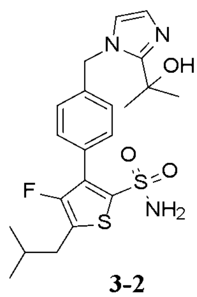

**3-2**

Under a nitrogen atmosphere, trifluoroacetic acid (2 mL) was added to a solution of N-(tert-butyl)-4-fluoro-3-(4-((2-(2-hydroxypropan-2-yl)-1H-imidazol-1-yl)methyl)phenyl)-5-isobutylthiophene-2-sulfonamide (60 mg, 0.12 mmol) in dichloromethane (2 mL). Then the mixture reacted overnight at room temperature. The solvent was concentrated under reduced pressure. The crude product was adsorbed onto silica gel and purified by silica gel column chromatography (dichloromethane: methanol (V/V)=50:1 to 10:1) to obtain 4-fluoro-3-(4-((2-(2-hydroxypropan-2-yl)-1H-imidazol-1-yl)methyl)phenyl)-5-isobutylthiophene-2-sulfonamide (25 mg, yield: 50%).
LC-MS, M/Z (ESI): 452.6 $[M+H]^+$

Step 3: Synthesis of butyl ((4-fluoro-3-(4-((2-(2-hydroxypropan-2-yl)-1H-imidazol-1-yl)methyl)phenyl)-5-isobutylthiophen-2-yl)sulfonyl)carbamate

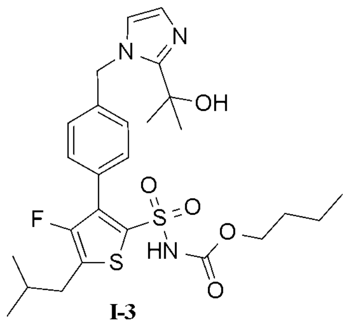

I-3

Under a nitrogen atmosphere and at 0°C, N,N-diisopropylethylamine (0.04 mL, 0.22 mmol) and butyl chloroformate (8.2 mg, 0.06 mmol) were added sequentially to a solution of 4-fluoro-3-(4-((2-(2-hydroxypropan-2-yl)-1H-imidazol-1-yl)methyl)phenyl)-5-isobutylthiophene-2-sulfonamide (25 mg, 0.055 mmol) in dichloromethane (1 mL). Then the mixture reacted at 0°C for 2 hours. The reaction was quenched with 20 mL of water, and the mixture was extracted three times with 20 mL of ethyl acetate each time. The organic phase was concentrated to dryness, and the residue was purified by silica gel column chromatography (dichloromethane: methanol (V/V)=10:1) to obtain butyl ((4-fluoro-3-(4-((2-(2-hydroxypropan-2-yl)-1H-imidazol-1-yl)methyl)phenyl)-5-isobutylthiophen-2-yl)sulfonyl)carbamate (13 mg, yield: 43%).

LC-MS, M/Z (ESI): 552.2 $[M+H]^+$
$^1H$ NMR (400 MHz, $CDCl_3$) δ 7.48-7.43 (m, 2 H), 7.17-7.12 (m, 2 H), 6.97-6.95 (m, 1 H), 6.85-6.83 (m, 1 H), 5.48 (s, 2 H), 4.05-3.98 (t, 2 H), 2.72-2.67 (d, 2 H), 1.98-1.86 (m, 1 H), 1.65 (s. 6 H), 1.54-1.46 (m, 2 H), 1.30-1.24 (m, 2 H), 0.97 (d, 6 H), 0.88 (t, 3 H)

**Example 4:** Preparation of Target Compound **I-4**

Butyl ((4-fluoro-5-isobutyl-3-(4-((2-(trifluoromethyl)-1H-imidazol-1-yl)methyl)phenyl)thiophen-2-yl)sulfonyl)carbamate

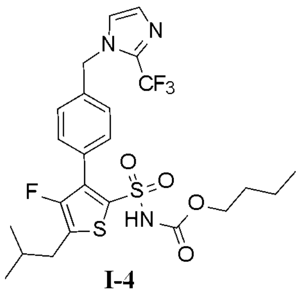

I-4

The synthetic scheme of Target Compound **I-4** is as follows:

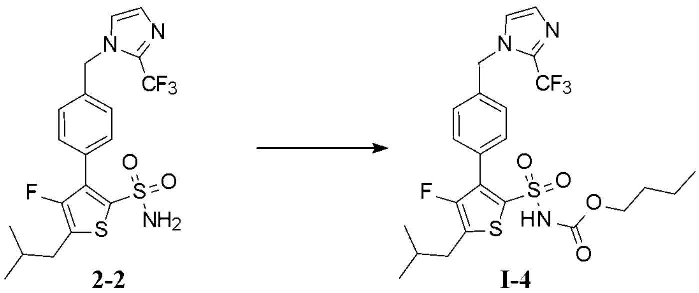

2-2 I-4

Step 1: Synthesis of butyl ((4-fluoro-5-isobutyl-3-(4-((2-(trifluoromethyl)-1H-imidazol-1-yl)methyl)phenyl)thiophen-2-yl) sulfonyl)carbamate

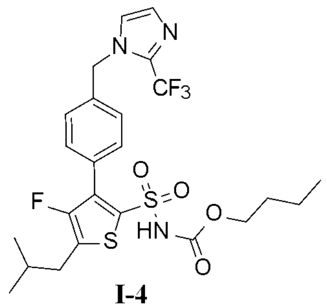

I-4

Under a nitrogen atmosphere and at 0°C, N,N-diisopropylethylamine (0.2 mL, 1.13 mmol) and methyl chloroformate (42.3 mg, 0.31 mmol) were added sequentially to a solution of 4-fluoro-5-isobutyl-3-(4-((2-(trifluoromethyl)-1H-imidazol-1-yl)methyl)phenyl)thiophene-2-sulfonamide (130 mg, 0.28 mmol) in dichloromethane (2 mL). Then the mixture reacted at 0°C for 2 hours. The reaction was quenched with 20 mL of water, and the mixture was extracted three times with 20 mL of ethyl acetate each time. The organic phase was concentrated to dryness, and the residue was purified by silica gel column chromatography (dichloromethane: methanol (V/V)=10:1) to obtain butyl ((4-fluoro-5-isobutyl-3-(4-((2-(trifluoromethyl)-1H-imidazol-1-yl)methyl)phenyl)thiophene-2-yl)sulfonyl)carbamate (35 mg, yield: 22%).

LC-MS, M/Z (ESI): 562.1 $[M+H]^+$
$^1H$ NMR (400 MHz, $CDCl_3$) δ 7.52-7.46 (m, 2 H), 7.23-7.19 (m, 2 H), 7.13-7.11 (m, 1 H), 7.03-6.99 (m, 1 H), 5.29 (s, 2 H), 4.01 (t, 3 H), 2.69 (d, 2 H), 2.01-1.89 (m, 1 H), 1.54-1.45 (m, 2 H), 1.29-1.20 (m, 2 H), 0.99 (d, 6 H), 0.87 (t, 3 H)

**Example 5:** Preparation of Target Compound **I-9**

Methyl ((5-isobutyl-4-methyl-3-(4-((2-(trifluoromethyl)-1H-imidazol-1-yl)methyl)phenyl)thiophen-2-yl)sulfonyl)carbamate

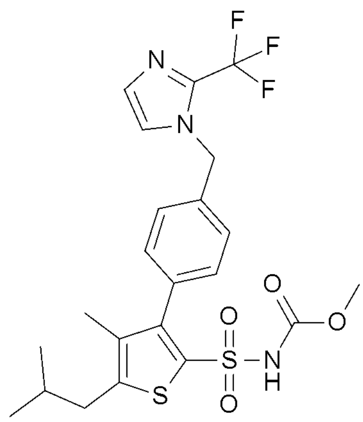

I-9

The synthetic scheme of Target Compound I-9 is as follows:

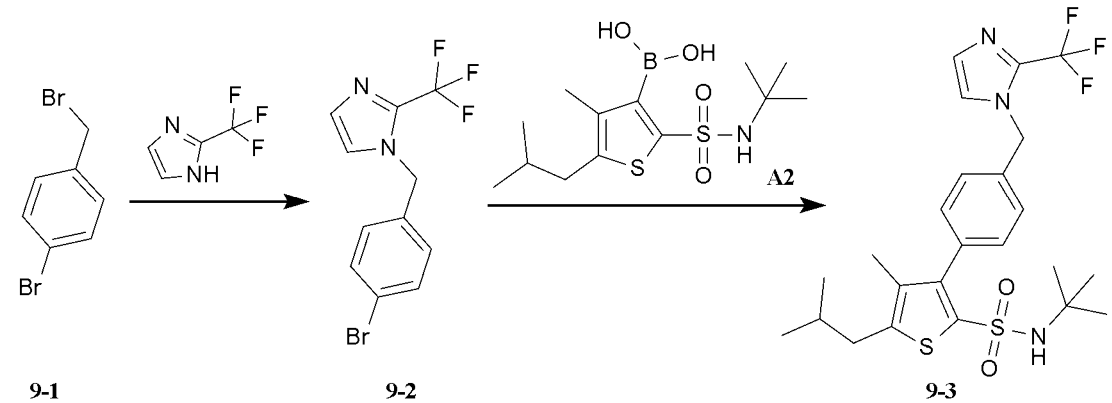


9-1 9-2 9-3

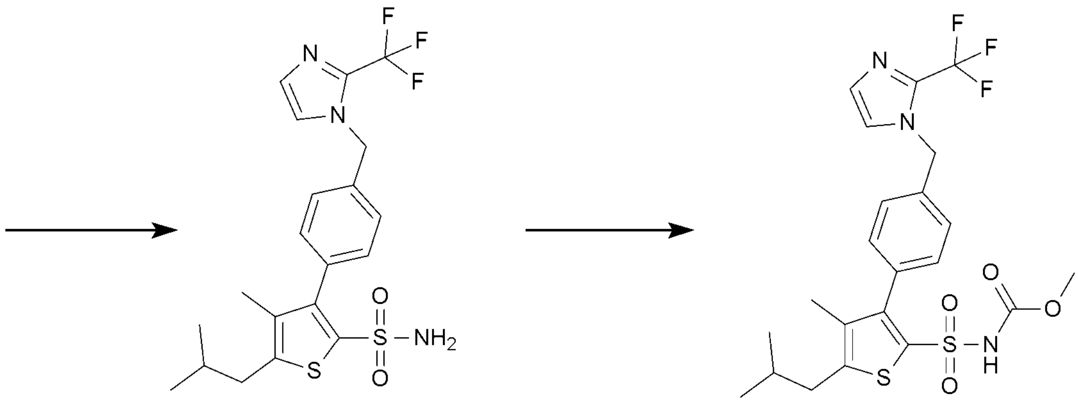

9-4 I-9

Step 1: Synthesis of 1-(4-bromobenzyl)-2-(trifluoromethyl)-1H-imidazole

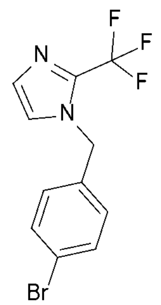

9-2

1-bromo-4-(bromomethyl)benzene (1.00 g, 4.00 mmol) and 2-(trifluoromethyl)-1H-imidazole (544 mg, 4.00 mmol) were dissolved in N,N-dimethylformamide (10 mL). Potassium carbonate (1.66 mg, 12.0 mmol) was added, and the mixture reacted at 50°C for 2 hours. The reaction solution was poured into water (100 mL), and the mixture was extracted three times with 80 mL of ethyl acetate each time. The organic phase was collected, dried over anhydrous sodium sulfate, filtered, and concentrated to obtain Compound 1-(4-bromobenzyl)-2-(trifluoromethyl)-1H-imidazole (1.20 g, yield 98.3%). LC-MS, M/Z (ESI): 304.9 $[M+H]^+$

Step 2: Synthesis of N-(tert-butyl)-5-isobutyl-4-methyl-3-(4-((2-(trifluoromethyl)-1H-imidazol-1-yl)methyl)phenyl)thiophene-2-sulfonamide

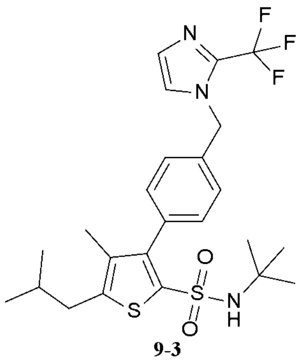

9-3

(2-(N-(tert-butyl)sulfamoyl)-5-isobutyl-4-methylthiophen-3-yl)boronic acid (400 mg, 1.20 mmol) was dissolved in tetrahydrofuran (10 mL) and water (3 mL). 1-(4-bromobenzyl)-2-(trifluoromethyl)-1H-imidazole (330 mg, 1.08 mmol) was added, followed by the addition of potassium phosphate (1.27 g, 6.00 mmol) and XPhos Pd G4 (103 mg, 120 μmol). The mixture was then stirred at 60 °C and reacted for 2 hours under a nitrogen atmosphere. The reaction solution was concentrated to obtain a crude product. The crude product was purified by silica gel column chromatography (petroleum ether: ethyl acetate (V/V)=5:1 to 0:1) to obtain Compound N-(tert-butyl)-5-isobutyl-4-methyl-3-(4-((2-(trifluoromethyl)-1H-imidazol-1-yl)methyl)phenyl)thiophene-2-sulfonamide (560 mg, yield 90.8%).
LC-MS, M/Z (ESI): 514.0 $[M+H]^+$

Step 3: Synthesis of 5-isobutyl-4-methyl-3-(4-((2-(trifluoromethyl)-1H-imidazol-1-yl)methyl)phenyl)thiophene-2-sulfonamide

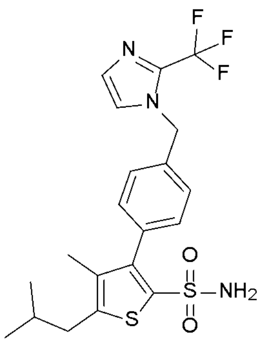

9-4

N-(tert-butyl)-5-isobutyl-4-methyl-3-(4-((2-(trifluoromethyl)-1H-imidazol-1-yl)methyl)phenyl)thiophene-2-sulfonamide (550 mg, 1.07 mmol) was dissolved in trifluoroacetic acid (8 mL) and dichloromethane (4 mL), and the mixture reacted at 40°C for 2 hours. The reaction solution was concentrated to obtain a crude product. The crude product was purified by silica gel column chromatography (petroleum ether: ethyl acetate (V/V)=5:1 to 1:1) to obtain Compound 5-isobutyl-4-methyl-3-(4-((2-(trifluoromethyl)-1H-imidazol-1-yl)methyl)phenyl)thiophene-2-sulfonamide (470 mg, yield 95.9%).
LC-MS, M/Z (ESI): 458.1 $[M+H]^+$

Step 4: Synthesis of methyl ((5-isobutyl-4-methyl-3-(4-((2-(trifluoromethyl)-1H-imidazol-1-yl)methyl)phenyl)thiophen-2-yl)sulfonyl)carbamate

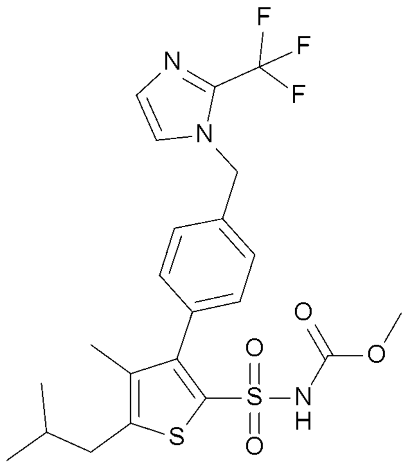

I-9

5-isobutyl-4-methyl-3-(4-((2-(trifluoromethyl)-1H-imidazol-1-yl)methyl)phenyl)thiophene-2-sulfonamide (470 mg, 1.03 mmol) and methyl chloroformate (194 mg, 2.05 mmol) were dissolved in dichloromethane (10 mL), and N,N-diisopropylethylamine (398 mg, 3.08 mmol) was added. The mixture was stirred at 0°C and reacted for 0.5 hours. The reaction solution was poured into a saturated aqueous sodium bicarbonate solution (20 mL), and the mixture was extracted three times with 20 mL of dichloromethane each time. The organic phase was collected, dried over anhydrous sodium sulfate, filtered, and concentrated. The crude product was purified by high performance liquid chromatography (column: Welch Xtimate 150*25mm*5μm; solvent: A=water+0.05 v/v formic acid (30%), B=acetonitrile; gradient: 58% to 78%, 10 minutes) to obtain the product methyl ((5-isobutyl-4-methyl-3-(4-((2-(trifluoromethyl)-1H-imidazol-1-yl)methyl)phenyl)thiophene-2-yl)sulfonyl)carbamate (319 mg, yield 61.1%).

LC-MS, M/Z (ESI): 516.1 $[M+H]^+$

$^1H$ NMR (400 MHz, DMSO-d6) δ: 11.83 (s, 1H), 7.60 (d, 1H), 7.17-7.23 (m, 4H), 7.15 (d, 1H), 5.42 (s, 2H), 3.53 (s, 3H), 2.67 (d, 2H), 1.81-1.91 (m, 1H), 1.75 (s, 3H), 0.93 (d, 6H)

**Example 6:** Preparation of Target Compound **I-10**

2-hydroxy-2-methylpropyl ((5-isobutyl-4-methyl-3-(4-((2-(trifluoromethyl)-1H-imidazol-1-yl)methyl)phenyl)thiophen-2-yl)sulfonyl)carbamate

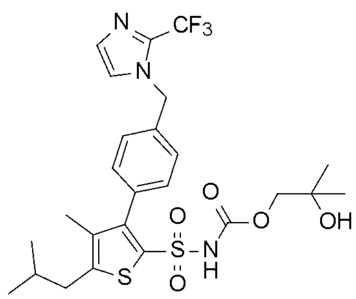

I-10

The synthetic scheme of Target Compound **I-10** is as follows:

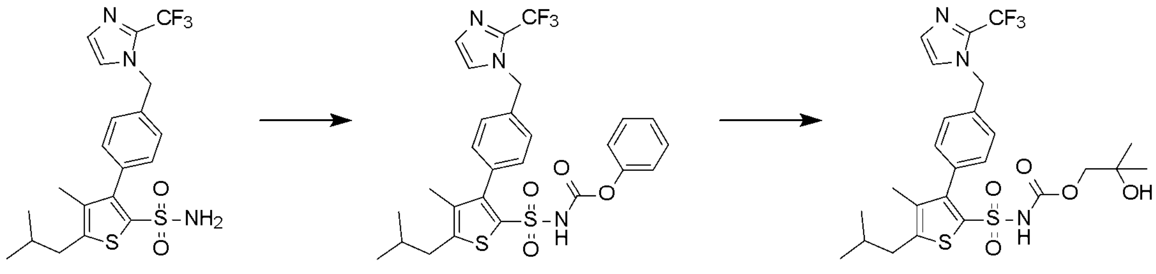

9-4 10-1 I-10

Step 1: Synthesis of phenyl ((5-isobutyl-4-methyl-3-(4-((2-(trifluoromethyl)-1H-imidazol-1-yl)methyl)phenyl)thiophen-2-yl)sulfonyl)carbamate

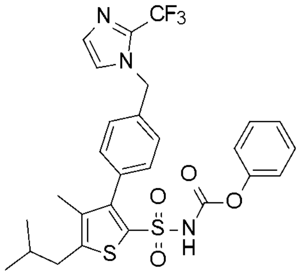

10-1

5-isobutyl-4-methyl-3-(4-((2-(trifluoromethyl)-1H-imidazol-1-yl)methyl)phenyl)thiophene-2-sulfonamide (300 mg, 656 μmol) and diphenyl carbonate (211 mg, 984 μmol) were dissolved in acetonitrile (5 mL), and potassium carbonate (181 mg, 1.31 mmol) was added. The mixture was stirred and reacted at 60°C for 4 hours under a nitrogen atmosphere. The reaction solution was filtered to remove insoluble matter, and the filtrate was concentrated under vacuum to obtain Compound phenyl ((5-isobutyl-4-methyl-3-(4-((2-(trifluoromethyl)-1H-imidazol-1-yl)methyl)phenyl)thiophen-2-yl)sulfonyl)carbamate (370 mg, yield 97.7%).
LC-MS, M/Z (ESI): 578.1 $[M+H]^+$

Step 2: Synthesis of 2-hydroxy-2-methylpropyl ((5-isobutyl-4-methyl-3-(4-((2-(trifluoromethyl)-1H-imidazol-1-yl)methyl)phenyl)thiophen-2-yl)sulfonyl)carbamate

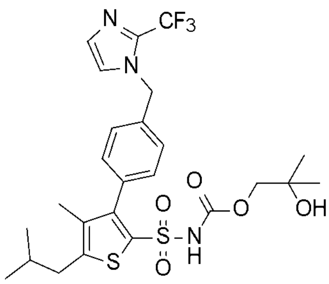

I-10

Phenyl ((5-isobutyl-4-methyl-3-(4-((2-(trifluoromethyl)-1H-imidazol-1-yl)methyl)phenyl)thiophen-2-yl)sulfonyl)carbamate (370 mg, 650 μmol) was dissolved in dioxane (8 mL), and isobutylene glycol (173 mg, 1.92 mmol) was added. The mixture was stirred and reacted at 100°C for 4 hours under a nitrogen atmosphere. The reaction solution was concentrated, and the residue was purified by high performance liquid chromatography (column: C18 150*30mm; mobile phase: A=water+0.05 v/v formic acid (30%), B=acetonitrile; gradient: 58% to 88%, 7 minutes) to obtain the product 2-hydroxy-2-methylpropyl ((5-isobutyl-4-methyl-3-(4-((2-(trifluoromethyl)-1H-imidazol-1-yl)methyl)phenyl)thiophen-2-yl) sulfonyl)carbamate (38.6 mg, yield 10.5%).

LC-MS, M/Z (ESI): 574.1 $[M+H]^+$
$^1$H NMR (400 MHz, $CDCl_3$) δ: 7.29 (s, 1H), 7.21-7.25 (m, 2H), 7.17 (d, 1H), 7.08 (d, 2H), 5.33 (s, 2H), 3.97 (s, 2H), 2.69 (d, 2H), 1.92-2.00 (m, 1H), 1.86 (s, 3H), 1.19 (s, 6H), 1.01 (d, 6H)

**Example 7:** Preparation of Target Compound **I-11**

(2S)-2-hydroxypropyl ((5-isobutyl-4-methyl-3-(4-((2-(trifluoromethyl)-1H-imidazol-1-yl)methyl)phenyl)thiophen-2-yl)sulfonyl)carbamate

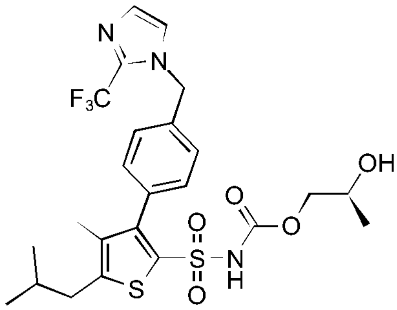

I-11

The synthetic scheme of Target Compound **I-11** is as follows:

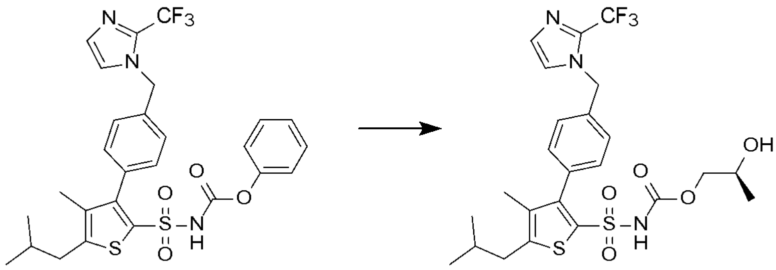

10-1

I-11

Step 1: Synthesis of (2S)-2-hydroxypropyl ((5-isobutyl-4-methyl-3-(4-((2-(trifluoromethyl)-1H-imidazol-1-yl)methyl) phenyl)thiophen-2-yl)sulfonyl)carbamate

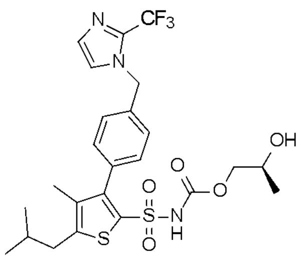

I-11

Phenyl ((5-isobutyl-4-methyl-3-(4-((2-(trifluoromethyl)-1H-imidazol-1-yl)methyl)phenyl)thiophen-2-yl)sulfonyl) carbamate (250 mg, 433 μmol) was dissolved in tetrahydrofuran (2.00 mL), and (2S)-propane-1,2-diol (330 mg, 4.33 mmol) and triethylamine (131 mg, 1.30 mmol) were added. The mixture was stirred and reacted at 20°C for 10 hours under

a nitrogen atmosphere. After the reaction was completed, the reaction solution was diluted with water (20 mL). The mixture was then extracted three times with 20 mL of ethyl acetate each time. The organic phase was collected, dried over anhydrous sodium sulfate, filtered, and concentrated. The crude product was purified by high performance liquid chromatography (column: C18 150*30 mm; mobile phase: A=water+0.05 v/v formic acid (30%), B=acetonitrile; gradient: 55% to 85%, 7 minutes) to obtain the product (2S)-2-hydroxypropyl ((5-isobutyl-4-methyl-3-(4-((2-(trifluoromethyl)-1H-imidazol-1-yl)methyl)phenyl)thiophen-2-yl)sulfonyl)carbamate (79.6 mg, yield 32.8%).

LC-MS, M/Z (ESI): 560.3 $[M+H]^+$
$^1H$ NMR (400 MHz, $CDCl_3$) δ: 7.40-7.45 (m, 1H), 7.20-7.27 (m, 4H), 7.12-7.17 (m, 1H), 7.06-7.10 (m, 1H), 5.33 (s, 2H), 4.05-4.18 (m, 1H), 3.86-4.04 (m, 2H), 2.69 (d, 2H), 1.93-2.02 (m, 1H), 1.84-1.87 (m, 3H), 1.10-1.20 (m, 3H), 1.01 (d, 6H)

**Example 8:** Preparation of Target Compound **I-12**

(2R)-2-hydroxypropyl ((5-isobutyl-4-methyl-3-(4-((2-(trifluoromethyl)-1H-imidazol-1-yl)methyl)phenyl)thiophen-2-yl)sulfonyl)carbamate

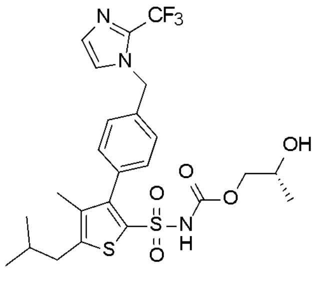

I-12

The synthetic scheme of Target Compound I-12 is as follows:

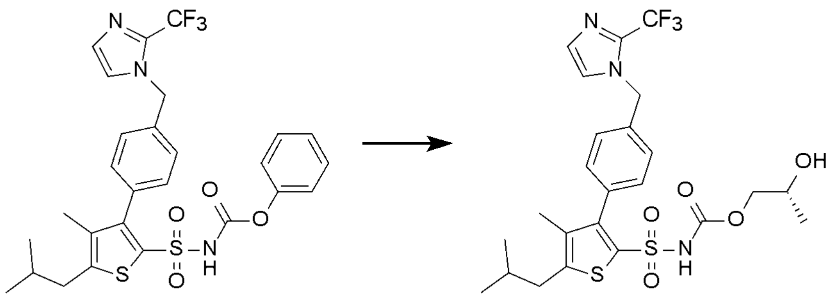

10-1 I-12

Step 1: Synthesis of (2R)-2-hydroxypropyl ((5-isobutyl-4-methyl-3-(4-((2-(trifluoromethyl)-1H-imidazol-1-yl)methyl) phenyl)thiophen-2-yl)sulfonyl)carbamate

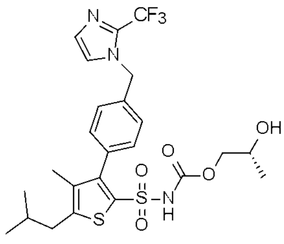

I-12

Phenyl ((5-isobutyl-4-methyl-3-(4-((2-(trifluoromethyl)-1H-imidazol-1-yl)methyl)phenyl)thiophen-2-yl)sulfonyl) carbamate (250 mg, 433 μmol) was dissolved in tetrahydrofuran (2 mL), and (2R)-propane-1,2-diol (330 mg, 4.33 mmol) and triethylamine (131 mg, 1.30 mmol) were added. The mixture was stirred and reacted at 20°C for 10 hours under a nitrogen atmosphere. After the reaction was completed, the reaction solution was diluted with water (20 mL). The mixture was then extracted three times with 20 mL of ethyl acetate each time. The organic phase was collected, dried over anhydrous sodium sulfate, filtered, and concentrated. The crude product was purified by high performance liquid chromatography (column: Welch xextreme C18 150*30 mm; mobile phase: A=water+0.05 v/v formic acid (30%), B=acetonitrile; gradient: 55% to 85%, 7 minutes) to obtain the product (2R)-2-hydroxypropyl ((5-isobutyl-4-methyl-3-(4-((2-(trifluoromethyl)-1H-imidazol-1-yl)methyl)phenyl)thiophen-2-yl)sulfonyl)carbamate (67.7 mg, yield 27.9%).

LC-MS, M/Z (ESI): 560.3 $[M+H]^+$
$^1H$ NMR (400 MHz, $CDCl_3$) δ: 7.39-7.48 (m, 1H), 7.28-7.30 (m, 1H), 7.20-7.26 (m, 3H), 7.12-7.17 (m, 1H), 7.04-7.10 (m, 1H), 5.33 (s, 2H), 4.11 (d, 1H), 3.85-4.03 (m, 2H), 2.69 (d, 2H), 1.97 (dt, 1H), 1.86 (s, 3H), 1.16 (d, 3H), 1.02 (d, 6H)

**Example 9:** Preparation of Target Compound **I-13**

2-hydroxyethyl ((5-isobutyl-4-methyl-3-(4-((2-(trifluoromethyl)-1H-imidazol-1-yl)methyl)phenyl)thiophen-2-yl)sulfonyl) carbamate

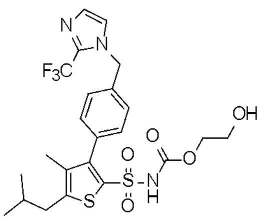

I-13

The synthetic scheme of Target Compound **I-13** is as follows:

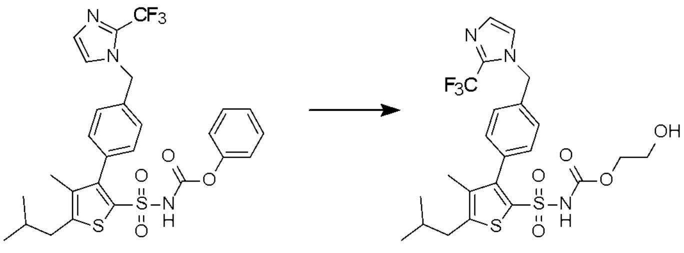

10-1 I-13

Step 1: Synthesis of 2-hydroxyethyl ((5-isobutyl-4-methyl-3-(4-((2-(trifluoromethyl)-1H-imidazol-1-yl)methyl)phenyl) thiophen-2-yl)sulfonyl)carbamate

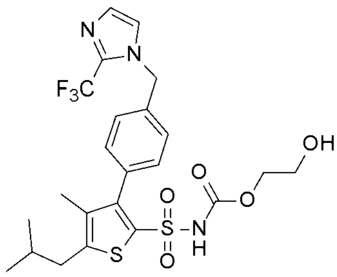

I-13

Phenyl ((5-isobutyl-4-methyl-3-(4-((2-(trifluoromethyl)-1H-imidazol-1-yl)methyl)phenyl)thiophen-2-yl)sulfonyl) carbamate (365 mg, 632 μmol) was dissolved in dioxane (5 mL), and ethylene glycol (118 mg, 1.90 mmol) was added. The mixture was stirred and reacted at 100°C for 2 hours under a nitrogen atmosphere. The reaction solution was concentrated, and the residue was purified by high performance liquid chromatography (column: Welch xextreme C18 150*25mm*5μm; mobile phase: A=water+0.05 v/v formic acid (30%), B=acetonitrile; gradient: 53% to 73%, 10 minutes) to obtain the product 2-hydroxyethyl ((5-isobutyl-4-methyl-3-(4-((2-(trifluoromethyl)-1H-imidazol-1-yl)methyl)phenyl)thiophen-2-yl)sulfonyl)carbamate (38.91 mg, yield 11.3%).

LC-MS, M/Z (ESI): 546.3 $[M+H]^+$
$^1H$ NMR (400 MHz, $CDCl_3$) δ: 7.27-7.30 (d, 1H), 7.26 (d, 1H), 7.21-7.24 (d, 2H), 7.15 (d, 1H), 7.08 (d, 1H), 5.33 (s, 2H), 4.17-4.23 (t, 2H), 3.72-3.77 (t, 2H), 2.69 (d, 2H), 1.97 (m, 1H), 1.86 (s, 3H), 1.02 (d, 6H)

**Example 10:** Preparation of Target Compound **I-14**

Methyl ((3-(4-((2-chloro-1H-imidazol-1-yl)methyl)-3-fluorophenyl)-5-isobutyl-4-methylthiophen-2-yl)sulfonyl)carbamate

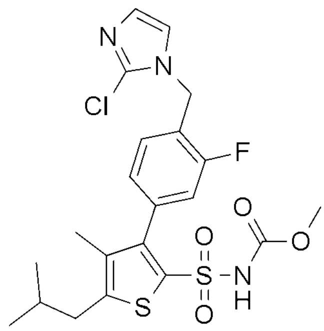

I-14

The synthetic scheme of Target Compound **I-14** is as follows:

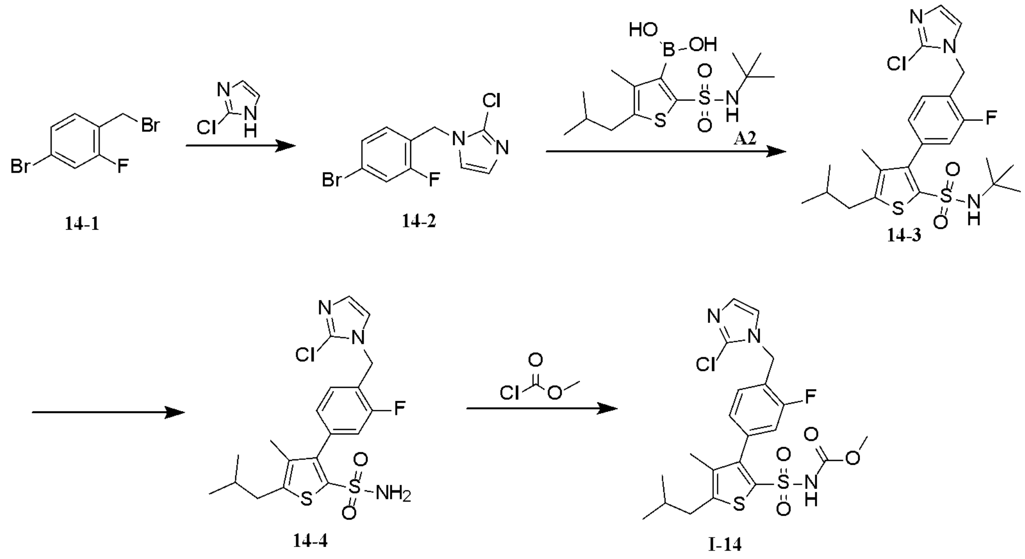

14-1
14-2
14-3
14-4
I-14

Step 1: Synthesis of 1-(4-bromo-2-fluorobenzyl)-2-chloro-1H-imidazole

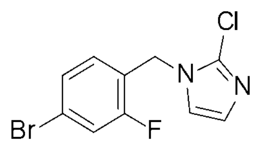

14-2

4-bromo-1-(bromomethyl)-2-fluorobenzene (500 mg, 1.87 mmol) and 2-chloro-1H-imidazole (210 mg, 2.05 mmol) were dissolved in N,N-dimethylformamide (5.00 mL), and potassium carbonate (774 mg, 5.60 mmol) was then added. The mixture reacted at 50°C for 2 hours. After the reaction was completed, the reaction solution was poured into water (20 mL). The mixture was then extracted three times with 40 mL of ethyl acetate each time. The organic phase was collected, washed five times with 20 mL of water each time, dried over anhydrous sodium sulfate, filtered, and concentrated to obtain the crude product. The product was then purified by silica gel column chromatography (petroleum ether: ethyl acetate (V/V)=1:0 to 3:1) to obtain 1-(4-bromo-2-fluorobenzyl)-2-chloro-1H-imidazole (450 mg, yield 83.3%). LC-MS, M/Z (ESI): 290.9 $[M+H]^+$

Step 2: Synthesis of N-(tert-butyl)-3-(4-((2-chloro-1H-imidazol-1-yl)methyl)-3-fluorophenyl)-5-isobutyl-4-methylthiophene-2-sulfonamide

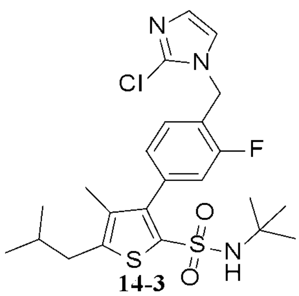

14-3

(2-(N-(tert-butyl)sulfamoyl)-5-isobutyl-4-methylthiophene-3-yl)boronic acid (350 mg, 1.05 mmol) was dissolved in tetrahydrofuran (5.00 mL) and water (1.00 mL), and 1-(4-bromo-2-fluorobenzyl)-2-chloro-1H-imidazole (274 mg, 945 µmol) was added, followed by the addition of potassium phosphate (1.11 mg, 5.25 mmol) and XPos Pd G4 (90.4 mg, 105

μmol). The mixture was stirred and reacted at 60°C for 1 hour under a nitrogen atmosphere. The reaction solution was concentrated to obtain a crude product. The crude product was purified by silica gel column chromatography (petroleum ether: ethyl acetate (V/V)=1:0 to 3:1) to obtain N-(tert-butyl)-3-(4-((2-chloro-1H-imidazol-1-yl)methyl)-3-fluorophenyl)-5-isobutyl-4-methylthiophene-2-sulfonamide (450 mg, yield 86.0%).
LC-MS, M/Z (ESI): 498.2 $[M+H]^+$

Step 3: Synthesis of 3-(4-((2-chloro-1H-imidazol-1-yl)methyl)-3-fluorophenyl)-5-isobutyl-4-methylthiophene-2-sulfonamide

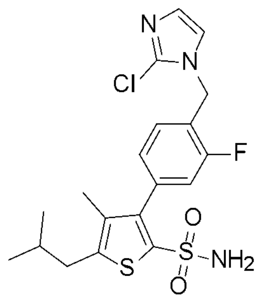

14-4

N-(tert-butyl)-3-(4-((2-chloro-1H-imidazol-1-yl)methyl)-3-fluorophenyl)-5-isobutyl-4-methylthiophene-2-sulfonamide (450 mg, 904 μmol) was dissolved in trifluoroacetic acid (4.00 mL) and dichloromethane (4.00 mL), and the mixture reacted at 40°C for 1 hour. The reaction solution was poured into water (20 mL), and the mixture was then extracted three times with 40 mL of dichloromethane each time. The organic phase was collected, dried over anhydrous sodium sulfate, filtered, and concentrated. The residue was then purified by silica gel column chromatography (petroleum ether: ethyl acetate (V/V)=1:0 to 0:1) to obtain 3-(4-((2-chloro-1H-imidazol-1-yl)methyl)-3-fluorophenyl)-5-isobutyl-4-methylthiophen-2-sulfonamide (350 mg, yield 87.7%).
LC-MS, M/Z (ESI): 442.1 $[M+H]^+$

Step 4: Synthesis of methyl ((3-(4-((2-chloro-1H-imidazol-1-yl)methyl)-3-fluorophenyl)-5-isobutyl-4-methylthiophen-2-yl)sulfonyl)carbamate

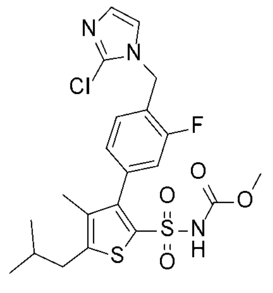

I-14

At 0°C, 3-(4-((2-chloro-1H-imidazol-1-yl)methyl)-3-fluorophenyl)-5-isobutyl-4-methylthiophene-2-sulfonamide (350 mg, 792 μmol) and methyl chloroformate (150 mg, 1.58 mmol) were dissolved in dichloromethane (5.00 mL), and N,N-diisopropylethylamine (307 mg, 2.38 mmol) was added. The mixture was stirred and reacted at 0°C for 0.5 hours. The reaction solution was concentrated, and the residue was purified by high performance liquid chromatography (column: Waters Xbridge 150*30 mm; solvent: A=water+0.05 v/v formic acid (30%), B=acetonitrile; gradient: 55% to 85%, 7 minutes) to obtain methyl ((3-(4-((2-chloro-1H-imidazol-1-yl)methyl)-3-fluorophenyl)-5-isobutyl-4-methylthiophen-2-yl) sulfonyl)carbamate (50.0 mg, yield 12.6%).

LC-MS, M/Z (ESI): 500.0 $[M+H]^+$
$^1H$ NMR (400 MHz, $CDCl_3$) δ 7.76 (s, 1H), 7.16 (t, 1H), 7.02-7.08 (m, 3H), 7.00 (s, 1H), 5.22 (s, 2H), 3.72 (s, 3H), 2.69

(d, 2H), 1.92-2.00 (m, 1H), 1.87 (s, 3H), 1.02 (d, 6H)

**Example 11:** Preparation of Target Compound **I-18**

2-hydroxyethyl ((3-(4-((2-chloro-1H-imidazol-1-yl)methyl)phenyl)-5-isobutyl-4-methylthiophen-2-yl)sulfonyl)carbamate

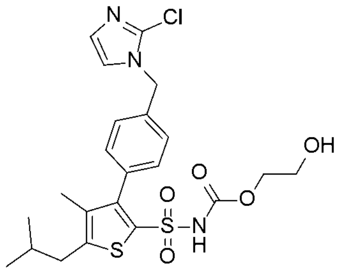

**I-18**

The synthetic scheme of the Target Compound **I-18** is as follows:

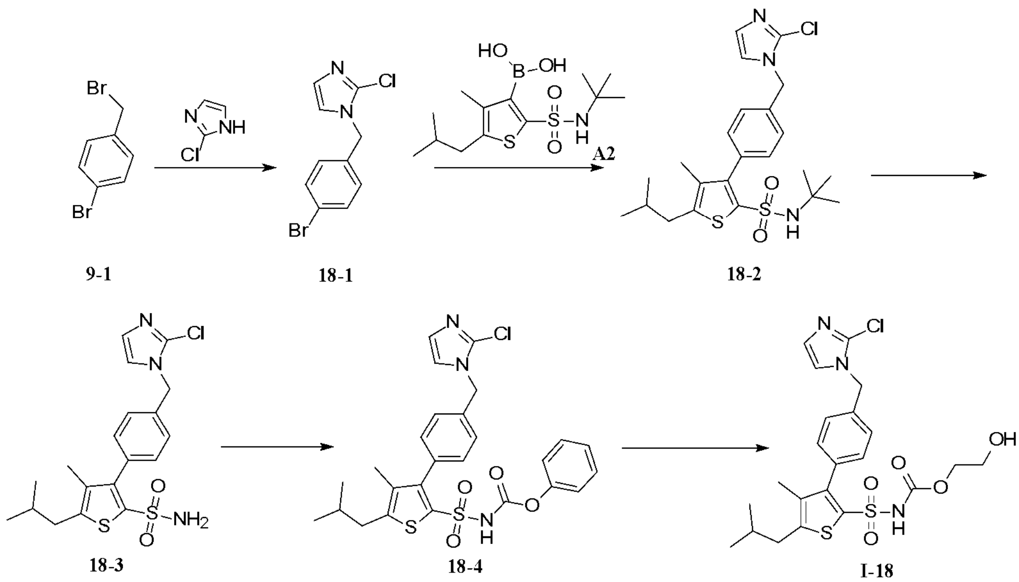


Step 1: Synthesis of 1-(4-bromobenzyl)-2-chloro-1H-imidazole

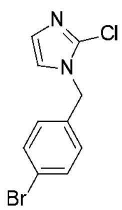

**18-1**

1-bromo-4-(bromomethyl)benzene (1.00 g, 4.00 mmol) and 2-chloro-1H-imidazole (410 mg, 4.00 mmol) were dissolved in N,N-dimethylformamide (20 mL), and potassium carbonate (1.66 g, 12.0 mmol) was added. The mixture reacted at 55°C for 1 hour. The reaction solution was poured into water (50 mL), and the mixture was extracted three times with 50 mL of ethyl acetate each time. The organic phase was collected, dried over anhydrous sodium sulfate, filtered, and concentrated. The crude product was purified by silica gel column chromatography (petroleum ether: ethyl acetate (V/V)=50:1 to 1:1) to obtain 1-(4-bromobenzyl)-2-chloro-1H-imidazole (900 mg, yield 82.8%).
LC-MS, M/Z (ESI): 271.0 [M+H]+

Step 2: Synthesis of N-(tert-butyl)-3-(4-((2-chloro-1H-imidazol-1-yl)methyl)phenyl)-5-isobutyl-4-methylthiophene-2-sulfonamide

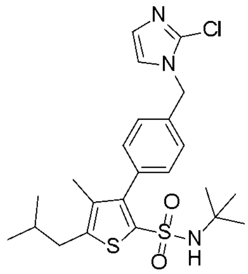

18-2

(2-(N-(tert-butyl)sulfamoyl)-5-isobutyl-4-methylthiophene-3-yl)boronic acid (400 mg, 1.20 mmol) was dissolved in tetrahydrofuran (6 mL) and water (2 mL), and 1-(4-bromobenzyl)-2-chloro-1H-imidazole (293 mg, 1.08 mmol) was added, followed by the addition of potassium phosphate (1.27 g, 6.00 mmol) and XPhos Pd G4 (103 mg, 120 μmol). Then the mixture was stirred and reacted at 60°C for 2 hours under a nitrogen atmosphere. The reaction solution was concentrated to obtain a crude product. The crude product was then purified by silica gel column chromatography (petroleum ether: ethyl acetate (V/V)=50:1 to 0:1) to obtain N-(tert-butyl)-3-(4-((2-chloro-1H-imidazol-1-yl)methyl)phenyl)-5-isobutyl-4-methylthiophene-2-sulfonamide (510 mg, yield 88.5%).
LC-MS, M/Z (ESI): 480.2 [M+H]+

Step 3: Synthesis of 3-(4-((2-chloro-1H-imidazol-1-yl)methyl)phenyl)-5-isobutyl-4-methylthiophene-2-sulfonamide

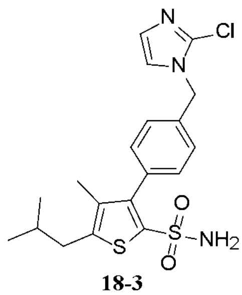

18-3

N-(tert-butyl)-3-(4-((2-chloro-1H-imidazol-1-yl)methyl)phenyl)-5-isobutyl-4-methylthiophene-2-sulfonamide (500 mg, 1.04 mmol) was dissolved in trifluoroacetic acid (15 mL) and dichloromethane (5 mL), and the mixture reacted at 40°C for 1 hour. The reaction solution was poured into water (10 mL), and the mixture was then extracted three times with 20 mL of dichloromethane each time. The organic phase was collected, dried over anhydrous sodium sulfate, filtered, and concentrated. The crude product was purified by silica gel column chromatography (petroleum ether: ethyl acetate (V/V) =10:1 to 1:1) to obtain 3-(4-((2-chloro-1H-imidazol-1-yl)methyl)phenyl)-5-isobutyl-4-methylthiophene-2-sulfonamide as a brown oily compound (400 mg, yield 90.6%).
LC-MS, M/Z (ESI): 424.1 [M+H]+

Step 4: Synthesis of phenyl ((3-(4-((2-chloro-1H-imidazol-1-yl)methyl)phenyl)-5-isobutyl-4-methylthiophen-2-yl)sulfonyl)carbamate

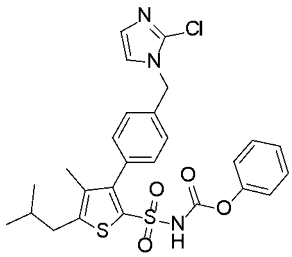

18-4

3-(4-((2-chloro-1H-imidazol-1-yl)methyl)phenyl)-5-isobutyl-4-methylthiophene-2-sulfonamide (200 mg, 472 μmol) and potassium carbonate (131 mg, 944 μmol) were dissolved in acetonitrile (2 mL), and diphenyl carbonate (152 mg, 708 μmol) was added. The mixture was stirred and reacted at 50°C for 2 hours. The reaction solution was poured into water (20 mL), and the mixture was then extracted three times with 20 mL of ethyl acetate each time. The organic phase was collected, dried over anhydrous sodium sulfate, filtered, and concentrated to obtain phenyl ((3-(4-((2-chloro-1H-imidazol-1-yl)methyl)phenyl)-5-isobutyl-4-methylthiophen-2-yl)sulfonyl)carbamate (240 mg, yield 93.5%).
LC-MS, M/Z (ESI): 544.2 $[M+H]^+$

Step 5: Synthesis of 2-hydroxyethyl ((3-(4-((2-chloro-1H-imidazol-1-yl)methyl)phenyl)-5-isobutyl-4-methylthiophen-2-yl)sulfonyl)carbamate

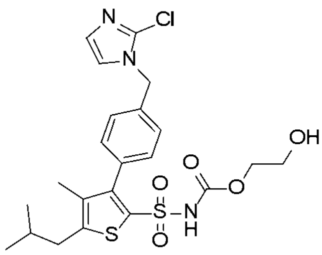

I-18

Phenyl ((3-(4-((2-chloro-1H-imidazol-1-yl)methyl)phenyl)-5-isobutyl-4-methylthiophen-2-yl)sulfonyl)carbamate (240 mg, 441 μmol) was dissolved in tetrahydrofuran (3 mL), and ethylene glycol (274 mg, 4.41 mmol) and triethylamine (134 mg, 1.32 mmol) were added. The mixture was stirred and reacted at 25°C for 10 hours. The reaction solution was poured into water (20 mL), and the mixture was then extracted three times with 20 mL of ethyl acetate each time. The organic phase was collected, dried over anhydrous sodium sulfate, filtered, and concentrated. The crude product was purified by high performance liquid chromatography (column: C18 150*30 mm; solvent: A=water+0.05 v/v formic acid (30%), B=acetonitrile; gradient: 48% to 78%, 7 minutes) to obtain the product 2-hydroxyethyl ((3-(4-((2-chloro-1H-imidazol-1-yl)methyl)phenyl)-5-isobutyl-4-methylthiophen-2-yl)sulfonyl)carbamate (115 mg, yield 50.9%).

LC-MS, M/Z (ESI): 512.2 $[M+H]^+$
$^1H$ NMR (400 MHz, $CDCl_3$) δ 7.22-7.27 (m, 4H), 6.97 (s, 2H), 5.17 (s, 2H), 4.18-4.22 (m, 2H), 3.74 (dd, 2H), 2.69 (d, 2H), 1.93-1.99 (m, 1H), 1.87 (s, 3H), 1.02 (d, 6H).

**Example 12:** Preparation of Target Compound **I-19**

Methyl ((3-(4-((2-(2-hydroxypropan-2-yl)-1H-imidazol-1-yl)methyl)phenyl)-5-isobutyl-4-methylthiophen-2-yl)sulfonyl) carbamate

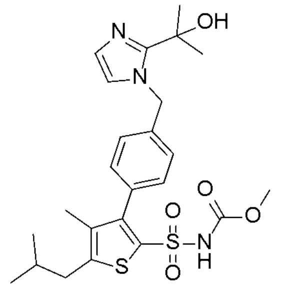

I-19

The synthetic scheme of Target Compound **I-19** is as follows:

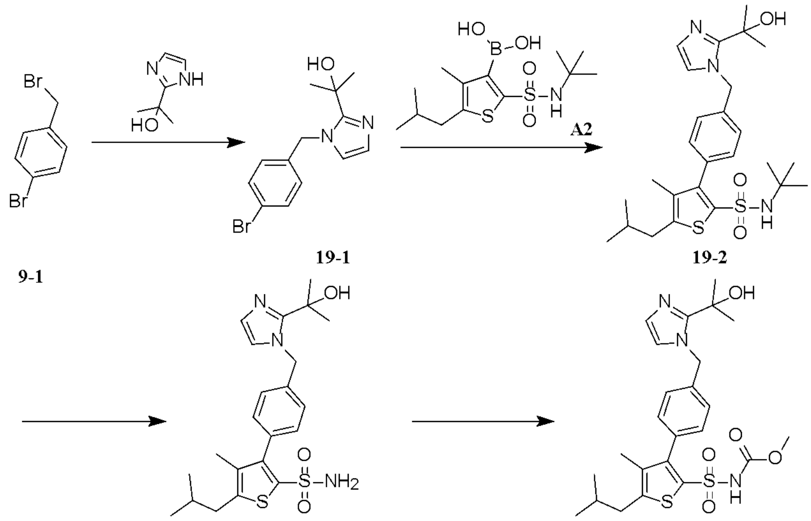


9-1 19-1 19-2

19-3 I-19

Step 1: Synthesis of 2-(1-(4-bromobenzyl)-1H-imidazol-2-yl)propan-2-ol

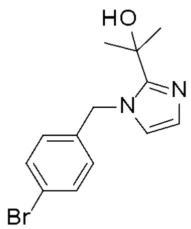

19-1

1-bromo-4-(bromomethyl)benzene (1.00 g, 4.00 mmol) and 2-(1H-imidazol-2-yl)propane-2-ol (505 mg, 4.00 mmol) were dissolved in N,N-dimethylformamide (20 mL), and then potassium carbonate (1.66 mg, 12.0 mmol) was added. The mixture reacted at 50°C for 2 hours. After the reaction was complete, the reaction solution was poured into water (40 mL). The mixture was extracted three times with 40 mL of ethyl acetate each time. The organic phase was collected, washed five times with 40 mL of water each time, dried over anhydrous sodium sulfate, filtered, and concentrated. The crude product was purified by silica gel column chromatography (petroleum ether: ethyl acetate (V/V)=1:1 to ethyl acetate: methanol (V/V)=10:1) to obtain 2-(1-(4-bromophenylmethyl)-1H-imidazol-2-yl)propane-2-ol (830 mg, yield 70.3%).
LC-MS, M/Z (ESI): 295.0 $[M+H]^+$

Step 2: Synthesis of N-(tert-butyl)-3-(4-((2-(2-hydroxypropane-2-yl)-1H-imidazol-1-yl)methyl)phenyl)-5-isobutyl-4-methylthiophene-2-sulfonamide

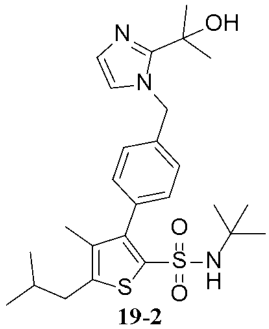

19-2

(2-(N-(tert-butyl)sulfamoyl)-5-isobutyl-4-methylthiophen-3-yl)boronic acid (350 mg, 1.05 mmol) was dissolved in tetrahydrofuran (7 mL) and water (3 mL), 2-(1-(4-bromobenzyl)-1H-imidazol-2-yl)propan-2-ol (279 mg, 945 μmol) was added, followed by the addition of potassium phosphate (1.11 g, 5.25 mmol) and XPos Pd G4 (90.4 mg, 105 μmol). The mixture was stirred and reacted at 60°C for 2 hours under a nitrogen atmosphere. After the reaction was completed, the reaction solution was concentrated to obtain a crude product. The crude product was then purified by silica gel column chromatography (petroleum ether: ethyl acetate (V/V)=1:0 to 0:1) to obtain N-(tert-butyl)-3-(4-((2-(2-hydroxypropane-2-yl)-1H-imidazol-1-yl)methyl)phenyl)-5-isobutyl-4-methylthiophene-2-sulfonamide (420 mg, yield 79.4%).
LC-MS, M/Z (ESI): 504.1 $[M+H]^+$

Step 3: Synthesis of 3-(4-((2-(2-hydroxypropane-2-yl)-1H-imidazol-1-yl)methyl)phenyl)-5-isobutyl-4-methylthiophene-2-sulfonamide

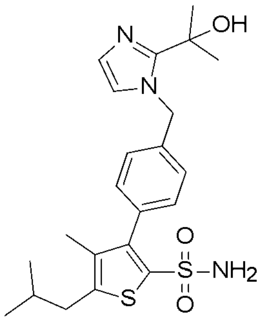

19-3

N-(tert-butyl)-3-(4-((2-(2-hydroxypropan-2-yl)-1H-imidazol-1-yl)methyl)phenyl)-5-isobutyl-4-methylthiophene-2-sulfonamide (410 mg, 814 μmol) was dissolved in trifluoroacetic acid (8 mL) and dichloromethane (3 mL), and the mixture reacted at 40°C for 2 hours. The reaction solution was concentrated to obtain a crude product. The crude product was purified by silica gel column chromatography (petroleum ether: ethyl acetate (V/V)=0:1 to dichloromethane: methanol (V/V)=5:1) to obtain Compound 3-(4-((2-(2-hydroxypropan-2-yl)-1H-imidazol-1-yl)methyl)phenyl)-5-isobutyl-4-methylthiophene-2-sulfonamide (500 mg, crude product).
LC-MS, M/Z (ESI): 448.2 $[M+H]^+$

Step 4: Synthesis of methyl ((3-(4-((2-(2-hydroxypropan-2-yl)-1H-imidazol-1-yl)methyl)phenyl)-5-isobutyl-4-methylthiophen-2-yl)sulfonyl)carbamate

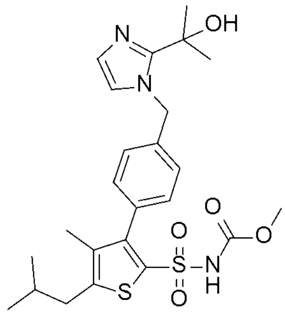

I-19

3-(4-((2-(2-hydroxypropan-2-yl)-1H-imidazol-1-yl)methyl)phenyl)-5-isobutyl-4-methylthiophene-2-sulfonamide (500 mg, 1.12 mmol) was dissolved in dichloromethane (8 mL), and N,N-diisopropylethylamine (433 mg, 3.35 mmol) and methyl chloroformate (211 mg, 2.23 mmol) were added at 0°C. The mixture was stirred and reacted for 0.5 hours. After the reaction was completed, the reaction solution was poured into a saturated aqueous sodium bicarbonate solution (20 mL), and the mixture was extracted three times with 20 mL of dichloromethane each time. The organic phase was collected, dried over anhydrous sodium sulfate, filtered, and concentrated. The crude product was purified by high performance liquid chromatography (column: Welch Xtimate C18 150*25mm*5μm; solvent: A=water+0.05 v/v formic acid (30%), B=acetonitrile; gradient: 23% to 43%, 10 minutes), to obtain the product methyl ((3-(4-((2-(2-hydroxypropan-2-yl)-1H-imidazol-1-yl)methyl)phenyl)-5-isobutyl-4-methylthiophen-2-yl)sulfonyl)carbamate (168 mg, yield 39.9%).

LC-MS, M/Z (ESI): 506.2 $[M+H]^+$
$^1H$ NMR (400 MHz, $CD_3OD$) δ: 7.28-7.31 (m, 2H), 7.24-7.27 (m, 2H), 7.16 (d, 1H), 7.08 (d, 1H), 5.68 (s, 2H), 3.54 (s, 3H), 2.70 (d, 2H), 1.91-1.98 (m, 1H), 1.85 (s, 3H), 1.66 (s, 6H), 1.01 (d, 6H)

**Example 13:** Preparation of Target Compound **I-21**

Methyl ((3-(4-(((2-chloro-1H-imidazol-1-yl)methyl)-2,6-difluorophenyl)-5-isobutyl-4-methylthiophen-2-yl)sulfonyl)carbamate

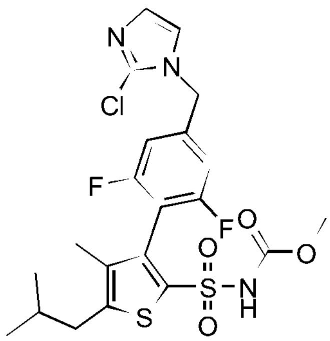

I-21

The synthetic scheme of Target Compound **I-21** is as follows:

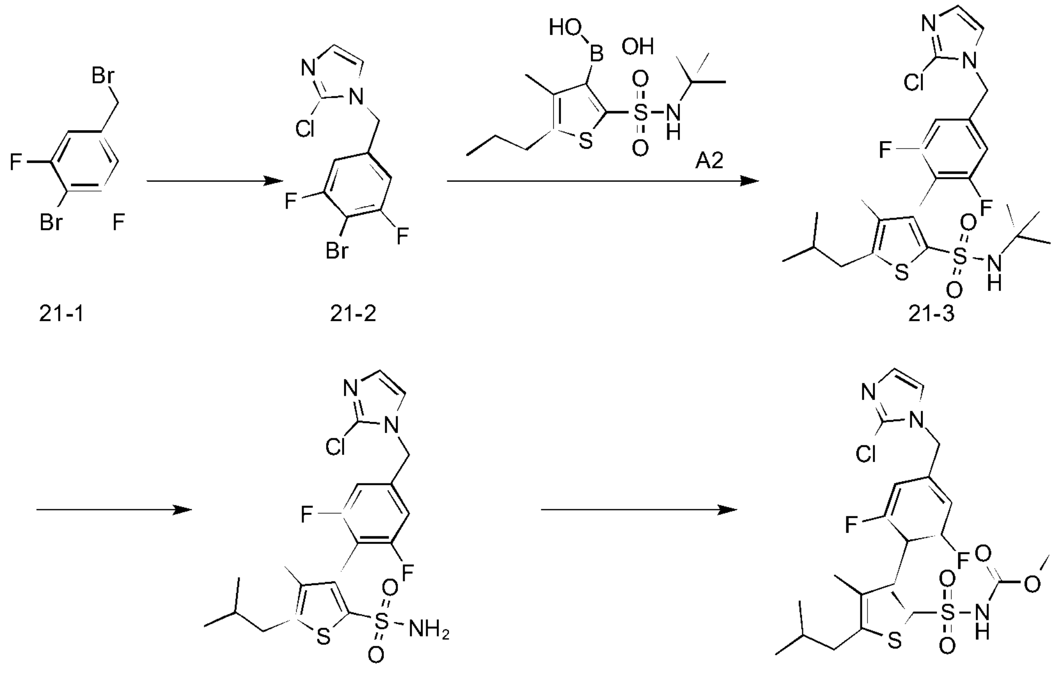


21-1 21-2 21-3

21-4 I-21

Step 1: Synthesis of 1-(4-bromo-3,5-difluorobenzyl)-2-chloro-1H-imidazole

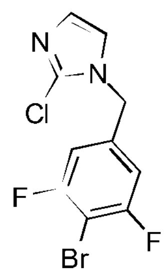

21-2

2-bromo-5-(bromomethyl)-1,3-difluorobenzene (500 mg, 1.75 mmol) and 2-chloro-1H-imidazole (197 mg, 1.92 mmol) were dissolved in N,N-dimethylformamide (5 mL), and then potassium carbonate (725 mg, 5.25 mmol) was added. The mixture reacted at 25°C for 1 hour. The reaction solution was poured into water (30 mL), and the mixture was then extracted three times with 60 mL of ethyl acetate each time, and washed five times with 60 mL of water each time. The organic phase was collected, dried over anhydrous sodium sulfate, filtered, and concentrated to obtain the crude product. Then, the product was purified by silica gel column chromatography (petroleum ether: ethyl acetate (V/V)=1:0 to 3:1) to obtain 1-(4-bromo-3,5-difluorobenzyl)-2-chloro-1H-imidazole (520 mg, yield 96.7%).
LC-MS, M/Z (ESI): 308.9 $[M+H]^+$

Step 2: Synthesis of N-(tert-butyl)-3-(4-((2-chloro-1H-imidazol-1-yl)methyl)-2,6-difluorophenyl)-5-isobutyl-4-methylthiophene-2-sulfonamide

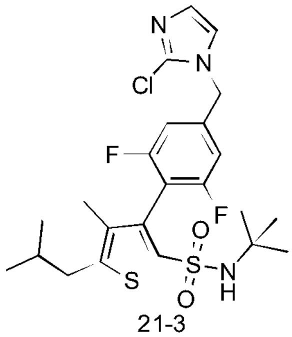

21-3

(2-(N-(tert-butyl)sulfamoyl)-5-isobutyl-4-methylthiophen-3-yl)boronic acid (500 mg, 1.50 mmol) was dissolved in tetrahydrofuran (5 mL) and water (1 mL), and 1-(4-bromo-3,5-difluorobenzyl)-2-chloro-1H-imidazole (415 mg, 1.35 mmol) was added, followed by the addition of potassium phosphate (955 mg, 4.50 mmol) and SPhos Pd G3 (117 mg, 150 μmol). The mixture was stirred and reacted at 60°C for 1 hour under a nitrogen atmosphere. The reaction solution was concentrated. The residue was purified by high performance liquid chromatography (column: Waters Xbridge 150*30 mm; solvent: A=water+0.05 v/v formic acid (30%), B=acetonitrile; gradient: 65% to 95%, 7 minutes), and dried to obtain N-(tert-butyl)-3-(4-((2-chloro-1H-imidazol-1-yl)methyl)-2,6-difluorophenyl)-5-isobutyl-4-methylthiophene-2-sulfonamide (150 mg, yield 19.4%).
LC-MS, M/Z (ESI): 516.1 $[M+H]^+$

Step 3: Synthesis of 3-(4-((2-chloro-1H-imidazol-1-yl)methyl)-2,6-difluorophenyl)-5-isobutyl-4-methylthiophene-2-sulfonamide

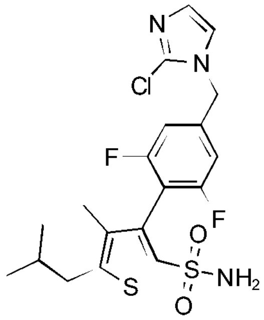

21-4

N-(tert-butyl)-3-(4-((2-chloro-1H-imidazol-1-yl)methyl)-2,6-difluorophenyl)-5-isobutyl-4-methylthiophene-2-sulfonamide (150 mg, 291 μmol) was dissolved in trifluoroacetic acid (3 mL) and dichloromethane (3 mL), and the mixture reacted at 40°C for 1 hour. The reaction solution was poured into water (10 mL), and the mixture was then extracted three times with 20 mL of dichloromethane each time. The organic phase was collected, dried over anhydrous sodium sulfate, filtered, and concentrated. Then, the residue was purified by silica gel column chromatography (petroleum ether: ethyl acetate (V/V)=1:0 to 0:1) to obtain 3-(4-((2-chloro-1H-imidazol-1-yl)methyl)-2,6-difluorophenyl)-5-isobutyl-4-methylthiophene-2-sulfonamide (100 mg, yield 74.8%).
LC-MS, M/Z (ESI): 460.0 $[M+H]^+$

Step 4: Synthesis of methyl ((3-(4-((2-chloro-1H-imidazol-1-yl)methyl)-2,6-difluorophenyl)-5-isobutyl-4-methylthiophen-2-yl)sulfonyl)carbamate

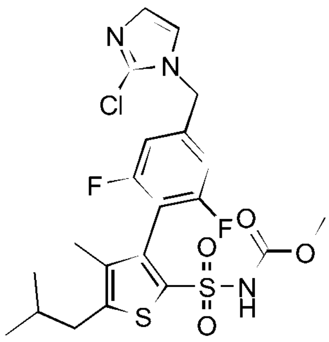

I-21

3-(4-((2-chloro-1H-imidazol-1-yl)methyl)-2,6-difluorophenyl)-5-isobutyl-4-methylthiophene-2-sulfonamide (100 mg, 217 μmol) and methyl chloroformate (82.2 mg, 870 μmol) were dissolved in dichloromethane (2 mL), and N,N-diisopropylethylamine (84.3 mg, 652 μmol) was added. The mixture was stirred and reacted at 25°C for 0.5 hours. The reaction solution was concentrated, and the residue was purified by high performance liquid chromatography (column: Waters Xbridge 150*25mm*5μm; solvent: A=water+0.05 volume ammonia (30%), B=acetonitrile; gradient: 5% to 35%, 10 min), and dried to obtain methyl ((3-(4-((2-chloro-1H-imidazol-1-yl)methyl)-2,6-difluorophenyl)-5-isobutyl-4-methylthiophen-2-yl)sulfonyl)carbamate (4.00 mg, yield 3.57%).

LC-MS, M/Z (ESI): 518.2 $[M+H]^+$
$^1H$ NMR (400 MHz, $CDCl_3$) δ 7.07 (d, 1H), 7.01 (d, 1H), 6.78 (d, 2H), 5.17 (s, 2H), 3.74 (s, 3H), 2.71 (d, 2H), 1.97-2.05 (m, 1H), 1.89 (s, 3H), 1.01 (d, 6H)

**Example 14:** Preparation of Target Compound **I-22**

N-((5-isobutyl-4-methyl-3-(4-((2-(trifluoromethyl)-1H-imidazol-1-yl)methyl)phenyl)thiophen-2-yl)sulfonyl)-2-oxa-6-azaspiro[3.3]heptane-6-carboxamide

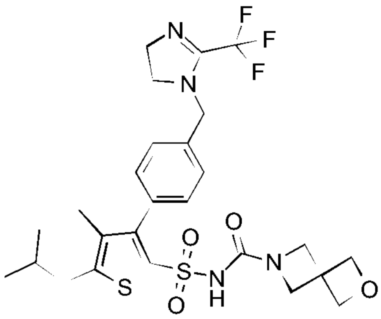

I-22

The synthetic scheme of Target Compound I-22 is as follows:

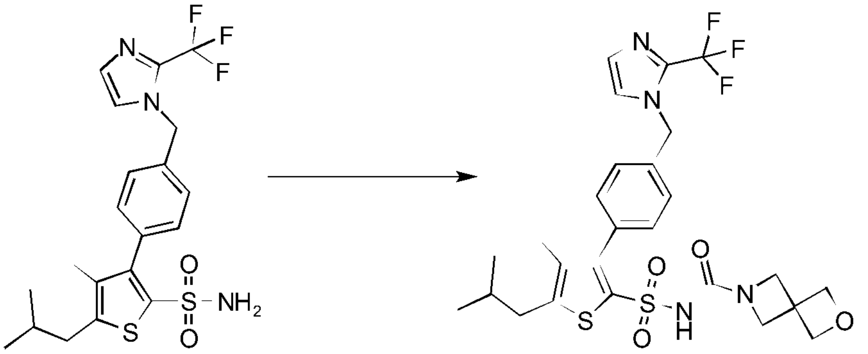

9-4 I-22

Step 1: Synthesis of N-((5-isobutyl-4-methyl-3-(4-((2-(trifluoromethyl)-1H-imidazol-1-yl)methyl)phenyl)thiophen-2-yl) sulfonyl)-2-oxa-6-azaspiro[3.3]heptane-6-carboxamide

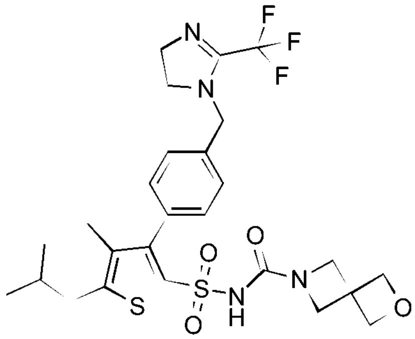

I-22

5-isobutyl-4-methyl-3-(4-((2-(trifluoromethyl)-1H-imidazol-1-yl)methyl)phenyl)thiophene-2-sulfonamide (200 mg, 437 μmol) and 1,1'-carbonyldiimidazole (106 mg, 656 μmol) were dissolved in dichloromethane (4 mL), and N,N-diisopropylethylamine (226 mg, 1.75 mmol) was added. The mixture was stirred and reacted at room temperature for 4 hours. The above reaction solution was mixed with 2-oxa-6-azaspiro[3.3]heptane (43.3 mg, 437 μmol) and dissolved in dichloromethane (2 mL), and N,N-diisopropylethylamine (328 mg, 2.54 mmol) was added. The mixture reacted at 60°C for 8 hours. The reaction solution was concentrated, and the residue was purified by high performance liquid chromatography (column: Waters Xbridge 150*25mm*5μm; mobile phase: A=water+0.05 volume ammonium hydroxide (30%), B=acetonitrile; gradient: 10% to 40%, over 10 minutes), and dried to obtain N-((5-isobutyl-4-methyl-3-(4-((2-(trifluoromethyl)-1H-imidazol-1-yl)methyl)phenyl)thiophen-2-yl)sulfonyl)-2-oxa-6-azaspiro[3.3]heptane-6-carboxamide (126 mg, yield 49.6%).

LC-MS, M/Z (ESI): 583.3 $[M+H]^+$
$^1H$ NMR (400 MHz DMSO-$d_6$) δ: 7.65 (s, 1H), 7.08-7.32 (m, 6H), 5.44 (s, 2H), 4.55 (s, 4H), 3.64-3.87 (s, 4H), 2.63 (d, 2H), 1.80-1.88 (m, 1H), 1.74 (s, 3H), 0.94 (d, 6H).

**Example 15:** Preparation of Target Compound **I-23**

3-(4-((2-chloro-1H-imidazol-1-yl)methyl)phenyl)-5-isobutyl-4-methyl-N-(pyrimidin-2-yl)thiophene-2-sulfonamide

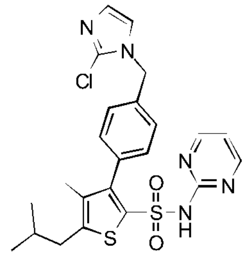

I-23

The synthetic scheme of Target Compound **I-23** is as follows:

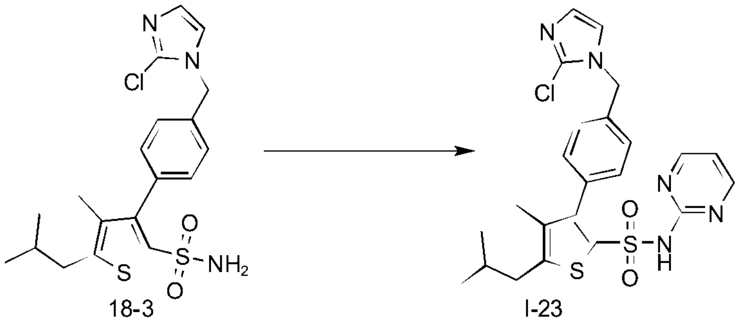

18-3 I-23

Step 1: 3-(4-((2-chloro-1H-imidazol-1-yl)methyl)phenyl)-5-isobutyl-4-methyl-N-(pyrimidin-2-yl)thiophene-2-sulfonamide

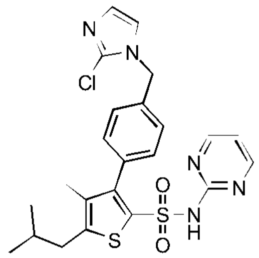

I-23

3-(4-((2-chloro-1H-imidazol-1-yl)methyl)phenyl)-5-isobutyl-4-methylthiophene-2-sulfonamide (150 mg, 354 μmol) and 2-chloropyrimidine (40.5 mg, 354 μmol) were dissolved in dioxane (6 mL), and cesium carbonate (288 mg, 884 μmol) and XPhos Pd G3 (30.0 mg, 35.4 μmol) were added. The mixture was stirred and reacted at 100°C for 1 hour under a nitrogen atmosphere. The reaction solution was concentrated, and the crude product was slurried with methanol (50 mL) at 25°C for 30 minutes to obtain 3-(4-((2-chloro-1H-imidazol-1-yl)methyl)phenyl)-5-isobutyl-4-methyl-N-(pyrimidin-2-yl)thiophene-2-sulfonamide (48.1 mg, yield 26.9%).

LC-MS, M/Z (ESI): 502.1 $[M+H]^+$

$^1H$ NMR (400 MHz DMSO-$d_6$) δ: 8.46 (d, 2H), 7.45 (d, 1H), 7.16-7.24 (d, 2H), 7.08-7.16 (d, 2H), 7.02 (t, 1H), 6.96 (d, 1H), 5.23 (s, 2H), 2.62-2.67 (d, 2H), 1.85 (m, 1H), 1.74 (s, 3H), 0.93 (d, 6H)

**Example 16:** Preparation of Target Compound **I-24**

N-((4-methyl-5-isopropyl-3-(4-((2-(trifluoromethyl)imidazol-1-yl)methyl)phenyl)thiophen-2-yl)sulfonyl)-1-((pyridin-2-ylmethyl)amino)formamide

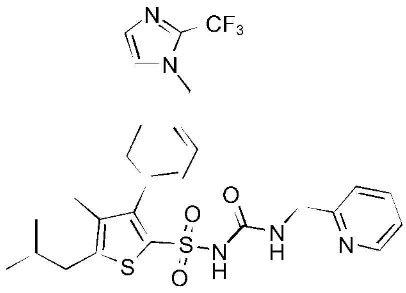

I-24

The synthetic scheme of Target Compound 1-24 is as follows:

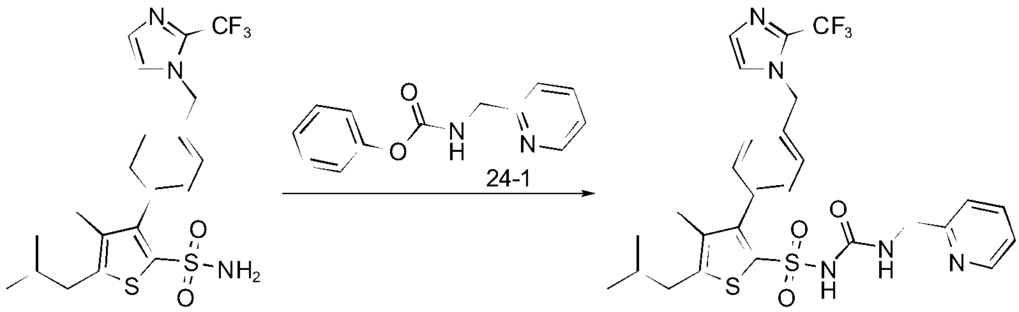


9-4 I-24

Step 1: Synthesis of N-((4-methyl-5-isopropyl-3-(4-((2-(trifluoromethyl)imidazol-1-yl)methyl)phenyl)thiophen-2-yl)sulfonyl)-1-((pyridin-2-ylmethyl)amino)formamide

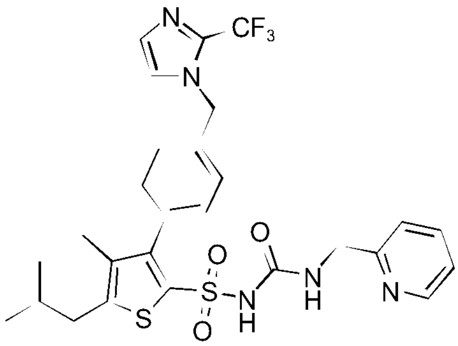

I-24

Under an ice-bath cooling condition, phenyl N-(pyridin-2-ylmethyl) carbamate (0.182 g, 0.8 mmol) and 1,8-diazabicyclo[5.4.0]undec-7-ene (0.152 g, 1 mmol) were added sequentially to a solution of 5-isobutyl-4-methyl-3-(4-((2-(trifluoromethyl)-1H-imidazol-1-yl)methyl)phenyl)thiophene-2-sulfonamide (0.228 g, 0.5 mmol) in acetonitrile (11 mL), and the mixture reacted for 6 hours at 80°C. The reaction was monitored by LCMS. After no starting material remained, the reaction solution was cooled to room temperature, extracted three times with 30 mL of ethyl acetate each time, washed three times with 20 mL of water each time, washed once with saturated brine (50 mL), dried over anhydrous sodium sulfate, and concentrated. Then, the crude product was purified by preparative high performance liquid chromatography (formic acid aqueous solution/acetonitrile system) to obtain N-((4-methyl-5-isopropyl-3-(4-((2-(trifluoromethyl)-imidazol-1-yl)methyl)phenyl)thiophen-2-yl)sulfonyl)-1-((pyridin-2-ylmethyl)amino)formamide (0.128 g, yield 43.4%).

LC-MS, M/Z (ESI): 592.1 $[M+H]^+$

$^1H$ NMR (400 MHz, DMSO-$d_6$) δ 10.50 (s, 1H), 8.50-845 (m, 1H), 7.73 (td, J=7.7, 1.8 Hz, 1H), 7.61 (d, J=1.1 Hz, 1H),

7.28-7.23 (m, 1H), 7.23-7.15 (m, 6H), 6.75 (t, J=5.5 Hz, 1H), 5.40 (s, 2H), 4.25 (d, J=5.6 Hz, 2H), 2.64 (d, J=7.1 Hz, 2H), 1.90-1.78 (m, 1H), 1.74 (s, 3H), 0.92 (d, J=6.6 Hz, 6H).

**Example** 17: The following compounds can be prepared by referring to the preparation methods of the compounds described above.

| Number | Structural Formula | Name | LC-MS, M/Z (ESI) |
|---|---|---|---|
| I-5 | 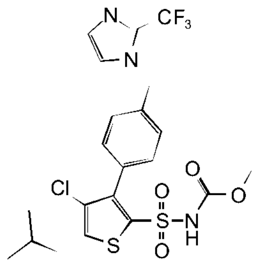 | Methyl ((4-chloro-5-isobutyl-3-(4-((2-(trifluoromethyl)-1H-imidazol-1-yl)methyl)phenyl)thiophen-2-yl)sulfonyl)carbamate | 537.0 |
| I-6 | 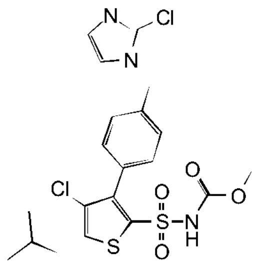 | Methyl ((4-chloro-5-isobutyl-3-(4-((2-chloro-1H-imidazol-1-yl)methyl)phenyl)thiophen-2-yl)sulfonyl)carbamate | 503.4 |
| I-7 | 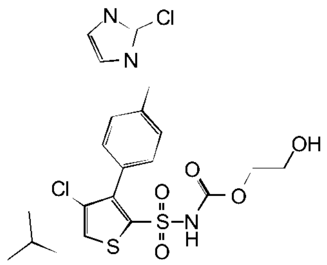 | 2-hydroxyethyl ((4-chloro-3-(4-((2-chloro-1H-imidazol-1-yl)methyl)phenyl)-5-isobutylthiophen-2-yl)sulfonyl)carbamate | 533.5 |
| I-8 | 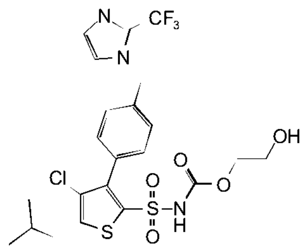 | 2-hydroxyethyl ((4-chloro-5-isobutyl-3-(4-((2-(trifluoromethyl)-1H-imidazol-1-yl)methyl)phenyl)thiophen-2-yl)sulfonyl)carbamate | 567.0 |
| I-15 | 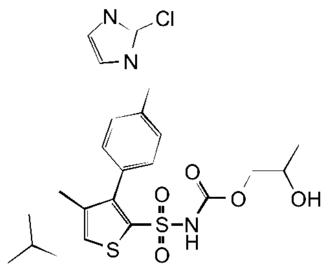 | 2-hydroxy-2-methylpropyl ((3-(4-((2-chloro-1H-imidazol-1-yl)methyl)phenyl)-4-methyl-5-isobutylthiophen-2-yl)sulfonyl)carbamate | 541.1 |

(continued)

| Number | Structural Formula | Name | LC-MS, M/Z (ESI) |
|---|---|---|---|
| I-16 | 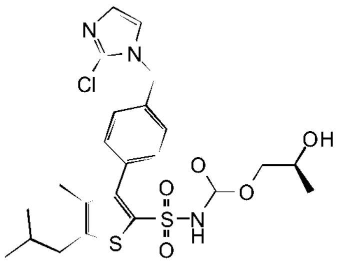 | (2S)-2-hydroxypropyl ((3-(4-((2-chloro-1H-imidazol-1-yl)methyl)phenyl)-4-methyl-5-isobutylthiophen-2-yl)sulfonyl)carbamate | 527.1 |
| I-17 | 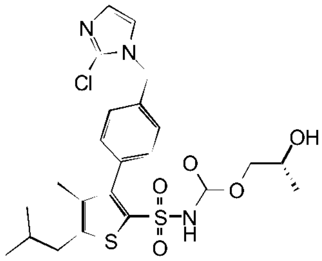 | (2R)-2-hydroxypropyl ((3-(4-((2-chloro-1H-imidazol-1-yl)methyl)phenyl)-4-methyl-5-isobutylthiophen-2-yl)sulfonyl)carbamate | 527.1 |
| I-20 | 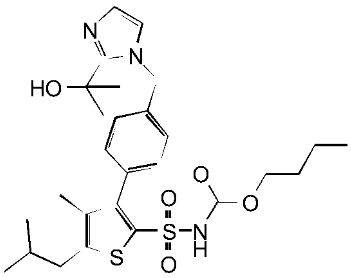 | Butyl ((3-(4-((2-(2-hydroxypropan-2-yl)-1H-imidazol-1-yl)methyl)phenyl)-4-methyl-5-isobutylthiophen-2-yl)sulfonyl)carbamate | 548.7 |
| I-25 | 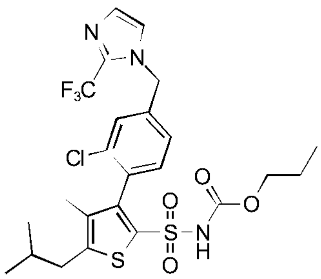 | Butyl (3-(2-chloro-4-((2-(trifluoromethyl)-1H-imidazol-1-yl)methyl)phenyl)-4-methyl-5-isobutylthiophen-2-sulfonyl)carbamate | 592.1 |
| I-26 | 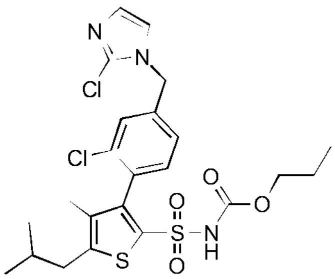 | Butyl (3-(2-chloro-4-((2-chloro-1H-imidazol-1-yl)methyl)phenyl)-4-methyl-5-isobutylthiophen-2-sulfonyl)carbamate | 558.1 |

(continued)

| Number | Structural Formula | Name | LC-MS, M/Z (ESI) |
|---|---|---|---|
| I-27 | 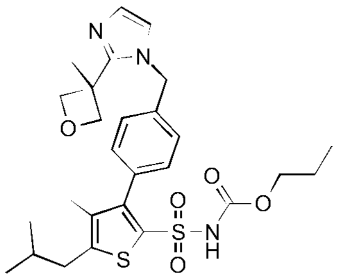 | Butyl (4-methyl-3-(4-((2-(3-methyloxetan-3-yl)-1H-imidazol-1-yl)methyl)phenyl)-5-isobutylthiophen-2-sulfonyl)carbamate | 560.2 |
| I-28 | 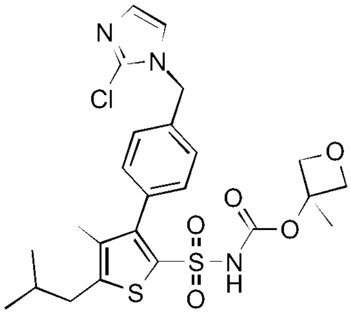 | (3-methyloxetan-3-yl) (3-(4-((2-chloro-1H-imidazol-1-yl)methyl)phenyl)-4-methyl-5-isobutylthiophen-2-sulfonyl)carbamate | 538.1 |
| I-29 | 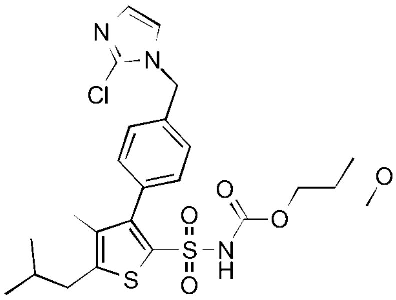 | (Oxetan-3-yl)methyl (3-(4-((2-chloro-1H-imidazol-1-yl)methyl)phenyl)-4-methyl-5-isobutylthiophen-2-sulfonyl)carbamate | 538.1 |

In the test examples of the present disclosure, Control Compound I was prepared by the method referring to Patent WO2021229244A1. The Control Compound I has a structure represented by:

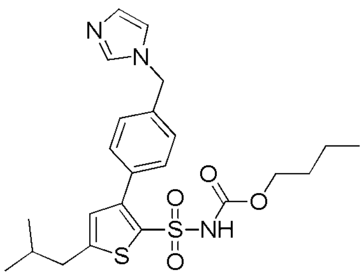

Control Compound I.

**Bioassays**

The following test methods can be adopted.

**Test Example 1:** Binding Assay of Compounds to AT2R

The assay was performed in accordance with the manufacturer instructions of Angiotensin AT2 Receptor Ligand Binding Assay Kit (#C1TT1AT2, Cisbio). First, a 10 mM compound stock solution was diluted in a gradient at a 5× dilution

ratio (including 10 concentrations, each with two replicates). 160 nL of compounds at different concentrations was added to a 384-well plate. 40 μL of 1× TLB (TLB: Tag-lite buffer) was added to each well, and the mixture was shaken at room temperature for 15 minutes. A 15 mL centrifuge tube added with 5 mL of 1×TLB was prepared in advance. The frozen labeled cells were thawed in a water bath at 37°C (for 1 to 2 minutes), and the thawed cells were quickly transferred to the above 15 mL centrifuge tube. The mixture was mixed well and centrifuged at 1,000 g at room temperature for 5 minutes. The supernatant was removed, and the cells were resuspended by adding 2.7 mL 1× TLB. A new 384-well plate was taken, and 10 μL of mixed cells were added into the corresponding wells according to the assay design. 5 μL of 4× compound solution and 5 μL of 4× Tag-lite red fluorescent labeled ligand were added to each well. After incubation at room temperature for 1 hour, data were read using EnVision with an HTRF mode. The excitation light intensities at 665nM and 615nM in each well were read respectively, and the ratio was calculated (Ratio=A665nM/B615nM). The $IC_{50}$ value was calculated by means of GraphPad Prism8 software, where X represents logarithmic value of compound concentration; and Y represents ratio of A665nM/B615nM.

[Table 1] Binding activity of compounds to AT2R

| Compound No. | AT2R $IC_{50}$ (nM) |
|---|---|
| Control Compound I | 4.46 |
| I-1 | 6.67 |
| I-2 | 6.56 |
| I-3 | 3.21 |
| I-4 | 3.46 |
| I-9 | 5.13 |
| I-10 | 2.31 |
| I-11 | 4.49 |
| I-12 | 3.88 |
| I-13 | 2.60 |
| I-14 | 1.39 |
| I-16 | 4.49 |
| I-17 | 3.88 |
| I-18 | 3.52 |
| I-19 | 2.91 |
| I-23 | 1.79 |
| I-24 | 4.41 |

The experimental results showed that the compounds of the present disclosure exhibited a strong binding affinity to AT2R.

**Test Example 2:** CYP Inhibition Assay of Compounds

The inhibitory effects of the compounds on six enzymes, i.e., CYP1A2, CYP2C9, CYP2C19, CYP2D6, CYP3A4 (midazolam), and CYP2B6, were evaluated at a concentration of 10 μM. Mixed human liver microsomes, which were previously stored in a -80°C refrigerator before use, were taken out and thawed in a water bath at 37°C before use, and placed on ice for further operation. 100 μL of microsome working solution was taken, and added with 2 μL of compound or positive inhibitor working solution. 2 μL of solvent was added to the solvent control. The mixture was pre-incubated in a water bath at 37°C for 10 minutes. After the preincubation, 98 μL of NADPH regeneration solution was added to all samples to initiate the reaction. Then, the samples were put back into the water bath and incubated for a period of time. The reaction was quenched with 200 μL of stop solution. The plate was centrifuged at 3,220 g for 10 minutes, 100 μL of supernatant was transferred, and 100 μL of water was added and mixed well to the assay plate for LC/MS/MS analysis.

[Table 2] CYP450 Inhibition Results of Compounds

| | Inhibition Rate % | | | | | |
|---|---|---|---|---|---|---|
| Compound No. | **CYP1A2** | **CYP2C9** | **CYP2C19** | **CYP2D6** | **CYP3A4 (midazolam)** | **CYP2B6** |
| Control Compound I | 95.38 | 100.00 | 100.00 | 86.46 | 92.64 | 63.19 |
| I-9 | 0.00 | 18.6 | 29.8 | 0.00 | 0.00 | 22.2 |
| I-10 | 0.00 | 5.3 | 7.4 | 0.00 | 0.00 | 13.7 |
| I-13 | 3.5 | 12.2 | 17.3 | 0.00 | 9.6 | 11.3 |
| I-14 | 5.6 | 24.8 | 11.9 | 2.1 | 14.0 | 4.3 |
| I-19 | 0.00 | 0.00 | 0.00 | 0.00 | 7.6 | 3.8 |
| I-23 | 8.3 | 20.5 | 15.4 | 0.00 | 0.00 | 7.6 |

The experimental results showed that compared with the control compound, the compounds of the present disclosure had a relatively weaker inhibitory effect on CYP enzymes, and they would not affect normal metabolism of the body, indicating a lower probability of drug-drug interaction in the human body and higher safety.

**Test Example 3:** Free Plasma Protein Binding (PPB) Assay of Compounds

The dried dialysis membrane was soaked in ultrapure water for 1 hour, then soaked in 20% ethanol for 20 minutes, and then rinsed with ultrapure water for 2 to 3 times. Finally, the dialysis membrane was soaked in ultrapure water for 20 minutes for later use. The frozen plasma was thawed in a water bath at 37°C for about 20 minutes. The pH value of the plasma was measured after the completion of thawing, and was adjusted to 7.4 with 1% phosphoric acid solution or 0.1M sodium hydroxide solution. The compound was diluted into plasma preheated to 37°C to a final concentration of 1 μM, and warfarin (internal standard) in plasma had a final concentration of 2 μM. The pretreated dialysis membrane was assembled into the dialysis plate according to product instructions, and 100 μL of receiving solution (100 mM phosphate buffer solution plus 0.002% Tween 80) was added to one side of the permeable membrane in each dialysis well. 20 μL of each of the final solution of the test compound and the final solution of the control compound were taken in a 96-well sample plate, two duplicate samples were made, and TO sample was obtained, which was stored in a -20°C refrigerator. Another 100 μL of each of the above-mentioned final solutions was taken to the other side of the membrane in the dialysis device, two duplicate samples were made, and incubated at 37°C with constant shaking for 6 hours. After 6 hours of incubation, 20 μL of each of the dialyzed receiving solution and the administered plasma were taken, two duplicate samples were made, and sample A and sample B were obtained. Corresponding blank plasma or receiving solution of corresponding volume was added to sample B and sample A, respectively, allowing the volume ratio of plasma to buffer in each sample well to be 1:1. 300 μL of sample-containing internal standard acetonitrile solution was added to all the sample wells. The mixture was mixed well, and centrifuged at 5,500×g for 10 minutes. 150 μL of corresponding ultrapure water was added to the corresponding sample wells of the 96-well sample plate. 150 μL of the supernatant was taken into the sample wells, and mixed well. Then, LC/MS/MS analysis was performed. The calculation formula of $f_u$ is:

$$f_u = \frac{C_R}{C_D} \times 100\%,$$

where:

$C_R$: peak area ratio measured in the receiving chamber well
$C_D$: peak area ratio measured in the supply chamber well.

The degree of freeness of a compound in plasma is related to its efficacy in the body. The experimental results showed that compared with the control compound, the compounds in the present disclosure had a higher degree of freeness, which was beneficial for the compounds to reach target organs.

**Test Example 4:** Liver Microsomal Stability Assay of Compounds

The human liver microsome stability assay was performed by co-incubating the compound with human liver

microsomes *in vitro*. The test compounds were first prepared as 10 mM stock solutions in DMSO solvent, and then diluted to 0.5 mM with acetonitrile. Human liver microsomes (Corning) were diluted with PBS to prepare a microsome/buffer solution, and this solution was used to dilute 0.5 mM of the compound to prepare a working solution. In the working solution, a concentration of the compound was 1.5 μM and a concentration of the human liver microsome was 0.75 mg/mL. A deep-well plate was taken, 30 μL of working solution was added to each well of the plate, and then 15 μL of pre-heated NADPH solution (6 mM) was added to start the reaction. The mixture was incubated at 37°C. 135 μL of acetonitrile was added to the corresponding wells to stop the reaction at 0 minute, 5 minutes, 15 minutes, 30 minutes, and 45 minutes of incubation. Subsequent to the stop of the reaction by means of acetonitrile at the last time point of 45 minutes, the plate was vortexed for 10 minutes (600 rpm/min) and then centrifuged for 15 minutes. After the centrifugation, the supernatant was taken, and purified water was added in a 1:1 ratio for LC-MS/MS detection. The ratio of a peak area of the compound to a peak area of the internal standard at individual time points was obtained. The peak area ratios of the compound at 5 minutes, 15 minutes, 30 minutes, and 45 minutes were compared with the peak area ratio at 0 minute, and the remaining percentage of the compound at each time point was calculated. $T_{1/2}$ was calculated using Graphpad8 software.

[Table 3] Liver Microsomal Stability of Compounds

| | $T_{1/2}$ (min) |
|---|---|
| Compound No. | Human |
| Control Compound I | 9.13 |
| I-9 | 50.2 |
| I-10 | 83.2 |
| I-13 | 46.4 |
| I-14 | 45.0 |
| I-19 | 71.2 |
| I-23 | 39.3 |

The stability of a compound in liver microsomes reflects its risk of being metabolized and eliminated *in vivo*. The experimental results showed that the test compounds had better stability in humans compared to the control compound.

**Test Example 5:** Bidirectional Permeability Study of Compounds in CACO-2 Cells

Caco-2 cells were inoculated onto polyethylene membrane (PET) in 96-well inserted plates at a rate of $1\times10^5$ cells/$cm^2$, and the medium was changed every 4 to 5 days until day 21 to 28, to form a fused cell monolayer. The transport buffer in the assay was HBSS and 10.0 mM HEPES, with a pH of 7.40±0.05. The test compounds were subjected to a bidirectional test at 2.00 μM in the presence and absence of GF120918 (10.0 μM). Digoxin was subjected to a bidirectional test at 10.0 μM in the presence and absence of GF120918 (10.0 μM), while Nadolol and Metoprolol were subjected to a test from direction A to direction B at 2.00 μM in the absence of GF120918 (10.0 μM). The above tests were performed in duplicate. The final concentration of DMSO was adjusted to be lower than 1%. The plate was incubated in a $CO_2$ incubator at 37°C±1°C for 2 hours, and 5% $CO_2$ was added under saturated humidity without shaking. All the samples, after being mixed with acetonitrile containing internal standard, were centrifuged at 3,200 g for 10 minutes. For Nadolol and Metoprolol, 200 μL of supernatant was diluted with 600 μL of distilled water for LC-MS/MS analysis. For Digoxin and test compounds, 200 μL of supernatant was diluted with 200 μL of distilled water for LC-MS/MS analysis. The concentrations of the test compounds and control compounds in the starting solution, the donor solution, and the acceptor solution were quantified by LC-MS/MS based on a peak area ratio of the analyte to the internal standard.

After the transport assay, the integrity of the Caco-2 cell monolayer was determined using Lucifer Yellow Exclusion Assay.

[Table 4] Bidirectional Permeability of Compounds in Caco-2 Cells

| Compound No. | $P_{app}$ mean value ($10^{-6}$ cm/s) |
|---|---|
| | A to B |
| Control Compound I | 0.381 |
| I-9 | 2.29 |

(continued)

| Compound No. | $P_{app}$ mean value ($10^{-6}$ cm/s) |
|---|---|
| | A to B |
| I-10 | 2.63 |
| I-13 | 1.98 |
| I-14 | 3.73 |
| I-19 | 3.24 |
| I-23 | 5.22 |

The experimental results showed that compared with the control compound, the permeability of the test compounds was improved, which was beneficial to absorption of the compounds.

**Test Example 6:** Induction of CYP 3A4 Enzyme by Compounds

Cell viability of thawed hepatocytes was assessed. The hepatocytes were diluted to $0.7\times10^6$ cells/μL in seeding medium. 100 μL of preheated seeding medium was added to each well of a 48-well collagen-coated plate for pre-wetting, and then the medium was discarded. 200 μL of cell suspension was added to each well of the 48-well pre-collagen-coated plate, and then incubated in a 37°C incubator with 95% humidity and 5% $CO_2$. Four hours after cell attachment, the seeding medium was aspirated, and 200 μL of incubation medium containing 2% matrix metalloproteinase was added to each well. On the next day, the cell plate was removed from the incubator, and the supernatant was aspirated. 200 μL of compound solution was added to each well, and the plate was placed in the incubator for further incubation. At 24 hours and 48 hours post-treatment, the 48-well plate was taken out from the incubator, and 100 μL of culture medium from each well was transferred to a clean 96-well cell culture plate for LDH assay to determine the cell viability.

Enzyme Activity Assay:

At the 48-hour time point, the supernatant was aspirated, and the cells were washed twice with 400 μL/well of preheated HBSS solution. After aspirating the HBSS, 100 μL of enzyme-labeled substrate working solution was added to each well. Then, the cell plate was incubated in an incubator for 30 minutes, and the reaction was stopped by transferring the supernatant to a 96-well plate containing a stop solution. The plate was sealed, vortexed, and centrifuged to collect the supernatant. The supernatant was then diluted in 0.1% FA water at a ratio of 1:4. All samples were mixed and analyzed by liquid chromatography/tandem mass spectrometry (LC/MS/MS). Calibration was performed using a peak area ratio of metabolites to internal standards (IS). The activity of CYP enzymes was calculated using a standard curve consisting of 7 standard concentrations, and the fold change relative to the vehicle control (0.1% dimethyl sulfoxide) was calculated. Induction fold=enzyme activity of compound-treated sample/enzyme activity of vehicle control.

Gene Induction Assay of Enzymes:

1. Extraction of Total Cellular RNA

After 48 hours, the supernatant was aspirated, and the cells were rinsed once with 400 μL/well HBSS. RLT buffer (QIAGEN, cat: 1015762) was added to the plate, and the extraction was performed in accordance with the protocol of the QIAGEN RNA extraction kit.

2. Synthesis of cDNA

The synthesis of cDNA was carried out using the High-Capacity cDNA Reverse Transcription Kit (ABI, cat: 4368813).

3. Quantitative Real-time PCR Reaction

Nuclease-free water was added to a new plate, and cDNA samples were added to the plate. The mixture was gently mixed for dilution. Reaction mixtures were prepared for 18S rRNA and CYP3A4 respectively, with 18S rRNA serving as an internal standard. The quantitative real-time PCR plate was sealed, and centrifuged briefly to remove air bubbles and

deposit the liquid at the bottom of the tube. The plate was transferred to a quantitative real-time PCR instrument and analyzed according to the manufacturer's protocol. Conditions for PCR were as follows: 50°C for 2 minutes, 95°C for 10 minutes, and 40 cycles of 95°C for 15 seconds and 60°C for 1 minute.

4. Quantitative Real-time PCR Data Analysis

Fold change in relative expression=2-ΔΔCt, where ΔCt=Ct (target gene)-Ct (18S rRNA), and ΔΔCt=ΔCt (treated sample)-ΔCt (untreated control).

The experimental results showed that the test compounds exhibited no toxic effect on the hepatocytes, weaker induction of CYP3A4 enzyme activity, and relatively weaker induction of CYP3A4 gene expression compared to the positive control.

**Test Example 7:** Pharmacokinetic Study of Compounds Following Single Oral Gavage Administration to Cynomolgus Monkeys

The compounds were dissolved in a vehicle consisting of 5% DMSO+10% Solutol+85% Saline to a concentration of 1 mg/mL, and administered at a dose of 5 mpk. The animals were weighed before the administration, and the dosing volume was calculated based on the body weight. The compounds were administered to the animals via oral gavage. Blood samples were collected via a femoral vein or other suitable methods at time points of 0.083 hours, 0.25 hours, 0.5 hours, 1 hour, 2 hours, 4 hours, 6 hours, 8 hours, and 24 hours. Approximately 1 mL of each sample was collected at each time point, anticoagulated with sodium heparin, and placed on ice immediately after the collection. After the blood samples were collected, they were placed on ice and centrifuged within 1 hour to separate plasma (centrifugation conditions: 2,200g, 10 minutes, and 2°C to 8°C). The plasma samples were stored in a -80°C refrigerator until analysis.

The bioanalytical method and sample analysis were performed by the Analytical Laboratory of Medicilon Inc. (Shanghai, China). Intra-day accuracy of quality control samples was evaluated concurrently with the sample analysis, requiring that accuracy of more than 66.7% of the quality control samples fell within the range of 80% to 120%.

[Table 5] Pharmacokinetics of Compounds in Monkeys

| | Monkey PK PO (5 mpk) | |
|---|---|---|
| Compound No. | Cmax (ng/mL) | $AUC_{0-t}$ (h*ng/mL) |
| Control Compound I | 1316.7 | 4922.7 |
| I-9 | 5309.2 | 7794.1 |
| I-10 | 3563.4 | 5573.1 |
| I-14 | 5035.8 | 7348.3 |
| I-19 | 4026.7 | 6815.6 |

The experimental results showed that the test compounds exhibited higher Cmax and exposure levels than the control compound, indicating that a favorable agonistic effect.

**Test Example 8:** *In Vivo* Efficacy Study of Compounds in a Mouse Model of Idiopathic Pulmonary Fibrosis (IPF)

A specific mouse IPF model was required for the assay. Shanghai Pengli Biotechnology Co., Ltd. was responsible for model establishment and efficacy study of the compounds. All animal experimental protocols were approved by Pengli's Institutional Animal Care and Use Committee (IACUC).

The experimental steps were as follows.

1. Compound Preparation: An appropriate amount of compound powder was weighed and dissolved to prepare a DMSO stock solution. The stock solution was aliquoted into daily required doses and freshly prepared on the day of use. The required volume of 10% Solutol was added according to the dilution ratio, followed by vortexing to dissolve and mix thoroughly. Finally, the required volume of 85% saline was added according to the dilution ratio to achieve a final DMSO concentration of 5%, thereby preparing a clear solution with a concentration of 0.03 mg/mL.

2. Animal Grouping: On the day of modeling, 10 animals were randomly selected and directly assigned in Group G1 (blank control group), while all other animals received intratracheal injection of bleomycin for model construction. After the bleomycin injection, the animals were randomly assigned to groups using BioBook before the first administration, based on an animal body weight change, a body weight, and an animal condition, in order to achieve an approximate average

weight in each group and minimize inter-group variation.

3. Model Construction: An appropriate amount of bleomycin was dissolved in commercially available saline. Animals in the model group were anesthetized by inhaling 1% to 4% isoflurane and received 2 U/kg of bleomycin via intratracheal administration. The specific dosage was calculated based on individual body weights. Mice in G1 group were anesthetized with 1% to 4% isoflurane and then received an equal volume of saline via intratracheal injection.

4. Drug Administration: The day of bleomycin injection was designated as Day 0 of the experiment. The dosing regimen is shown in the table below:

[Table 6-1] Grouping and Dosing Regimen

| **Group** | **Test Article** | **Number of animal** | **Route of administration** | **Concentration mg/mL** | **Dosing Volume** | | **Dosing regimen** [b] |
|---|---|---|---|---|---|---|---|
| | | | | | **mL/kg** | **mg/kg** | |
| 1 | Normal-Vehicle Group (5% DMSO+10% Solutol+85% Saline)[a] | 10 | p.o. | NA | 10 | NA | From Day 7, bid, Vehicle for 15 consecutive days |
| 2 | Model-Vehicle Group (5% DMSO+10% Solutol+85% Saline)[a] | 10 | p.o. | NA | 10 | NA | From Day 7, bid, Vehicle for 15 consecutive days |
| 3 | Model-Control Compound I | 10 | p.o. | 0.03 | 10 | 0.3 | From Day 7, bid, Vehicle for 15 consecutive days |
| 4 | Model-Test Compound | 10 | P.o. | 0.03 | 10 | 0.3 | From Day 7, bid, Vehicle for 15 consecutive days |

*a: The vehicle was 5% DMSO+10% solutol+85% saline;*
*b: bid, once in the morning and once in the afternoon, about a 6-hour interval;* ***no administration on the morning of Day 22***.

5. Pulmonary Function Test: On Day 22, all experimental animals were anesthetized with zoletil (25 mg/kg to 50 mg/kg) and xylazine (5 mg/kg to 10 mg/kg). Pulmonary function was assessed using a pulmonary function test (PFT) instrument, including the following indicators: Pressure-Volume (P-V) curve, Forced Vital Capacity (FVC), Inspiratory Capacity (IC), Vital Capacity (VC), Dynamic Compliance (Cdyn), and Chord Compliance (Cchord, quasi-static lung compliance).

6. Pathological Examination of Lung Tissue: Lung tissues were collected, rinsed twice, blotted dry with filter paper, and weighed. After the weighing was completed, the left lung was perfused and fixed with 10% neutral formalin solution, then immersed in 10% neutral formalin for fixation and storage for histopathological scoring. The formalin-fixed left lung was transversely sectioned into upper, middle, and lower segments, which were embedded in the same paraffin block. Sections were stained with Masson's trichrome and evaluated by a pathologist. The scoring criteria are shown in the table below:

[Table 6-2] Fibrosis Scoring Criteria

| Score | Criteria |
|---|---|
| 0 | Normal |
| 1 | Tiny fibrous thickening of the alveolar or bronchiolar walls |
| 2 | Moderate thickening of lung walls without significant damage to the lung structure |
| 3 | Aggravated fibrosis with definite lung structure damage, forming fibrous bands or small fibrotic nodules |
| 4 | Severe structural distortion and extensive fibrosis, presenting a "honeycombing" appearance |
| 5 | Total fibrosis visual field occlusion |

7. Animal Euthanasia: All experimental animals were euthanized by $CO_2$ inhalation followed by cervical dislocation after the completion of the in vivo study.

Statistical Analysis: Experimental data were expressed as mean±standard error (mean±S.E.M), and SPSS or Graphpad Prism software were used to analyze the data. $P<0.05$ was considered statistically significant.

The experimental results showed that compared with the control compound, the compounds of the present disclosure can significantly improve the pulmonary function indicators including FVC, IC, and VC, and can significantly optimize the pulmonary fibrosis scores.

Although embodiments of the present disclosure have been shown and described, it would be appreciated that the above embodiments are exemplary and cannot be construed to limiting the present disclosure, and changes, modifications, alternatives, and alterations can be made in the embodiments by those skilled in the art without departing from scope of the present disclosure.

## Claims

**1.** A compound, or a tautomer, stereoisomer, hydrate, solvate, pharmaceutically acceptable salt, or prodrug thereof, wherein the compound has a structure represented by Formula III:

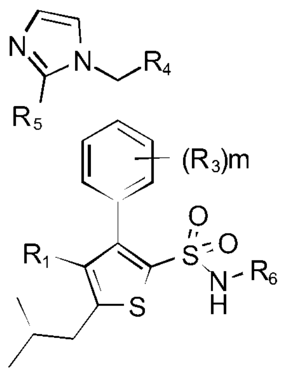

III,

where:

$R_1$ is selected from halogen, $C_1$ to $C_6$ alkyl, and halogenated $C_1$ to $C_6$ alkyl;
each $R_3$ is independently selected from hydrogen, halogen, cyano, $C_1$ to $C_6$ alkyl, and $C_3$ to $C_6$ cycloalkyl, wherein the $C_1$ to $C_6$ alkyl and the $C_3$ to $C_6$ cycloalkyl are optionally substituted with one, two, three, or four substituents selected from halogen, hydroxyl, amino, cyano, $C_1$ to $C_6$ alkyl, and $C_3$ to $C_7$ cycloalkyl, and wherein when a plurality of substituents are present, the plurality of substituents are the same or different;
m is selected from 1, 2, 3, and 4;
$R_4$ is selected from hydrogen, $C_1$ to $C_6$ alkyl, and halogenated $C_1$ to $C_6$ alkyl;
$R_5$ is selected from halogen, $C_1$ to $C_6$ alkyl, and 4- to 7-membered heterocycloalkyl, wherein the $C_1$ to $C_6$ alkyl is substituted with one, two, three, or four substituents selected from halogen, hydroxyl, and 4- to 7-membered heterocycloalkyl, wherein the 4- to 7-membered heterocycloalkyl is substituted with zero, one, two, three, or four substituents selected from halogen, hydroxyl, cyano, $C_1$ to $C_6$ alkyl, and 4- to 7-membered heterocycloalkyl, and wherein when a plurality of substituents are present, the plurality of substituents are the same or different;
$R_6$ is selected from a 5- to 7-membered heteroaromatic ring and -C(O)-X-$R_2$;
X is selected from NRa and O;
$R_2$ is $C_1$ to $C_6$ alkyl, 4- to 7-membered heterocycloalkyl, and a 5- to 7-membered heteroaromatic ring, wherein the $C_1$ to $C_6$ alkyl is substituted with zero, one, two, three, or four substituents selected from halogen, hydroxyl, $C_1$ to $C_3$ alkyl, 3- to 7-membered heterocycloalkyl, and a 5- to 7-membered heteroaromatic ring, wherein the 4- to 7-membered heterocycloalkyl and the 5- to 7-membered heteroaromatic ring are substituted with zero, one, two, three, or four substituents selected from halogen, hydroxyl, cyano, amino, $C_1$ to $C_6$ alkyl, halogenated $C_1$ to $C_6$ alkyl, $C_1$ to $C_6$ alkoxy, halogenated $C_1$ to $C_6$ alkoxy, $C_3$ to $C_7$ cycloalkyl, a 5- to 7-membered heteroaromatic ring, and 3- to 7-membered heterocycloalkyl, and wherein when a plurality of substituents are present, the plurality of substituents are the same or different;
Ra is selected from hydrogen and $C_1$ to $C_3$ alkyl, or Ra and $R_2$ together with nitrogen atoms bonded thereto form a 5- to 8-membered heterocycloalkyl bicyclic system;
when $R_5$ is selected from halogen and the halogen is Cl, when $R_3$ is selected from hydrogen, when $R_1$ is selected from $C_1$ to $C_6$ alkyl and the $C_1$ to $C_6$ alkyl is methyl, and when $R_6$ is selected from -C(O)-O-$R_2$, $R_2$ is not methyl; and

when $R_6$ is selected from the 5- to 7-membered heteroaromatic ring, $R_3$ is hydrogen or F.

**2.** The compound, or the tautomer, stereoisomer, hydrate, solvate, pharmaceutically acceptable salt, or prodrug thereof according to claim 1, wherein the compound has a compound represented by Formula I:

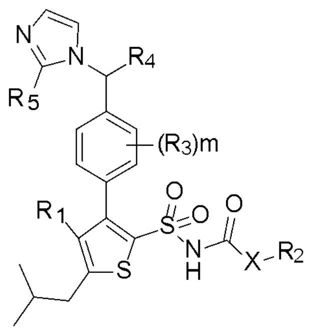

I,

where:

$R_1$ is selected from halogen, $C_1$ to $C_6$ alkyl, and halogenated $C_1$ to $C_6$ alkyl;
X is selected from NRa and O;
$R_2$ is $C_1$ to $C_6$ alkyl or a 5- to 7-membered heteroaromatic ring, wherein the $C_1$ to $C_6$ alkyl is substituted with zero, one, two, three, or four substituents selected from halogen, hydroxyl, $C_1$ to $C_3$ alkyl, and a 5- to 7-membered heteroaromatic ring, and wherein when a plurality of substituents are present, the plurality of substituents are the same or different;
each $R_3$ is independently selected from hydrogen, halogen, cyano, $C_1$ to $C_6$ alkyl, and $C_3$ to $C_6$ cycloalkyl, wherein the $C_1$ to $C_6$ alkyl and the $C_3$ to $C_6$ cycloalkyl are optionally substituted with one, two, three, or four substituents selected from halogen, hydroxyl, amino, cyano, $C_1$ to $C_6$ alkyl, and $C_3$ to $C_7$ cycloalkyl, and wherein when a plurality of substituents are present, the plurality of substituents are the same or different;
m is selected from 1, 2, 3, and 4;
$R_4$ is selected from hydrogen, $C_1$ to $C_6$ alkyl, and halogenated $C_1$ to $C_6$ alkyl;
$R_5$ is selected from halogen and $C_1$ to $C_6$ alkyl, wherein the $C_1$ to $C_6$ alkyl is substituted with one, two, three, or four substituents selected from halogen and hydroxyl, and wherein when a plurality of substituents are present, the plurality of substituents are the same or different;
Ra is selected from hydrogen and $C_1$ to $C_3$ alkyl, or Ra and $R_2$ together with nitrogen atoms bonded thereto form a 5- to 8-membered heterocycloalkyl bicyclic system;
when $R_5$ is selected from halogen and the halogen is Cl, when $R_3$ is selected from hydrogen, when $R_1$ is selected from $C_1$ to $C_6$ alkyl and the $C_1$ to $C_6$ alkyl is methyl, and when X is selected from O, $R_2$ is not methyl.

**3.** The compound, or the tautomer, stereoisomer, hydrate, solvate, pharmaceutically acceptable salt, or prodrug thereof according to claim 1, wherein:

$R_6$ is selected from the 5- to 7-membered heteroaromatic ring, a heteroatom in the 5- to 7-membered heteroaromatic ring being N; and/or
$R_6$ is selected from

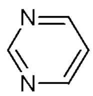

;

and/or
the compound has a structure represented by Formula IV:

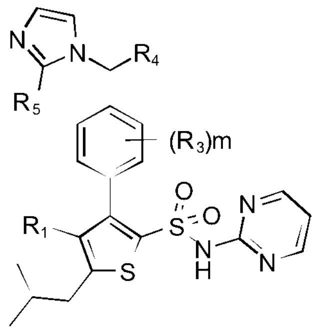

IV.

**4.** The compound, or the tautomer, stereoisomer, hydrate, solvate, pharmaceutically acceptable salt, or prodrug thereof according to any one of claims 1 to 3, wherein:

$R_1$ is selected from halogen, $C_1$ to $C_3$ alkyl, and halogenated $C_1$ to $C_3$ alkyl;
preferably, $R_1$ is selected from F, Cl, Br, I, methyl, ethyl, propyl, isopropyl, $CH_2F$, $CHF_2$, and $CF_3$;
more preferably, $R_1$ is selected from F, Cl, and methyl; and
more preferably, $R_1$ is selected from methyl.

**5.** The compound, or the tautomer, stereoisomer, hydrate, solvate, pharmaceutically acceptable salt, or prodrug thereof according to any one of claims 1 to 2, wherein:

X is selected from NRa and O, and Ra is selected from hydrogen and methyl; and/or
X is selected from O; and/or
X is selected from NRa, and Ra is selected from hydrogen and methyl; and/or
X is selected from NRa, and Ra and $R_2$ together with nitrogen atoms bonded thereto form a 5- to 8-membered heterocycloalkyl bicyclic system, the 5- to 8-membered heterocycloalkyl bicyclic system containing one, two, or three heteroatoms selected from N, O, and S; and/or
X is selected from NRa, and Ra and $R_2$ together with nitrogen atoms bonded thereto form a 5- to 8-membered heterocyclic spiro ring, the 5- to 8-membered heterocyclic spiro ring containing one, two, or three heteroatoms selected from N, O, and S; and/or
X is selected from NRa, and Ra and $R_2$ together with nitrogen atoms bonded thereto form

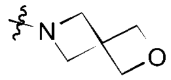

.

**6.** The compound, or the tautomer, stereoisomer, hydrate, solvate, pharmaceutically acceptable salt, or prodrug thereof according to any one of claims 1 to 2, wherein:

$R_2$ is selected from $C_1$ to $C_4$ alkyl, 4- to 7-membered heterocycloalkyl, and a 5- to 7-membered heteroaromatic ring, wherein the $C_1$ to $C_4$ alkyl is substituted with zero, one, two, three, or four substituents selected from halogen, hydroxyl, $C_1$ to $C_3$ alkyl, 3- to 7-membered heterocycloalkyl, a 5- to 7-membered heteroaromatic ring, and 4- to 7-membered heterocycloalkyl, wherein the 4- to 7-membered heterocycloalkyl and the 5- to 7-membered heteroaromatic ring are substituted with zero, one, two, three, or four substituents selected from halogen, hydroxyl, $C_1$ to $C_3$ alkyl, and a 5- to 7-membered heteroaromatic ring, and wherein when a plurality of substituents are present, the plurality of substituents are the same or different;
preferably, $R_2$ is selected from $C_1$ to $C_4$ alkyl, 4- to 7-membered heterocycloalkyl, -$C_1$ to $C_4$ alkyl-4- to 7-membered heterocycloalkyl, -$C_1$ to $C_4$ alkyl-5- to 7-membered heteroaromatic ring, and -4- to 7-membered heterocycloalkyl-$C_1$ to $C_4$ alkyl, wherein the $C_1$ to $C_4$ alkyl is substituted with zero, one, two, three, or four substituents selected from halogen, hydroxyl, and $C_1$ to $C_3$ alkyl;
preferably, $R_2$ is selected from methyl, ethyl, propyl, isopropyl, butyl, isobutyl, pyrimidinyl, pyridinyl, pyridazinyl, and pyrazinyl, and $R_2$ is substituted with zero, one, two, three, or four substituents selected from halogen, hydroxyl, $C_1$ to $C_3$ alkyl, pyridinyl, pyrimidinyl, pyridazinyl, and pyrazinyl;
preferably, $R_2$ is selected from methyl, ethyl, propyl, isopropyl, butyl, isobutyl, azetidinyl, oxiranyl, oxetanyl, tetrahydrofuran, -$C_1$ to $C_4$ alkyl-azetidinyl, -$C_1$ to $C_4$ alkyl-oxiranyl, -$C_1$ to $C_4$ alkyl-oxetanyl, -oxetanyl-$C_1$ to $C_4$

alkyl, -$C_1$ to $C_4$ alkyl- tetrahydrofuran, -$C_1$ to $C_4$ alkylpyridinyl, pyrimidinyl, pyridinyl, pyridazinyl, and pyrazinyl, wherein the methyl, ethyl, propyl, isopropyl, butyl, and isobutyl are substituted with zero, one, two, three, or four substituents selected from halogen, hydroxyl, and $C_1$ to $C_3$ alkyl;
preferably, $R_2$ is selected from methyl, ethyl, propyl, butyl, isobutyl, pyridinyl, pyrimidinyl, -methyl-pyridinyl, -methyl-oxetanyl, and -oxetanyl-methyl, wherein the ethyl, propyl, isopropyl, butyl, and isobutyl are substituted with hydroxyl; and
more preferably, $R_2$ is selected from methyl, ethyl,

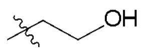 , 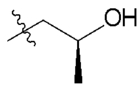 , 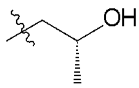 ,

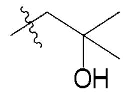 ,

butyl, pyrimidinyl,

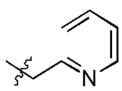 , 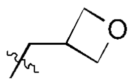 , and 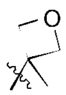 .

**7.** The compound, or the tautomer, stereoisomer, hydrate, solvate, pharmaceutically acceptable salt, or prodrug thereof according to any one of claims 1 to 3, satisfying one or more of the following conditions:

a) $R_3$ is selected from hydrogen, halogen, cyano, $C_1$ to $C_3$ alkyl, and $C_3$ to $C_4$ cycloalkyl, wherein the $C_1$ to $C_3$ alkyl and the $C_3$ to $C_4$ cycloalkyl are optionally substituted with one, two, three, or four substituents selected from halogen, hydroxyl, amino, cyano, $C_1$ to $C_6$ alkyl, and $C_3$ to $C_7$ cycloalkyl, and wherein when a plurality of substituents are present, the plurality of substituents are the same or different, and m is selected from 1, 2, 3, and 4;

preferably, $R_3$ is selected from hydrogen, F, Cl, methyl, and cyano, and m is selected from 1, 2, 3, and 4; and
more preferably, $R_3$ is selected from hydrogen, F, and Cl;

b) $R_4$ is selected from hydrogen and $C_1$ to $C_3$ alkyl;

preferably, $R_4$ is selected from hydrogen and methyl; and
more preferably, $R_4$ is selected from hydrogen; or

c) $R_5$ is selected from halogen, $C_1$ to $C_3$ alkyl, and 4- to 5-membered heterocycloalkyl, wherein the $C_1$ to $C_3$ alkyl is substituted with one, two, three, or four substituents selected from halogen, hydroxyl, and 4- to 7-membered heterocycloalkyl, wherein the 4- to 5-membered heterocycloalkyl is substituted with zero, one, two, three, or four substituents selected from halogen, hydroxyl, cyano, and $C_1$ to $C_6$ alkyl, and wherein when a plurality of substituents are present, the plurality of substituents are the same or different;

preferably, $R_5$ is selected from F, Cl, $CF_3$,

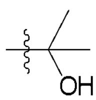 , and 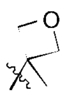 ; and

more preferably, $R_5$ is selected from Cl, $CF_3$,

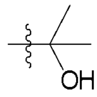 , and 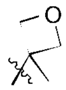 .

**8.** The compound, or the tautomer, stereoisomer, hydrate, solvate, pharmaceutically acceptable salt, or prodrug thereof according to claim 1, satisfying one or more of the following conditions:

1) when $R_5$ is selected from Cl, when $R_3$ is selected from hydrogen, when $R_1$ is selected from methyl, and when X is selected from O, $R_2$ is selected from methyl and $C_2$ to $C_6$ alkyl, wherein when $R_2$ is the methyl, the methyl is substituted with one, two, three, or four substituents selected from halogen, hydroxyl, and $C_1$ to $C_3$ alkyl, wherein when $R_2$ is the $C_2$ to $C_6$ alkyl, the $C_2$ to $C_6$ alkyl is substituted with zero, one, two, three, or four substituents selected from halogen, hydroxyl, and $C_1$ to $C_3$ alkyl, and wherein when a plurality of substituents are present, the plurality of substituents are the same or different; and
preferably, $R_2$ is selected from ethyl,

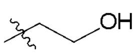 , 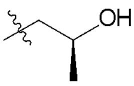 , 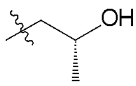 , 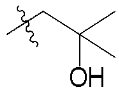 ,

and butyl;
2) when $R_5$ is selected from Cl, when $R_3$ is selected from hydrogen, when $R_4$ is selected from hydrogen, when $R_1$ is selected from methyl, and when X is selected from O, $R_2$ is selected from ethyl, butyl,

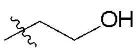 , 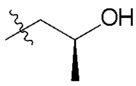 , 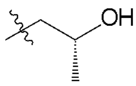 , 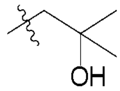 , 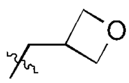 , and

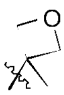 ;

3) when $R_3$ is selected from hydrogen, when $R_4$ is selected from hydrogen, when $R_1$ is selected from methyl, and when X is selected from NH, $R_2$ is selected from

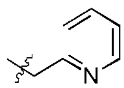 ;

4) when $R_1$ is selected from methyl, when $R_3$ is selected from hydrogen and F, and when $R_4$ is selected from hydrogen, $R_6$ is selected from -C(O)-O-$R_2$, $R_2$ is selected from methyl, butyl,

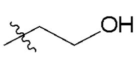 , 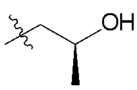 , 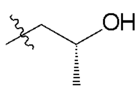 , 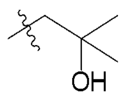 , 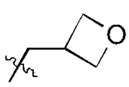 , and 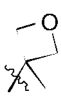 ,

and $R_5$ is selected from -$CF_3$, Cl,

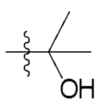 , and 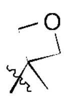 ;

5) when $R_1$ is selected from methyl, when $R_3$ is selected from Cl, and when $R_4$ is selected from hydrogen, $R_6$ is

selected from -C(O)-O-$R_2$, $R_2$ is selected from butyl, and $R_5$ is selected from -$CF_3$ and Cl;

6) when $R_1$ is selected from methyl, when $R_3$ is selected from hydrogen, and when $R_4$ is selected from hydrogen, $R_6$ is selected from a 5- to 7-membered heteroaromatic ring, a heteroatom in the 5- to 7-membered heteroaromatic ring being N;

preferably, $R_5$ is selected from -$CF_3$ and Cl; and
more preferably, $R_5$ is selected from Cl;

7) $R_1$ is selected from methyl, $R_3$ is selected from hydrogen, $R_4$ is selected from hydrogen, X is selected from NRa, and Ra and $R_2$ together with nitrogen atoms bonded thereto form

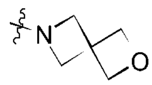
;

or

8) when $R_1$ is selected from methyl, when $R_2$ is selected from methyl, when $R_4$ is selected from hydrogen, and when X is selected from O, $R_3$ is selected from F and Cl, or $R_5$ is selected from -$CF_3$ and

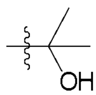
.

**9.** The compound, or the tautomer, stereoisomer, hydrate, solvate, pharmaceutically acceptable salt, or prodrug thereof according to claim 1, wherein the compound comprises:

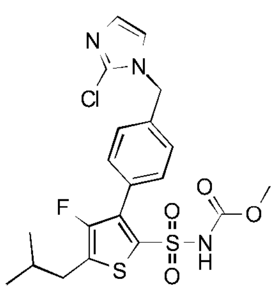
I-1,

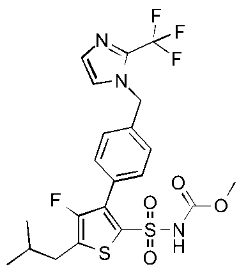
I-2,

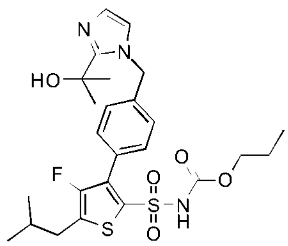
I-3,

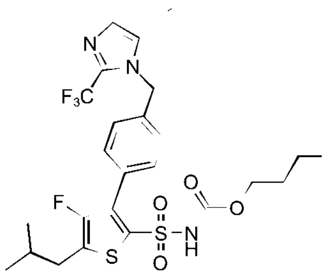
I-4,

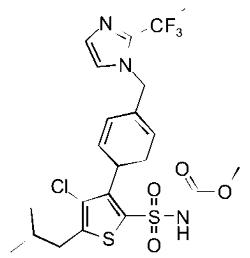
I-5,

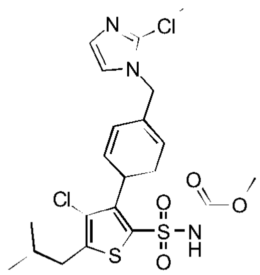
I-6,

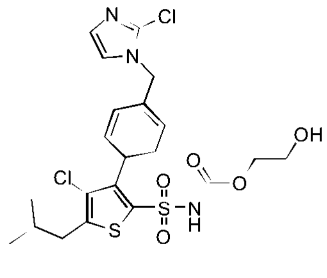
I-7,

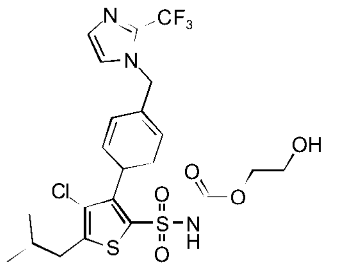
I-8,

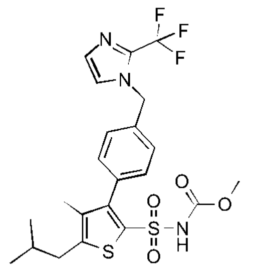
I-9,

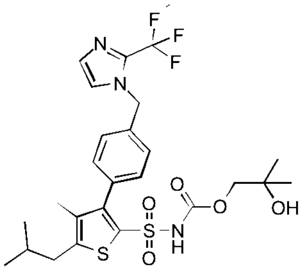
I-10,

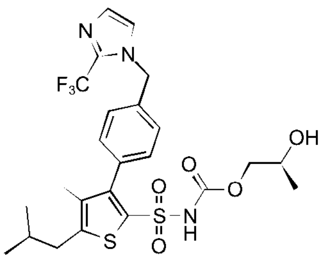
I-11,

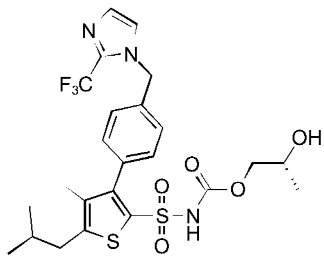
I-12,

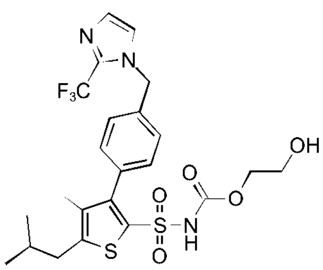
I-13,

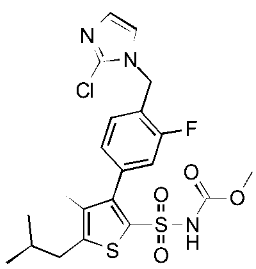
I-14,

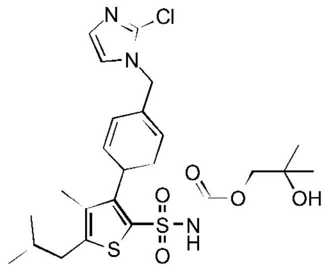
I-15,

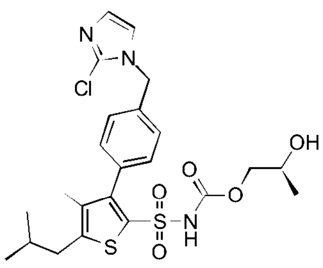
I-16,

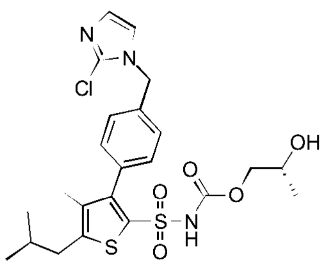
I-17,

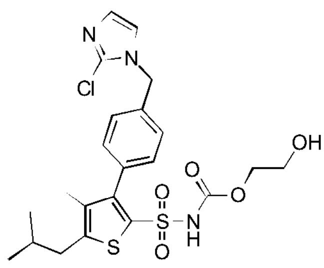
I-18,

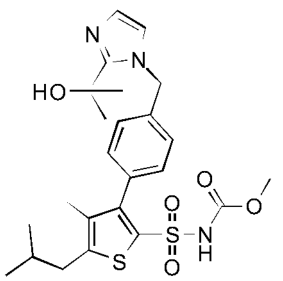
I-19,

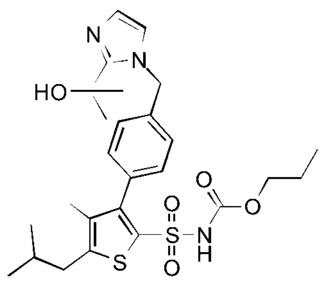
I-20,

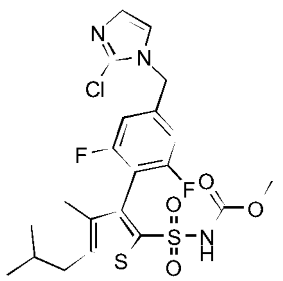
I-21,

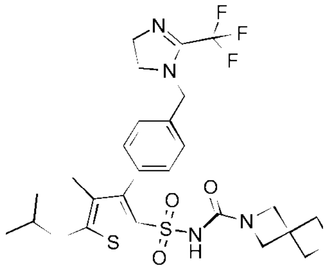
I-22,

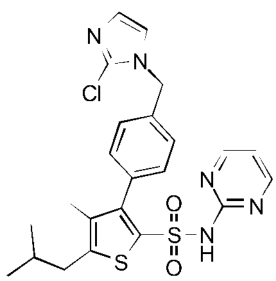
I-23,

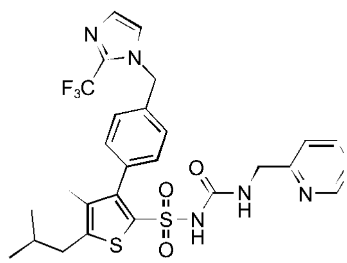
I-24,

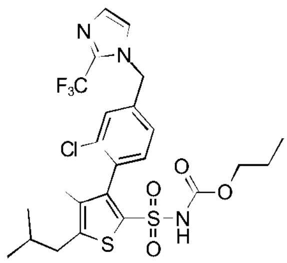
I-25,

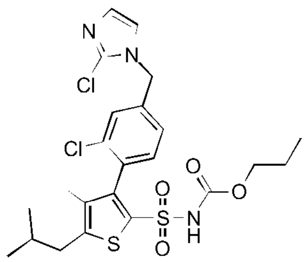
I-26,

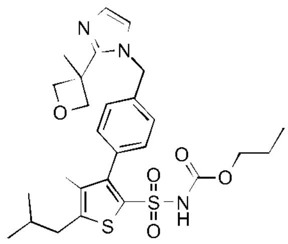
I-27,

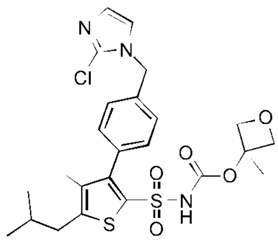
I-28, or

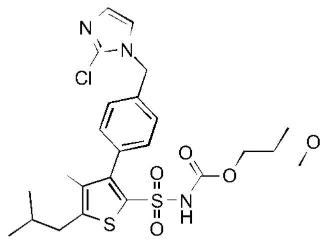
I-29.

**10.** A pharmaceutical composition, comprising:

the compound, or the tautomer, stereoisomer, hydrate, solvate, pharmaceutically acceptable salt, or prodrug thereof according to any one of claims 1 to 9; and
optionally, a pharmaceutically acceptable carrier.

**11.** Use of the compound, or the tautomer, stereoisomer, hydrate, solvate, pharmaceutically acceptable salt, or prodrug thereof according to any one of claims 1 to 9, or the pharmaceutical composition according to claim 10, the use comprising:

acting as an AT2R agonist; and/or
preventing and/or treating a disease of insufficient endogenous generation of Angiotensin II, Ang II; and/or
preventing and/or treating a disease where an increased effect of Ang II is desired or required; and/or
preparing an AT2R agonist; and/or
preparing a medicament, pharmaceutical composition, or formulation for preventing and/or treating a disease where AT2R is expressed and a stimulation of AT2R is desired or necessary.

**12.** A method for preventing and/or treating a disease, wherein the disease is:

a disease of insufficient endogenous generation of Ang II; and/or
a disease where an increased effect of Ang II is desired or necessary; and/or
a disease where AT2R is expressed and a stimulation of AT2R is desired or necessary, and
wherein the method comprises:
administering a therapeutically effective amount of the compound, or the tautomer, stereoisomer, hydrate, solvate, pharmaceutically acceptable salt, or prodrug thereof according to claims 1 to 9, or the pharmaceutical

composition according to claim 10, to a person suffering from or susceptible to the disease.

**13.** The use according to claim 11 or the method according to claim 12, wherein the disease is a disease of gastrointestinal tract, cardiovascular system, respiratory tract, kidney, eyes, female reproductive system, or central nervous system.

**14.** The use according to claim 11 or the method according to claim 12, wherein the disease is esophagitis, Barrett's esophagus, gastric ulcer, duodenal ulcer, dyspepsia, gastroesophageal reflux, allergic bowel syndrome, inflammatory enteritis, pancreatitis, liver disease, gallbladder disease, multiple organ failure, sepsis, xerostomia, gastritis, gastric stasis, hyperacidity, biliary tract disease, abdominal disease, segmental ileitis, ulcerative colitis, diarrhea, constipation, acute colic, dysphagia, nausea, vomiting, Sjögren's syndrome, inflammatory disease, asthma, obstructive pulmonary disease, pneumonia, pulmonary hypertension, adult respiratory distress syndrome, idiopathic pulmonary fibrosis, renal failure, nephritis, renal hypertension, diabetic retinopathy, retinopathy of prematurity, retinal microvascularization, ovulatory mechanism disorder, hypertension, myocardial hypertrophy, heart failure, atherosclerosis, arterial thrombosis, venous thrombosis, endothelial dysfunction, endothelial damage, stenosis after balloon dilation, angiogenesis, diabetic complications, microvascular dysfunction, angina pectoris, arrhythmia, intermittent claudication, pre-eclampsia, myocardial infarction, reinfarction, ischemic damage, erectile dysfunction, neointimal hyperplasia, cognitive dysfunction, feeding dysfunction, thirst, stroke, cerebral hemorrhage, cerebral embolism, cerebral infarction, hypertrophy, prostatic hyperplasia, autoimmune disease, psoriasis, obesity, nerve regeneration, ulcers, inhibition of adipose tissue hypertrophy, stem cell differentiation and proliferation, cancer, apoptosis, tumors, proliferative diabetes, nerve damage, or organ rejection.

**15.** The use according to claim 11 or the method according to claim 12, wherein the disease is asthma, obstructive pulmonary disease, pneumonia, pulmonary hypertension, adult respiratory distress syndrome, or idiopathic pulmonary fibrosis.

## INTERNATIONAL SEARCH REPORT

International application No.
**PCT/CN2024/114192**

### A. CLASSIFICATION OF SUBJECT MATTER

C07D409/10(2006.01)i; A61K31/4178(2006.01)i; A61P1/00(2006.01)i; A61P9/00(2006.01)i; A61P11/00(2006.01)i; A61P13/12(2006.01)i; A61P15/00(2006.01)i; A61P27/02(2006.01)i

According to International Patent Classification (IPC) or to both national classification and IPC

### B. FIELDS SEARCHED

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNABS; EPTXT; USTXT; WOTXT; JPTXT; CNTXT; CNKI, Registry (STN), Caplus (STN), Marpat (STN): 武汉人福, AT2R, AT2, 受体, receptor, 结构式检索, structural formula search

### C. DOCUMENTS CONSIDERED TO BE RELEVANT

| Category* | Citation of document, with indication, where appropriate, of the relevant passages | Relevant to claim No. |
|---|---|---|
| X | CN 1529697 A (VICORE PHARMA AB) 15 September 2004 (2004-09-15)<br>claims 1 and 25-44 | 1-2, 4-15 |
| X | WO 2015014634 A1 (CHARITE - UNIVERSITÄTSMEDIZIN BERLIN) 05 February 2015 (2015-02-05)<br>claim 4, and description, page 15 | 1-2, 4-15 |
| X | WO 2023281271 A1 (VICORE PHARMA AB) 12 January 2023 (2023-01-12)<br>description, pages 5-18, and claim 10 | 1, 3-15 |
| X | CN 101193886 A (VICORE PHARMA AB) 04 June 2008 (2008-06-04)<br>claims 1 and 20-37 | 1-2, 4-15 |
| X | CN 115605265 A (VICORE PHARMA AB) 13 January 2023 (2023-01-13)<br>claims 1 and 14-23, and description, page 2 | 1-2, 4-15 |
| X | CN 115666557 A (VICORE PHARMA AB) 31 January 2023 (2023-01-31)<br>claims 1 and 9-13 | 1-2, 4-15 |
| X | CN 114786774 A (VICORE PHARMA AB) 22 July 2022 (2022-07-22)<br>description, pages 3-4 | 1-2, 4-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 November 2024** | **19 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/114192**

**C. DOCUMENTS CONSIDERED TO BE RELEVANT**

| Category* | Citation of document, with indication, where appropriate, of the relevant passages | Relevant to claim No. |
|---|---|---|
| PX | WO 2024149712 A1 (VICORE PHARMA AB) 18 July 2024 (2024-07-18)<br>claims 1 and 12-25, and description, page 5 | 1, 3-15 |
| PX | CN 116891464 A (WUHAN HUMANWELL INNOVATIVE DRUG RESEARCH AND DEVELOPMENT CENTER LIMITED COMPANY et al.) 17 October 2023 (2023-10-17)<br>claims 1 and 18-23 | 1-2, 4-15 |
| A | EP 0512675 A1 (MERCK & CO. INC.) 11 November 1992 (1992-11-11)<br>description, pages 2-18 | 1-15 |

Form PCT/ISA/210 (second sheet) (July 2022)

**INTERNATIONAL SEARCH REPORT**

International application No.
**PCT/CN2024/114192**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11-15**
because they relate to subject matter not required to be searched by this Authority, namely:

The subject matter of claims 11-15 relates to a method for treating diseases of a human or animal body, which falls within the cases set out in PCT Rule 39.1(iv). The search is performed on the basis of the use of the compound, etc. in the preparation of a drug for the corresponding diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/114192**

| Patent document cited in search report | | | Publication date (day/month/year) | Patent family member(s) | | | Publication date (day/month/year) |
|---|---|---|---|---|---|---|---|
| CN | 1529697 | A | 15 September 2004 | US | 2004167176 | A1 | 26 August 2004 |
| | | | | US | 7652054 | B2 | 26 January 2010 |
| | | | | MXPA | 03011693 | A | 06 December 2004 |
| | | | | US | 2009326026 | A1 | 31 December 2009 |
| | | | | US | 8124638 | B2 | 28 February 2012 |
| | | | | EP | 1395566 | A1 | 10 March 2004 |
| | | | | EP | 1395566 | B1 | 12 September 2007 |
| | | | | ES | 2295339 | T3 | 16 April 2008 |
| | | | | AU | 2002257970 | B2 | 02 August 2007 |
| | | | | DE | 60222409 | D1 | 25 October 2007 |
| | | | | DE | 60222409 | T2 | 25 September 2008 |
| | | | | CA | 2449150 | A1 | 05 December 2002 |
| | | | | CA | 2449150 | C | 12 July 2011 |
| | | | | ATE | 372987 | T1 | 15 September 2007 |
| | | | | WO | 02096883 | A1 | 05 December 2002 |
| | | | | DK | 1395566 | T3 | 07 January 2008 |
| WO | 2015014634 | A1 | 05 February 2015 | US | 2016175286 | A1 | 23 June 2016 |
| | | | | US | 10016397 | B2 | 10 July 2018 |
| | | | | PT | 3027183 | T | 11 January 2019 |
| | | | | DK | 3027183 | T3 | 17 December 2018 |
| | | | | PL | 3027183 | T3 | 31 May 2019 |
| | | | | ES | 2704229 | T3 | 15 March 2019 |
| | | | | EP | 3027183 | A1 | 08 June 2016 |
| | | | | EP | 3027183 | B1 | 29 August 2018 |
| | | | | EP | 2832357 | A1 | 04 February 2015 |
| WO | 2023281271 | A1 | 12 January 2023 | CL | 2024000066 | A1 | 28 June 2024 |
| | | | | JP | 2024523576 | A | 28 June 2024 |
| | | | | EP | 4367110 | A1 | 15 May 2024 |
| | | | | CO | 2024000096 | A2 | 10 May 2024 |
| | | | | AU | 2022306778 | A1 | 18 January 2024 |
| | | | | CA | 3222721 | A1 | 12 January 2023 |
| | | | | IL | 309605 | A | 01 February 2024 |
| | | | | MX | 2024000518 | A | 31 January 2024 |
| | | | | KR | 20240035402 | A | 15 March 2024 |
| | | | | US | 2024262813 | A1 | 08 August 2024 |
| CN | 101193886 | A | 04 June 2008 | CA | 2604797 | A1 | 19 October 2006 |
| | | | | CA | 2604797 | C | 28 May 2013 |
| | | | | AU | 2006235698 | A1 | 19 October 2006 |
| | | | | AU | 2006235698 | B2 | 29 March 2012 |
| | | | | WO | 2006109048 | A1 | 19 October 2006 |
| | | | | MX | 2007012634 | A | 11 January 2008 |
| | | | | JP | 2008535898 | A | 04 September 2008 |
| | | | | JP | 5202301 | B2 | 05 June 2013 |
| | | | | ATE | 540947 | T1 | 15 January 2012 |
| | | | | ES | 2395194 | T3 | 11 February 2013 |
| | | | | EP | 1869023 | A1 | 26 December 2007 |
| | | | | EP | 1869023 | B1 | 11 January 2012 |
| | | | | JP | 2013040208 | A | 28 February 2013 |
| | | | | JP | 5718301 | B2 | 13 May 2015 |
| | | | | US | 2009042931 | A1 | 12 February 2009 |

Form PCT/ISA/210 (patent family annex) (July 2022)

**INTERNATIONAL SEARCH REPORT**
**Information on patent family members**

International application No.
**PCT/CN2024/114192**

| Patent document cited in search report | Publication date (day/month/year) | Patent family member(s) | Publication date (day/month/year) |
|---|---|---|---|
| | | US 8080571 B2 | 20 December 2011 |
| | | DK 1869023 T3 | 10 April 2012 |
| | | KR 20080009275 A | 28 January 2008 |
| | | KR 101429484 B1 | 13 August 2014 |
| CN 115605265 A | 13 January 2023 | JP 2023518948 A | 09 May 2023 |
| | | AU 2021238939 A1 | 10 November 2022 |
| | | WO 2021186185 A1 | 23 September 2021 |
| | | IL 296247 A | 01 November 2022 |
| | | US 2023159467 A1 | 25 May 2023 |
| | | EP 4121165 A1 | 25 January 2023 |
| | | KR 20230004501 A | 06 January 2023 |
| | | CL 2022002540 A1 | 21 April 2023 |
| | | CA 3175559 A1 | 23 September 2021 |
| | | CO 2022014673 A2 | 16 January 2023 |
| | | BR 112022018549 A2 | 29 November 2022 |
| | | MX 2022011613 A | 21 October 2022 |
| CN 115666557 A | 31 January 2023 | WO 2021186180 A1 | 23 September 2021 |
| | | EP 4121047 A1 | 25 January 2023 |
| | | EP 4121047 B1 | 30 October 2024 |
| | | JP 2023518474 A | 01 May 2023 |
| | | US 2023114153 A1 | 13 April 2023 |
| | | GB 202004094 D0 | 06 May 2020 |
| | | CA 3174437 A1 | 23 September 2021 |
| CN 114786774 A | 22 July 2022 | MX 2022003286 A | 12 April 2022 |
| | | EP 4031240 A1 | 27 July 2022 |
| | | JP 2022549086 A | 24 November 2022 |
| | | GB 201913603 D0 | 06 November 2019 |
| | | BR 112022003130 A2 | 17 May 2022 |
| | | AU 2020351301 A1 | 17 March 2022 |
| | | CO 2022003091 A2 | 19 April 2022 |
| | | CA 3152218 A1 | 25 March 2021 |
| | | CL 2022000656 A1 | 04 November 2022 |
| | | WO 2021053344 A1 | 25 March 2021 |
| | | IL 291172 A | 01 May 2022 |
| | | US 2022388994 A1 | 08 December 2022 |
| | | KR 20220101071 A | 19 July 2022 |
| WO 2024149712 A1 | 18 July 2024 | None | |
| CN 116891464 A | 17 October 2023 | TW 202404959 A | 01 February 2024 |
| | | WO 2023193733 A1 | 12 October 2023 |
| | | AU 2023249574 A1 | 17 October 2024 |
| EP 0512675 A1 | 11 November 1992 | JPH 05140153 A | 08 June 1993 |
| | | US 5198438 A | 30 March 1993 |
| | | CA 2063895 A1 | 08 November 1992 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311089313
- CN 202410298612
- CN 202411103404
- WO 2021229244 A1


### Non-patent literature cited in the description


- **CAREY** ; **SUNDBERG**. ADVANCED ORGANIC CHEMISTRY. Plenum Press, 2000, vol. A
- **CAREY, SUNDBERG**. ADVANCED ORGANIC CHEMISTRY. Plenum Press, 2001, vol. B
- **MAEHR**. *J. Chem. Ed.*, 1985, vol. 62, 114-120